# EUROPEAN PATENT SPECIFICATION

(11) **EP 2 272 975 B1**
(45) Date of publication and mention of the grant of the patent: **22.04.2015**
(21) Application number: 09725639.0
(22) Date of filing: 25.03.2009
(51) Int. Cl.: C12Q 1/68, C12N 15/09, G01N 33/53

(54) **METHOD FOR DETERMINATION OF DNA METHYLATION**
BESTIMMUNGSVERFAHREN FÜR DNA-METHYLIERUNG
PROCÉDÉ SERVANT À DÉTERMINER LA MÉTHYLATION DE L'ADN

(30) Priority: 25.03.2008 JP 2008077967
(43) Date of publication of application: 12.01.2011
(73) Proprietor: Sumitomo Chemical Company, Limited, Tokyo 104-8260 (JP)
(72) Inventor: TOMIGAHARA, Yoshitaka, Toyonaka-shi Osaka 560-0013 (JP); SATOH, Hideo, Ibaraki-shi Osaka 567-0826 (JP); TARUI, Hirokazu, Toyonaka-shi Osaka 561-0802 (JP)
(74) Representative: Duckworth, Timothy John
(86) International application number: PCT/JP2009/056785
(87) International publication number: WO 2009/119891

(56) References cited:
- WO-A1-2006/056478
- WO-A1-2007/119518
- WO-A1-2008/123537
- WO-A1-2008/123538
- WO-A2-2007/081791
- US-A1- 2007 264 642
- STERKY F ET AL: "Direct sequencing of bacterial artificial chromosomes (BACs) and prokaryotic genomes by biotin-capture PCR", JOURNAL OF BIOTECHNOLOGY, ELSEVIER SCIENCE PUBLISHERS, AMSTERDAM, NL, vol. 60, no. 1-2, 5 February 1998 (1998-02-05), pages 119-129, XP004115549, ISSN: 0168-1656, DOI: DOI:10.1016/S0168-1656(97)00196-X
- YU K TONG ET AL: "Detection of restriction enzyme-digested target DNA by PCR amplification using a stem-loop primer:application to the detection hypomethylated fetal DNA in maternal plasma", CLINICAL CHEMISTRY, AMERICAN ASSOCIATION FOR CLINICAL CHEMISTRY, WASHINGTON, DC, vol. 53, no. 11, 27 September 2007 (2007-09-27), pages 1906-1914, XP008132142, ISSN: 0009-9147, DOI: DOI:10.1373/CLINCHEM.2007.092619
- WEBER MICHAEL ET AL: "Chromosome-wide and promoter-specific analyses identify sites of differential DNA methylation in normal and transformed human cells.", NATURE GENETICS AUG 2005 LNKD- PUBMED:16007088, vol. 37, no. 8, August 2005 (2005-08), pages 853-862, XP002634922, ISSN: 1061-4036
- TONG, YU K. ET AL.: 'Detection of restriction enzyme-digested target DNA by PCR amplification using a stem-loop primer: application to the detection of hypomethylated fetal DNA in maternal plasma.' CLINICAL CHEMISTRY vol. 53, no. 11, 2007, pages 1906 - 1914, XP008132142
- SHIN SEIKAGAKU JIKKEN KOZA 2 KAKUSAN I -BUNRI SEISEI 1991, TOKYO, pages 169 - 172, 180, XP008150058
- YARMUSH, MARTIN L. ET AL.: 'Immunoadsorption: strategies for antigen elution and production of reusable adsorbents.' BIOTECHNOLOGY PROGRESS vol. 8, no. 3, 1992, pages 168 - 178, XP008143270
- HEISKANEN, MERVI ET AL.: 'A novel method to quantitate methylation of specific genomic regions.' PCR METHODS AND APPLICATIONS vol. 4, no. 1, 1994, pages 26 - 30, XP000465079
- ALLEN, R. CUTLER ET AL.: 'Methylation of HpaII and HhaI sites near the polymorphic CAG repeat in the human androgen-receptor gene correlates with X chromosome inactivation.' AMERICAN JOURNAL OF HUMAN GENETICS vol. 51, no. 6, 1992, pages 1229 - 1239, XP008143271
- NYGREN, ANDERS O. H. ET AL.: 'Methylation- specific MLPA (MS-MLPA): simultaneous detection of CpG methylation and copy number changes of up to 40 sequences.' NUCLEIC ACIDS RESEARCH vol. 33, no. 14, 2005, pages E128 - 1-9, XP002596755
- STERKY, FREDRIK ET AL.: 'Direct sequencing of bacterial artificial chromosomes (BACs) and prokaryotic genomes by biotin-capture PCR.' JOURNAL OF BIOTECHNOLOGY vol. 60, no. 1-2, 1998, pages 119 - 129, XP004115549
- SCHMIDT, PETER M. ET AL.: 'Detection of activity of telomerase in tumor cells using fiber optical biosensors.' BIOSENSORS & BIOELECTRONICS vol. 17, no. 11-12, 2002, pages 1081 - 1087, XP001206070
- BUCKLE, MALCOLM. ET AL.: 'Real time measurements of elongation by a reverse transcriptase using surface plasmon resonance.' PROCEEDINGS OF THE NATIONAL ACADEMY OF SCIENCES OF THE UNITED STATES OF AMERICA vol. 93, no. 2, 1996, pages 889 - 894, XP002252240

### SEQUENCE LISTING

<110> Sumitomo Chemical Co., Ltd.
<120> Method for measuring DNA methylation
<130> N112271
<140> 09725639.0
   <141> 2009-03-25
<150> JP2008-077967
   <151> 2008-03-25
<160> 62
<210> 1
   <211> 2661
   <212> DNA
   <213> Homo sapiens
<400> 1
   acagacatgt gccaccatgc ccagctaatt ttttgtttgt ttgtttgttt gtttgtattt    60
   ttagtagaga tggggttttg ccatgttggc caggctggtc tcgaactcct gacctcgaat   120
   gataatgatc cgccgcttgg cctccaaagt gctaggatta caggtgtgag ccactgcgcc   180
   aggcctgggc actttcttta gtagtttgag gagcaacatt tttgacagtg tccttctgct   240
   caagattcaa gatcccagat aaaattaaac catctagaga gatggcttga ttggccaaac   300
   ctggatctca tgaccacttc ttgaagtggg taagtctcat aaatgctcag tccttccact   360
   atgcaactga gtggggtggg tgggaagccc ctcaaaggaa aatccggttg ttcttactag   420
   aaagaaaagg aaaatggatg tgaggcagtc aaaatcagca gaggtccacc acaccaccaa   480
   aatgtggtga ttaaatatgg agagacagag actaacagag gtatgtgaat attgaagtat   540
   gtctggacaa tagcccaatg atgagaccaa taaaatggtt accaaaatct ggttttgagt   600
   agtagtgtta aatcagacca tttagtaacc attttttgtt gcaaagtttc tagcactgcc   660
   caaaccctga gtggtatatg aataactcgt ccattatgta tctctttcca gtcagcataa   720
   tttatccccc acctatattc ttttctgacc actcctactt ccttctcttt accaaaatct   780
   aaactctaag gctgtttctt cagcaacttc tttgtttaga ttggaagata aattaaacag   840
   catgcgatgt tttactgact ttcagtattt aacagaggtg atttaatttt tttttaaatc   900
   caaagtcaaa cttctttata agatgaagga gaaaaatgtc ttataaaatg catatgtgaa   960
   gatgccttct gagtgctttc tcatgcagac ttgttctagt ctttaatgaa tcttccttgt  1020
   agacactgtg gagatgaaag atggttctcc acttctactc aaagtacaaa tcaggccggc  1080
   attttgaaaa agagacaggt ttattcatag ctgcagcgtt agctggcttt gttccctgta  1140
   caatttcact tttggttatt aaaatattca ctgtaggaaa taaatttgta acccatttct  1200
   catattacct acacacagaa aaacaaaatt tgatatcctg ggtttatttt gctgagggcg  1260
   cttcccataa aagcgagaga gtgtgcgttg ggaaatgtgt ctggttaact cttttatgga  1320
   taaactttag tcacaatcct cccccgcccc cctctcaccc ccagcaccct cccaacctcc  1380
   cgacttcccg cctctcaagg gctggtgacc taatagcatt tttcttcgtg catattttgg  1440
   cgtcgcccca tggcctggct gccttcgcct gtctgagttt tttgaaattc ctgcatgttc  1500
   gccccagatt aagccagtgt gtctcaggat gtgtgttccg ttttgttctt tccccttaac  1560
   gctccctgtg caacgtgtct ggggggagga gggcagggac gggagagagg gaggggcaga  1620
   ggcgaggagc tgtccgcctt gcacgtttcc aatcgcatta cgtgaacaaa tagctgaggg  1680
   gcggccgggc cagaacggct tgtgtaactt tgcaaacgtg ccagaaagtt taaatctctc  1740
   ctccttcctt cactccagac actgcccgct ctccgggact gccgcgcggc tccccgttgc  1800
   cttccaggac tgagaaaggg gaaagggaag ggtgccacgt ccgagcagcc gccttgactg  1860
   gggaagggtc tgaatcccac ccttggcatt gcttggtgga gactgagata cccgtgctcc  1920
   gctcgcctcc ttggttgaag atttctcctt ccctcacgtg atttgagccc cgtttttatt  1980
   ttctgtgagc cacgtcctcc tcgagcgggg tcaatctggc aaaaggagtg atgcgcttcg  2040
   cctggaccgt gctcctgctc gggcctttgc agctctgcgc gctagtgcac tgcgcccctc  2100
   ccgccgccgg ccaacagcag cccccgcgcg agccgccggc ggctccgggc gcctggcgcc  2160
   agcagatcca atgggagaac aacgggcagg tgttcagctt gctgagcctg ggctcacagt  2220
   accagcctca gcgccgccgg gacccgggcg ccgccgtccc tggtgcagcc aacgcctccg  2280
   cccagcagcc ccgcactccg atcctgctga tccgcgacaa ccgcaccgcc gcggcgcgaa  2340
   cgcggacggc cggctcatct ggagtcaccg ctggccgccc caggcccacc gcccgtcact  2400
   ggttccaagc tggctactcg acatctagag cccgcgaacc tggcgcctcg cgcgcggaga  2460
   accagacagc gccgggagaa gttcctgcgc tcagtaacct gcggccgccc agccgcgtgg  2520
   acggcatggt gggcgacgac ccttacaacc cctacaagta ctctgacgac aacccttatt  2580
   acaactacta cgatacttat gaaaggccca gacctggggg caggtaccgg cccggatacg  2640
   gcactggcta cttccagtac g                                            2661
<210> 2
   <211> 1953
   <212> DNA
   <213> Homo sapiens
<400> 2
   tataaattcc acgcaggcat tgaattgaat ttgttcttaa ccaaatgcgt tttatctata   60
   cctggcagga atctagaagt gaaattacaa gatttatttc attttaattc tattatgaag  120
   catttaatca caaataccct gaaaatgaaa agataattta tcattttacc ttgactgagc  180
   aactctcctc acttcacatt catgaatcca taacgcagag aggagactgg atgattaagt  240
   gtttgattag agaaaacaga ttaacctagc aaacataata aatttggctc ataagcagga  300
   tggctttata aatgctcaca atacctctcc tgtataaaat catgaaccac ttcctacagt  360
   gatgactcca tcgaaatagt tgagaaacat aaagcaaatg catgtttatg gctttctctt  420
   tgagacatta aaagggtatt gaaaggcata tctgattcag cttataactc tggatatata  480
   ttaaggaaca tgtaagaaaa tattaatgca taaaaaaagc tacaacttct caagtgttct  540
   agtttccact ttgtcaataa ttacgttttc aatgtccttc tgtggactgt ttccaaaggt  600
   gccaatccag acccaaagtt tcagatcact cagattcacc cttaaccttc ataacacaac  660
   ccaatagctt tacgaaaaaa gttgcatatt taggtagttg ttatcccatt atgacaaaat  720
   acataaaatt agcgagatat tttttagcct tcaaataagt gggaaaaaat ccttttagct  780
   gagattccat ttacatcaga ataaaaatct aagttatgac taggttgaag caacgtcctg  840
   tgcagcgctc cataaagttc acttagtctt caagggttcc ttacttagct aggttagtat  900
   tcctggcctc tttttttagc agtgagaaaa aggatactct ccctgcccca gctttatttt  960
   taaactcaca gccatatcct ggaggtctct gctggctatt tggcgcgtgg gggcggaggg 1020
   gggccggggg aggggggcgg ggcggggtct ggaggtctgt gctggctatc tggcgtgtgt 1080
   gtgtgtgtgt gtgtgtgtgt gtgtggttgg aggtctctgc tggctatctg gcgtgtgtgt 1140
   gtgtgtggtg tggtgtgtgt aagcagtgag gttgttttag ggccagtcct tcctccgcca 1200
   ctttgctgac tcaaagaccc agaggctttc ttggggtgca ggtaccatga ttccttgggc 1260
   cctaagggaa tttttgttag gctagaagag tgggtgtact catgatgggt gtacccgaac 1320
   attcctgggc tcaacaaaac cgattatctt tataaccgcg gcgcctagca cagcgcctgg 1380
   tgccctaaac gttggctgcg ggaacgtccg agacgcgggt gcggagccgg gggcggaata 1440
   actggttgcg cggcgctttg accgtaggcg ctggagcgcg tgcgttgcgt gcgcgcgcgg 1500
   aggcggctgc gtcggggcgc gagaaggtgc agttccccgg cgggcgggcg ggcgggcggg 1560
   cgaagctggg ctcggggcca agcgaggtct agccggagcg actgtgcccc gcctcctggg 1620
   cggagcgggc ggctccccat ggtcagagcc tcgtgccggc tcggcagcgc ccggacgccg 1680
   agcccagcgc gtcggccccc cggcgtgcgg gcgtctcaga gccgcggagg ggccgccggg 1740
   accgtttcag cgtggcggcg ctggtgctgg cgttggccct ggaggacggc cccgagtgat 1800
   ggctggcgcc tgcctcccgg gtgtctcccg ggtacagatg gagtcgtccc gcggccgccg 1860
   gcggcaaggt cggcagctgc gaggccaaga gagaccccag gacacacaca gctgcctccc 1920
   ggtgcgagaa gaagaccccg gcttgagagt gag                              1953
<210> 3
   <211> 889
   <212> DNA
   <213> Homo sapiens
<400> 3
   cggccccatg gctccgtgtc gtgtccaagg gatgggctgg cacctcttgg accaggctta   60
   ccaccagggc ccttctctga agccccagtc tgaccggcct gctgctggga atccccctct  120
   gcccccacac taacctctgc tggggctgag ccagggcgcg tcggacagtc agggcgaccc  180
   agccagggcg accgttggcc ccgctcctat ggggcagcag ggaccgacgt cagcagggtg  240
   gggcgggcac ccgagtggta tgccccgccc tgccccgcct gcccgccctg gtggccgtct  300
   gggggcgaca agtcctgaga gaaccagacg gaagcgcgct gggactgaca cgtggacttg  360
   ggcggtgctg cccgggtggg tcagcctggg ctgggaggca gccccgggac acagctgtgc  420
   ccacgccgtc tgagcacccc aagcccgatg cagccacccc cagacgaggc ccgcagggac  480
   atggccgggg acacccagtg gtccaggtgt ggcgggggtg aggggagggg gggtgggagc  540
   ggtggagatg gggccgtggg gagggagctg agatactgcc acgtgggacg atgctaggtg  600
   gggagggctg agctgggcgg gctcctctgg ctgtggggcc ccctgtgttc cttgtgggag  660
   gtggaaggaa gtgagtgccc tgtccttcct ccctgccatg agattccagg accggacctg  720
   gcaagtgccc tatcccagcc agtgttcctg gggctcttcc aggcagggct atgttcccca  780
   ggccaggggc attgtcctgg acagtcagga ggcatacccc tcgccaggtg gaaccaccct  840
   gtgtatgcat gaccctgaca agcaggcgcc aggacagtca ggaggccag              889
<210> 4
   <211> 863
   <212> DNA
   <213> Homo sapiens
<400> 4
   gttgttgggt gtgaatggag aactgtgggc cctccccgac accttccagc gggacggcaa     60
   cgggggccca gggggtgggc gccatcaacc ccgtcccacc gccaggacgg cgcgggggag    120
   ggccggcggg ggcggggcgt cctgtaaggc gcggccccca cccgcgggcg gggcggcatt    180
   cctgggaggc cggcgctctg acgtggaccc gggggccgcg ggcacggcgg gggggcggcg    240
   gtccgggggc ttcttaaacc ccccgccccg gcccagcccg cacttcccga gcaccgctcc    300
   gaccctggag ggagagagag ccagagagcg gccgagcgcc taggaggccc gccgagcctc    360
   gccgagcccc gccagccccg gcgcgagaga agttggagag gagagcagcg cagcgcagcg    420
   agtcccgtgg tcgcgcccca acagcgcccg acagcccccg atagcccaaa ccgcggccct    480
   agccccggcc gcacccccag cccgcgccag catgatgaac aacagcggct actcagacgc    540
   cggcctcggc ctgggcgatg agacagacga gatgccgtcc acggagaagg acctggcgga    600
   ggacgcgccg tggaagaaga tccagcagaa cacattcacg cgctggtgca atgagcacct    660
   caagtgcgtg ggcaagcgcc tgaccgacct gcagcgcgac ctcagcgacg ggctccggct    720
   catcgcgctg ctcgaggtgc tcagccagaa gcgcatgtac cgcaagttcc atccgcgccc    780
   caacttccgc caaatgaagc tggagaacgt gtccgtggcc ctcgagttcc tcgagcgcga    840
   gcacatcaag ctcgtgtcca tag                                            863
<210> 5
   <211> 2198
   <212> DNA
   <213> Homo sapiens
<400> 5
   aagagaggca cactccctct accacaccga gggagggggc gttgagctga gaaaggttga     60
   gagaatgagg gacccaggta ggtggacatc ggccaagaaa ggaaccacag cgggaggtaa    120
   gaccgagagt ccccagcttg aagcgtcacc actccgggat tcccagattc caacgcgagc    180
   ctggggaaag cccacagtgg agagagtccg gctggcaggg aatggcccta cccccggggt    240
   gaaatctcgg agggtcgtgc agccgagtcg cgcctctgcg ctgatgcgtg agagatgccg    300
   gacgtcgcgt ttgcctgtgc gagcctcgcg gatgctgtgc agtcttggtc ccctctgcgt     360
   gtgtctaacg ccgaatgctg gtgtctcgag gtgtgagctt cggggccggt gtctttaaag    420
   aaccaaagat tcttaaggag tgatgatctg ggtagagcgg cccgacgtag ccgcgctccc    480
   aggtctcggt gcgagtcctg cggacagacc agaggagacc tgctggccag atgccccggg    540
   cccaaggcgg acgccagact gtctctgcgc cagccgggct ggccttcgga atggatcagg    600
   cacccgggag gccggagtgg atctcagacc ctcaagccgg gaacaaaccc gtcgatgccc    660
   gtgggcctgg agtccgcctc ctccttcccg ccccacccct acccctgcct ccgaaaggct    720
   tcttcgctgg tcagtagctg cgtgcccgtc tgcctgaggc tgggtcagaa ttggcgggct    780
   ggtaacgacc ccgtgcacaa gcggctccca gtctctccag aaagggccga tgactaaggg    840
   gtgggggtgg gggcggaggg ctggaaggtg ttagggaaga acgttagcgg cctatcctgt    900
   cttcagcagc gccctctcat cttctagctc tgacgccgag cagagcagtt ggagctcggg    960
   actgggaact gctggaattc ctatttagac ttctagacag tctagaaaca agaacctttc   1020
   tttccctggg cctcagtttc cttgtctgta aaatcaaaag gcgggctcta ggtgtaggcc   1080
   ttcttttcgc ttggtgattc tggattcctt tccttggatc cgtggggagg gggtggcagc   1140
   aacagtccag ggcgttggcc gtcctgtgcc tcaagtacgt agtccccgtg cccgccccct  1200
   caacaccccc agcagcccgc ccccctaagc ccgcagagca gggagctgag tgggaggggc  1260
   agaggcgggg ccggttccca gtccctgctg gcggactaga gtggcgcggg ctgagcgtaa   1320
   aacctgggat agccactccc ccttttcctt atccccgccc ccctgccatt ggctcccggg   1380
   agaggttgac atcaaagccg cggtcttata taagccagat ccgcagggga gtccgcagaa   1440
   gggttaaaca ggtctttggg cttcggcgac ctcgcccgcg gcagaaaccg gtaagaagac   1500
   agtgggctgc gcgtctcatt ttcagccttg cccggactct cccaaagccg gcgcccagta   1560
   gtggctccag agcccacagg tggcccccgg cagtctctgg ggcgcatgga gcggcgttaa   1620
   tagggctggc ggcgcaggcc agtagccgct ccaacatgaa cctcgtgggc agctacgcac   1680
   accatcacca ccatcaccac ccgcaccctg cgcaccccat gctccacgaa cccttcctct   1740
   tcggtccggc ctcgcgctgt catcaggaaa ggccctactt ccagagctgg ctgctgagcc   1800
   cggctgacgc tgccccggac ttccctgcgg gcgggccgcc gcccgcggcc gctgcagccg   1860
   ccaccgccta tggtcctgac gccaggcctg ggcagagccc cgggcggctg gaggcgcttg   1920
   gcggccgtct tggccggcgg aaaggctcag gacccaagaa ggagcggaga cgcactgaga   1980
   gcattaacag cgcattcgcg gagttgcgcg agtgcatccc caacgtgccg gccgacacca   2040
   agctctccaa gatcaagact ctgcgcctag ccaccagcta catcgcctac ctgatggacg   2100
   tgctggccaa ggatgcacag tctggcgatc ccgaggcctt caaggctgaa ctcaagaagg   2160
   cggatggcgg ccgtgagagc aagcggaaaa gggagctg                           2198
<210> 6
   <211> 1945
   <212> DNA
   <213> Homo sapiens
<400> 6
   ctggatgaca gagtgagact ccgtctcaaa aaaaaagctc catttgggag gccgaggagg     60
   gtggattacc tgaggtcagg agtttgagac cagcctggcc cacataggga aaccccatct    120
   ctactaaaaa tacaaaaatt agtcaggtgt ggtggctgac acctataatc ccagctactt    180
   gggaagctga ggcagggaga atcacttgaa ccggggaggt ggaggttgca gtgagctgag    240
   atcatgccac tgcactccag cctgggcgac agggtgagat tctgtctcaa acaaacaaat    300
   ttaaaagctc cgaatcctcc aaaaatacca agattttcct  gtcggtaact agagatgggt    360
   actgatgatt atttttaata ggtgattttc aaagatgtga acgttatcca tggagattta    420
   agtctccaaa aggaaaaaaa atgcatacct ttatactaaa acttcatcac cagtcaaatt    480
   tggatcatca ctaaattggc ttctacacct ctctcctaat ataaggtact tgtgtaagtt    540
   tgcagttgtg agacacttat ttcctcattt ttaatgtctt ctcagtaggg ccactgatat    600
   agtcactatt tgactgacca gaatggttgg cactggtgat tggctcataa agtgccctcg    660
   atttaggggg ctcaattatc aaaggtttaa atcctagccc aaaccattgc tgtgatgggg    720
   gttaatcaat gaaccactca gcttcacttg caaaagcggg atcacaatag ccgctttcgt    780
   catgacccag cctaggtgag atttagtact taagtacact gccaggcaca caaggttaat    840
   ttaacaattt aacacatttg tttcctcatc catttctcca aaccttccaa ctaatcctaa    900
   cgttcgttcg gccaaatggg ccaggaattc acttaaacaa aaacaaaaaa caaaacaaac    960
   aaaaaaacac tccctggggc ttggggaagg aggcaccgcc gcccatgtcg cagtctgggg   1020
   gtggctcagt cctcagcacc cagatctacg gccataatgc tcttcgaggc caaggagccc   1080
   ggatgcgggg cgttgccgaa ggcgtcttgc tcaggctgcg ggaaaggaga ggggtgggag   1140
   cggggtgggg gcatcgcgac ccagggcaag gcggcgagtc gccgtcttcg agtcccacct   1200
   gtccgaagcg gggtgagaaa aggcaaaaca tggcaaagcc atgcacctcc cagggtgggc   1260
   aactcacggc cggtgaacgc cggaccctta  gcagtttcca gacctttgga accggaagcg   1320
   gagcctgaga gcgcgcccga gagggcgtga acgggaccgc tttcccggaa gtgcttgcgg   1380
   cctctgccca gcgagctgcc ccggggtctc tctggtttcc taatcagggc aacgccgcgg   1440
   gagagaacct ttaccttggc tgcactaagt tctcggtgcc actccctggc agggcgggac   1500
   cttgtttagg ccctgtgatc gcgcggttcg tagtagcgca aggcgcagag tggaccttga   1560
   cccgcctagg gcgggaagag tttggcccgc cgggtcccaa agggcagaat ggacgggctc   1620
   ctaaatccca gggaatcctc taaattcatt gcagaaaaca gtcgggatgt gtttattgac   1680
   agcggaggcg tacggagggt ggcagagctg ctgctggcca aggcggcggg gccagagctg   1740
   cgcgtggagg ggtggaaagc ccttcatgag ctgaacccca gggcggccga cgaggccgcg   1800
   gtcaactggg tgttcgtgac agacacgctc aacttctcct tttggtcgga gcaggacgag   1860
   cacaagtgtg tggtgaggta cagagggaaa acatacagtg ggtactggtc cctgtgcgcc   1920
   gccgtcaaca gagccctcga cgaag                                         1945
<210> 7
   <211> 2379
   <212> DNA
   <213> Homo sapiens
<400> 7
   aagcttgtgg tttacttgga cctctgcctc atctttcttc ttttgcgctt cagcctgcgc 60
   attcgcttcc tccactaggc tctcatggtg cagaggtttc caagaagatg gtgtgaaggc 120
   cgagatcatt tggttatatt ataaaataga atgcaaattc acacaagttt ttgtttttta 180
   tttatttatt tttttagaga tgaggtcttg ctatgttgtt tagtctggtc tcgaactcct 240
   ggcctcgtga tcctcccacc ttgacctccc aaagtgctgg gattacaggc ctgaggcctg 300
   agccactaca cccaactgaa ttcacatttt tttttttctt ttctgagacg gagtctcact 360
   ctgtcaccca gtatggagtg cagtggcgcg actgcggctc actgcaagct ccgtctctcg 420
   ggttcaagtg attctcatgc ctcagccccc caagtagctg gaattacagg ggtgcactac 480
   cacacctggc taatttttct gttttagtag agatggggtt tcaccatgtt gcctggtctc 540
   aaactcctga ctttaagtga tccacacacc tcagcctccc aaagtgctgg gattacaggt 600
   gtgagcctcc acacccggcc gaattcacat gaattttaaa gtgatgtctt caaagtggtt 660
   tcactgtggg gatgggcagc tttttgttat acatctagaa cgttcctctt ctgtttctat 720
   gaatactcgg ttggaaaggg ctgaaaaacg gtcttaagag attatctgat tcgtttccca 780
   gttttattac tcacatatca gctgtaattt gagcacgttt tctgattgag acaagactca 840
   gatggtatta aacattacta caacacatcc gggcacggtg gctcacgcct gtaatcccag 900
   cactttggga ggccgaggcg ggcggatcac gaggtcagga gatcgagacc atcctggcta 960
   acacggtgaa gccctgtctc tactaaaaat acaaaaaatt aggcgggcat ggtggcgggc 1020
   gcctgtagtc ccagctactc gggaggctga ggcaggagaa tggcgtgaac ccgggaggcg 1080
   gagcttgcag tgagccgaga tcgcgccact gcactccagc ctgggcgaca gagcaagact 1140
   ccatctcaaa aaaaaaaaaa aaaaaaaaaa actacaacac tataaattca tatctattat 1200
   aatagtactt tgtgcagggc cctaccctaa gtccttaacc gaacccggaa gcgagaagat 1260
   gacttttgtt tgtttttaga gatgggcgcc tggctctgtc gccagcctgg agtgtggggg 1320
   cgcgatctcg actcacagca gcctccacct cccgagttca ggcgatcttc ctgcctcagc 1380
   ccctcgagga gctgggacca ccggcgcgct ccatcgcgcc cggctaggag ctgactttga 1440
   atccgggctc tgcgcctggc cttctgcatc tctataaggg aagacatctg tgacctcggg 1500
   gcaaaggtca aattagatcc tgggtaggat cctgttcccg ctgcccctcg ggctggcact 1560
   gccaggagta ctcagagctc aaagctggga tctgcagtcc cttacccact cagtgcacgc 1620
   cgcctaaggc tttgcgcttc acctttactc acctcgaagc cctggacatc cgcatctgcc 1680
   ctaagacttc tcacctcagt agcagaagga agtcgcgtca gctggccaca gcctctctcc 1740
   taggagaccg tccgggaaaa gcgagtcagg gtagaccctg aggcccctca gctccggcta 1800
   ttttcagatc tgtcgctcct tcaccctcag cctttcaaac aggccactcc aaaaaaaagc 1860
   ccaatcacag ccttccttct tctcctggcc ttccggcact gtccaatcaa cgtacgccat 1920
   ctatcggtta gtggtgttgc ggggccaccc ttcccgctgg tttccctcgt ggtgtgtaaa 1980
   ggcagagagg aaaggcgagg ggtgttgacg ccaggaaggt tccatcttgg ttaagggcag 2040
   gagtccctta cggacttgtc tgaggaaaga caggaaagcg ccagcatctc caccttcccc 2100
   ggaagcctcc ctttgccagg cagaaagggt ttcccatggg gccgcccctg gcgccgcgcc 2160
   cggcccacgt acccggggag gccgggcccc ggaggacgag ggaaagcagg ccgggcgccg 2220
   tgagcttcgc ggacgtggcc gtgtacttct ctcccgagga gtgggaatgc ctgcggccag 2280
   cgcagagggc cctgtaccgg gacgtgatgc gggagacctt cggccacctg ggcgcgctgg 2340
   gtgaggccgg gccctccggc cgggaccccc agtccgtcg 2379
<210> 8
   <211> 933
   <212> DNA
   <213> Homo sapiens
<400> 8
   gagacgtact ctggctctgt cgcccaggct ggagcgcaat ggcgccatct cggcgcactg 60
   caacctccac ctcccgggtt caagcgattc tactgcctca gcctcccgag tagctgggac 120
   tacaggcgcg cactaccaag cccggctaat ttcttttgta tttttagtag agactgggtt 180
   tcacgatgtt ggccgggctg gtctggaagt cttgacctca agcgtgcgcc ctctccgcca 240
   ctgggtaagg cggggggcgg aatagggggc ttgcaatttc acactagagg cgggcgccgt 300
   gggggaaaga agagtcacgt ctcccacggt tcgtagagga aggcctgcct gagcctggag 360
   cgggggcggg agagccacag tttggcatcc ccagggcatc ccccagcccg cagactacca 420
   ggcctccaga ggacaggacc ccacccccgg ccacaggccc tgcccccagc actccccgca 480
   ccccgcctcc aagactcctc cgcccactcc gcacccaact tataaaaacc gtcctcgggc 540
   gcggcgggga gaagccgagc tgagcggatc ctcacacgac tgtgatccga ttctttccag 600
   cggcttctgc aaccaagcgg gtcttacccc cggtcctccg cgtctccagt cctcgcacct 660
   ggaaccccaa cgtccccgag agtccccgaa tccccgctcc caggctacct aagaggatga 720
   gcggtgctcc gacggccggg gcagccctga tgctctgcgc cgccaccgcc gtgctactga 780
   gcgctcaggg cggacccgtg cagtccaagt cgccgcgctt tgcgtcctgg gacgagatga 840
   atgtcctggc gcacggactc ctgcggctcg gccaggggct gcgcgaacac gcggagcgca 900
   cccgcagcca gctgagcgcg ctggagcggc gcc 933
<210> 9
   <211> 6096
   <212> DNA
   <213> Homo sapiens
<400> 9
   atctgcacct cctcatatag ggttgatcca agtttcacag acatcactga gttcttagtg      60
   gactcagcta ttggggctgt tctcacactt tttttttctt tgcaagaatc agcaatgggt     120
   gcaagtggac ctgtgtagga cgtccagtga aacattgtgt tggtgaatca gctagaatcc     180
   atccaagaac tcagccagcc tggtgtgggg tgagatctga tccttgaatg tccctcagtg     240
   gcttttaggg ctggcaggtt cagaagggcc ctctcatcac ccccccaggg cctcattcct     300
   tgtttaacac tttgctatca cagtcttgaa tccttgtaat tgaacaatgg accccacatt     360
   ttcactttgc actggtttct gattctgtaa ccgatcctgt ccccctctct tgtctcattc     420
   actctgggaa ttgtccccac attctgagac ctttcagcag tgccccaacg aggttcctgc     480
   ccttatctga agctccaccc tcacccccat ggcggcaccg caggcagccc tgcttttgcg     540
   tcccgcgtag gcaggctgtg caccggagtc acgaccccct gattcagcct aggcagccac     600
   agcttgactg ctcccgccgg acaagcccta ctgtgctatc tgccgctctt cccttcctct     660
   tcccaggggg tccgcgtcag gggaggcgca gctgtgtgca ttccgggagc ttcagacccc     720
   cgtgtccagc agctccttcg tttcctgggt gctggggcgg ccttcccagc gaagagctca     780
   actcagcggg acgtttggag gctctctgcc ccaaggcgct ggggagtgtg cggcgggaca     840
   gtcgtgcttg cctttttcac tttcagagtg tccacgcccc acccgtttgg tcactgcagg     900
   tcagtccagt ccagcccggc ccaccccacc ggtgcgtgtc tgtcgcacgt ggcagacgcc     960
   atactctctg ttcttgttta aagcccagga tctactgggc cctggaggca agaggtgaac    1020
   gcagcggaat ccacgctgag ctgcccggga acggagcttc caaccccaga aggaggactc    1080
   tgtgctccta caccttaacc ctttttagcc cgaaacttct ccaacttcct tggctttgtt    1140
   tagagctcga cagcgccgcc ccctggcgct cgttgtgagg acagtagagg agagaggcaa    1200
   gggtgttttt aaacagtttg cctctcacca ttatgggggc gacccgaggg ggagacccac    1260
   tcttccgcat tcccggtaag tgaaccaccg gaagaggtcg aaagtgacgg attcccatgt    1320
   cctcctccag cccccccccc accctgccca tccacaggac ggtggctctt cagtgccctt    1380
   tgccgagcaa gtggcgtttc tatgcacgtg ggtatcaatt cggactctgg acgaaatgga    1440
   aacctcctta gccgacccgg gtgggatcag ctgggatcct gcgcgctccc ctggggggtt    1500
   gccagccact ctgttggggt gcaagaagca ccatccttcg gaagctgggc cgaaactggc    1560
   caggctgact cgctcccacg cgcccgcccc tacccggcgc cgcagcaatt cacctgccac    1620
   cgcctctgag ccgggtccgg acttcggcgc cctgacagtg tccccgcgac ttccccaccc    1680
   gatgagatgg ggtctggcgt tggccagtgc gtgtccaggg actcgcgggt ccctggccag 1740
   ccatggggca gagggcgctg gtgttaggcc agtcttcccc accctgcccc gtcaccccag 1800
   ccacacccac tgtcctgtga ggccaagcgc gctccgctgg tttcctgagc caggcacctt 1860
   ggccgcggac aggatccagc tgtctctcct tgcgatcctg tcttcgggga agtccacgtc 1920
   ctaggcaggt cctcccaaag tgcccttggt gccgatcacc cctcccagcg tcttgcaggt 1980
   cctgtgcacc acctccccca ctccccattc aaagccctct tctctgaagt ctccggttcc 2040
   cagagctctt gcaatccagg ctttccttgg aagtggctgt aacatgtatg aaaagaaaga 2100
   aaggaggacc aagagatgaa agagggctgc acgcgtgggg gcccgagtgg tgggcgggga 2160
   cagtcgtctt gttacagggg tgctggcctt ccctggcgcc tgcccctgtc ggccccgccc 2220
   gagaacctcc ctgcgccagg gcagggttta ctcatcccgg cgaggtgatc ccatgcgcga 2280
   gggcgggcgc aagggcggcc agagaaccca gcaatccgag tatgcggcat cagcccttcc 2340
   caccaggcac ttccttcctt ttcccgaacg tccagggagg gagggccggg cacttataaa 2400
   ctcgagccct ggccgatccg catgtcagag gctgcctcgc aggggctgcg cgcagcggca 2460
   agaagtgtct gggctgggac ggacaggaga ggctgtcgcc atcggcgtcc tgtgcccctc 2520
   tgctccggca cggccctgtc gcagtgcccg cgctttcccc ggcgcctgca cgcggcgcgc 2580
   ctgggtaaca tgcttggggt cctggtcctt ggcgcgctgg ccctggccgg cctggggttc 2640
   cccgcacccg cagagccgca gccgggtggc agccagtgcg tcgagcacga ctgcttcgcg 2700
   ctctacccgg gccccgcgac cttcctcaat gccagtcaga tctgcgacgg actgcggggc 2760
   cacctaatga cagtgcgctc ctcggtggct gccgatgtca tttccttgct actgaacggc 2820
   gacggcggcg ttggccgccg gcgcctctgg atcggcctgc agctgccacc cggctgcggc 2880
   gaccccaagc gcctcgggcc cctgcgcggc ttccagtggg ttacgggaga caacaacacc 2940
   agctatagca ggtgggcacg gctcgacctc aatggggctc ccctctgcgg cccgttgtgc 3000
   gtcgctgtct ccgctgctga ggccactgtg cccagcgagc cgatctggga ggagcagcag 3060
   tgcgaagtga aggccgatgg cttcctctgc gagttccact tcccagccac ctgcaggcca 3120
   ctggctgtgg agcccggcgc cgcggctgcc gccgtctcga tcacctacgg caccccgttc 3180
   gcggcccgcg gagcggactt ccaggcgctg ccggtgggca gctccgccgc ggtggctccc 3240
   ctcggcttac agctaatgtg caccgcgccg cccggagcgg tccaggggca ctgggccagg 3300
   gaggcgccgg gcgcttggga ctgcagcgtg gagaacggcg gctgcgagca cgcgtgcaat 3360
   gcgatccctg gggctccccg ctgccagtgc ccagccggcg ccgccctgca ggcagacggg 3420
   cgctcctgca ccgcatccgc gacgcagtcc tgcaacgacc tctgcgagca cttctgcgtt 3480
   cccaaccccg accagccggg ctcctactcg tgcatgtgcg agaccggcta ccggctggcg 3540
   gccgaccaac accggtgcga ggacgtggat gactgcatac tggagcccag tccgtgtccg 3600
   cagcgctgtg tcaacacaca gggtggcttc gagtgccact gctaccctaa ctacgacctg 3660
   gtggacggcg agtgtgtgga gcccgtggac ccgtgcttca gagccaactg cgagtaccag 3720
   tgccagcccc tgaaccaaac tagctacctc tgcgtctgcg ccgagggctt cgcgcccatt 3780
   ccccacgagc cgcacaggtg ccagatgttt tgcaaccaga ctgcctgtcc agccgactgc 3840
   gaccccaaca cccaggctag ctgtgagtgc cctgaaggct acatcctgga cgacggtttc 3900
   atctgcacgg acatcgacga gtgcgaaaac ggcggcttct gctccggggt gtgccacaac 3960
   ctccccggta ccttcgagtg catctgcggg cccgactcgg cccttgcccg ccacattggc 4020
   accgactgtg actccggcaa ggtggacggt ggcgacagcg gctctggcga gcccccgccc 4080
   agcccgacgc ccggctccac cttgactcct ccggccgtgg ggctcgtgca ttcgggcttg 4140
   ctcataggca tctccatcgc gagcctgtgc ctggtggtgg cgcttttggc gctcctctgc 4200
   cacctgcgca agaagcaggg cgccgccagg gccaagatgg agtacaagtg cgcggcccct 4260
   tccaaggagg tagtgctgca gcacgtgcgg accgagcgga cgccgcagag actctgagcg 4320
   gcctccgtcc aggagcctgg ctccgtccag gagcctgtgc ctcctcaccc ccagctttgc 4380
   taccaaagca ccttagctgg cattacagct ggagaagacc ctccccgcac cccccaagct 4440
   gttttcttct attccatggc taactggcga gggggtgatt agagggagga gaatgagcct 4500
   cggcctcttc cgtgacgtca ctggaccact gggcaatgat ggcaattttg taacgaagac 4560
   acagactgcg atttgtccca ggtcctcact accgggcgca ggagggtgag cgttattggt 4620
   cggcagcctt ctgggcagac cttgacctcg tgggctaggg atgactaaaa tatttatttt 4680
   ttttaagtat ttaggttttt gtttgtttcc tttgttctta cctgtatgtc tccagtatcc 4740
   actttgcaca gctctccggt ctctctctct ctacaaactc ccacttgtca tgtgacaggt 4800
   aaactatctt ggtgaatttt tttttcctag ccctctcaca tttatgaagc aagccccact 4860
   tattccccat tcttcctagt tttctcctcc caggaactgg gccaactcac ctgagtcacc 4920
   ctacctgtgc ctgaccctac ttcttttgct cttagctgtc tgctcagaca gaacccctac 4980
   atgaaacaga aacaaaaaca ctaaaaataa aaatggccat ttgctttttc accagatttg 5040
   ctaatttatc ctgaaatttc agattcccag agcaaaataa ttttaaacaa aggttgagat 5100
   gtaaaaggta ttaaattgat gttgctggac tgtcatagaa attacaccca aagaggtatt 5160
   tatctttact tttaaacagt gagcctgaat tttgttgctg ttttgatttg tactgaaaaa 5220
   tggtaattgt tgctaatctt cttatgcaat ttcctttttt gttattatta cttatttttg 5280
   acagtgttga aaatgttcag aaggttgctc tagattgaga gaagagacaa acacctccca 5340
   ggagacagtt caagaaagct tcaaactgca tgattcatgc caattagcaa ttgactgtca 5400
   ctgttccttg tcactggtag accaaaataa aaccagctct actggtcttg tggaattggg 5460
   agcttgggaa tggatcctgg aggatgccca attagggcct agccttaatc aggtcctcag 5520
   agaatttcta ccatttcaga gaggcctttt ggaatgtggc ccctgaacaa gaattggaag 5580
   ctgccctgcc catgggagct ggttagaaat gcagaatcct aggctccacc ccatccagtt 5640
   catgagaatc tatatttaac aagatctgca gggggtgtgt ctgctcagta atttgaggac 5700
   aaccattcca gactgcttcc aattttctgg aatacatgaa atatagatca gttataagta 5760
   gcaggccaag tcaggccctt attttcaaga aactgaggaa ttttctttgt gtagctttgc 5820
   tctttggtag aaaaggctag gtacacagct ctagacactg ccacacaggg tctgcaaggt 5880
   ctttggttca gctaagctag gaatgaaatc ctgcttcagt gtatggaaat aaatgtatca 5940
   tagaaatgta acttttgtaa gacaaaggtt ttcctcttct attttgtaaa ctcaaaatat 6000
   ttgtacatag ttatttattt attggagata atctagaaca caggcaaaat ccttgcttat 6060
   gacatcactt gtacaaaata aacaaataac aatgtg 6096
<210> 10
   <211> 2500
   <212> DNA
   <213> Homo sapiens
<400> 10
   acccacttct gtgtgtggat agtatcctgc aggagagatg ttgtctgcag tgtgagctgg 60
   gcccaccgga gtgtgtgaat aggatcctgc aggagaaatg gaatccggag tgtgagctgc 120
   atccgctgta gagggtggat aaaatcctgc aggaaagatg gcatctggaa tgtcagcggg 180
   agccaccgac ctctgaggat gcaccccgca ggtgtgatgc ggggccagtt ccaaggctgg 240
   gttaggtttt accctggctt ctgtgttgta ctctcattct cttcctcttt cttctaatac 300
   ctgctctggg aggcatcagg ccatgtccag tgtgcaggcc atggagaccc acacggcaag 360
   gaactggaac cccctgccag cagcctcggg ggtccagtcc ttagatggtg ccctgtggtc 420
   agcaatgcac ctgtgacctc cgggctatgt ctcgtggtag ttgcttttgt gttttaacat 480
   agcaacagga aactagccta ttacccacca atcccattcc aggctgcttt caaacgcagc 540
   tcaggctaga acaccagcac ggggacacag ctgagacttg gggtttgcga cgggaacacg 600
   cccatgctgt gcctctgaat ctggcaccgt caccctgtgg cctgggttca gcaacttggc 660
   ctcaccttcc ttgtctgtga aattcagact gggtccttgt gagatgattg gagagaatgt 720
   atgaactatg tgagaacgcc acctttgtgc gtatctcacg cagtgtcttc cctcctttcc 780
   aaagtcttct gctgtctcta gacacacccg acgtgggggg ggggggttcc ctgggtctcc 840
   tcctaggtct gtcccaggag ggcacgcact gaaggccgcg agaatcccgg gggctgcatt 900
   gcgccgcgcc aaggactcca cacaggacct ttcattttcc caactgtgct gagccaggcg 960
   gccggcagag agcaggtggc tgacaggccc cggggagccg gaccgcctgg gtctaatctt 1020
   cccgcagact cccttgctgt gcgctttggg gcttgggcct cagtttcctc aaaaggaatg 1080
   aggggctttt ttggaacgtt aaataatttc ctacgtggtt gcgggtaggg agaaggagaa 1140
   agagaggagc gcgcctgcgc gcctggaatc gtgcccggat cagagcaagc gctctaaaag 1200
   tgttacaaac attaaggcgc caactaaaaa acccgtagtg agcgcaggca gaaaccacgg 1260
   gtaagagaag tggagaagct tcgcgtaggc cccagggtcc cgagccccga gtctcgagcg 1320
   cagaatcagg ggtgccaatg ctctcctccg cgcccccgag cgctcgcctt ggccatgcgg 1380
   gccgccccac cgggatgagg gcgctcaggc cggacgctgg ggccccgggt tctcgccccg 1440
   ccccgccctc ggggattcag aggggccggg aggagcctcg cgcatgtgca cagctggcgc 1500
   cccccgcccc ccgcgcacag ctgggacgtg ggccgcggcc gggcgggcgc agtcgggagc 1560
   cggccgtggt ggctccgtgc gtccgagcgt ccgtccgcgc cgtcggccat ggccaagcgc 1620
   tccaggggcc ccgggcgccg ctgcctgttg gcgctcgtgc tgttctgcgc ctgggggacg 1680
   ctggccgtgg tggcccagaa gccgggcgca gggtgtccga gccgctgcct gtgcttccgc 1740
   accaccgtgc gctgcatgca tctgctgctg gaggccgtgc ccgccgtggc gccgcagacc 1800
   tccatcctgt gagtgccgcg ggggacgccg ggggcgcggg gtccggggct tcgtggagat 1860
   ccgggagcgc aggggtgatc ggaggtgggg ggcgcggagg gtggaggggg catcgggcgc 1920
   gcggggggcc tggggacttg ggacgcagaa gggaacctcc gaagggggac gtggggggac 1980
   ctgggcgcgg ggacccgctg ggcctttgtt cgccctgcgg gagacgccga ggggcggaac 2040
   agagcgctgt gcgcgcggcc ttcgtagccg cctttgttcg gaactcggaa tccccgcagg 2100
   actgggaagt tgttggagcc tccggggctc cccccgctcg cctcccgccg ccccctctca 2160
   tgctccgccg gcctcccgct tccccctggt tcgcggcccc tcctccgctc acctttcccc 2220
   cgctcaggac ccctcggtcc ccctccgctc cccgagcgcg gcgcagcccc ctccgtcctc 2280
   ccagccccct ccgccccgtt cctcgtcctg ttcgctcccc tcctccgctc ctcttcctcc 2340
   tccccttcct cctcctcctc cccttcctcc tcctcctccc cttcctcctc ctcctccctt 2400
   cccctcctcc tccccccctt ccttctcctc ccccagcctc cgccctctcc ccctcccccg 2460
   ccccttggag cgcagtgccc accccatccc cccgcgccgg 2500
<210> 11
   <211> 2200
   <212> DNA
   <213> Homo sapiens
<400> 11
   cctcgccccc tccagccggc cccccgggcc cctcctctcg gcgcccggac cttggccctc 60
   cctctccttt cccacttctc tctttgccct aacttcgccc ccatcccccg ctcatttcct 120
   ctcgcacccg ggctcgccaa tccctctttc caagtccctc ttccagcccg gccttcctct 180
   cgggttcgcc ccccttctcc ccaatctccg tcctcttccc tcccttcgcc ctccccccct 240
   tccttcctct tcccctcacc caaccctggt tcccctcgtt cctcagtccc gatctctccc 300
   ttactctgtc cccgcccact ctgcgccggc ctctcagtcc gggttgagcc ccacgtgtgg 360
   acggccgcgc ccccactgac agccgccgcc cgccggcccg ccccgcgccc cgccgggcct 420
   ctaaaacccc cgcgccgcgc cctccaccgc cgcatcttct ccagcgccca gcctcccgcc 480
   ctctctcttg ctggccgcac gccccggccc cgcgcacctc cgcccggctc cgcagccgct 540
   acccgcgctt cgttgccctg tgggactccg agcgagcccg gagggaaccc tcctcttctt 600
   ctgggggcga cttttgtttg cttgcctgtt tctttctggt gacttttgca gctttccaat 660
   atccgtcttc ggagcgcacg ggaatccgcc gagctctgcg tgcaggccct tttttctttt 720
   gaggttcaca ttttttgaaa ttttacgcca gggcttttgt aatttcctcc cccgcccgct 780
   gacggtcctg gagtcgctcg gggctttagg ccggttatgc aacgtgtacc gctcggggct 840
   gccggctgca cctccgccgc gcctcgccgc tcactgcgct agacccggcg ccccgcgtct 900
   cgcttcgcgg gcagtcaggg ggccggcgct ctgtcgaggt ctccagctag agcagggagc 960
   ccgagcccga gggagtcccc ggagccgacg aagggcttat tagaccctga ctcttttctg 1020
   aggcgcgcag attttgtctt tgatcactcc ctctccgcgg gtctacggcc gcgcgctttc 1080
   ggcgccggcg atggggagaa gacggaggct gtgtctccag ctctacttcc tgtggctggg 1140
   ctgtgtggtg ctctgggcgc agggcacggc cggccagcct cagcctcctc cgcccaagcc 1200
   gccccggccc cagccgccgc cgcaacaggt tcggtccgct acagcaggct ctgaaggcgg 1260
   gtttctagcg cccgagtatc gcgaggaggg tgccgcagtg gccagccgcg tccgccggcg 1320
   aggacagcag gacgtgctcc gagggtaagt gggcaagcgg ctccgcacct agggctccgg 1380
   cttgggggag gggggaatcc tcagtttggc ggctttctgg cccactccgt cccagaccct 1440
   ttagctggag cctagagctg cagccccctt tgccagaata tccaaagacc cccaggagcg 1500
   cgtccccctt ttccttccca accccgcagc tcagcgggcg gaaagccctc tctccggggg 1560
   ttgggcggcg ggtggttagg gggtccaggg gtgccgatcg cagagcgtgt gcagagctcg 1620
   cgctgcggga acaggttctg aatgtccggc ggcaggcggg cctgggtccg cctgctgcag 1680
   gggccagaga agcctgcttg ctccccacgt cggggccgcc gctcgtgagc cttttgtttg 1740
   aggacgtgtg cagggttcac agctcacctt ctcatcgtca acccgagcgc tccaccttgc 1800
   gacgcgcttt ccttgacacg tcggggccaa agtaacagtt gaccaaggag gaatggattt 1860
   gggaaggagg gcaaggattc tttggaacgg aatggtccct ttgttctctg catctggaag 1920
   ctagaatagt agcaaattat atgtttccat gcctcttttc gccctttaaa aaggcaggca 1980
   agggacgaca gatgaaaggc agtgtttaga catttctgac cctcctgcat tccagcatct 2040
   agctcttttg cttccacgtc tgcctcccga tctccaataa tttgaagtgt aattttgatt 2100
   tgtttgttgt cctgaaatct actcgctcgg ggcattgctt acgaagaccg tttatatgtt 2160
   gctgcatccc tctacctatc tgttacgtga ccgcgcttgt 2200
<210> 12
   <211> 2000
   <212> DNA
   <213> Homo sapiens
<400> 12
   ttggaagaaa aggatctccg aggaaggggc tgagagaagg gcagggtgaa ctggactaaa   60
   ggccagagta ggaaggagaa gaggggccaa aaaagaaggg gatgaaatta agcacagaag  120
   atgggtaaag aaaaaagtat cagggaaagg gcaaaataag agaaagcctt gaggataaga  180
   gggtagaagg ctaaagaaca aggggaccac tgggtcgggg aagcgctgcc tgaacggcgg  240
   gacagtgaca aagaaagggc gctggcgata ttcgcaccaa gggtgcgaaa cgcaatcggg  300
   aggtgagaaa tggaaagaag gcgaatgccc ggctacaagt agcctgggac tgaaagggga  360
   cctgggggag gggctgggcc cagggcagaa aagtccaggt tcccatgcgg cctgggccca  420
   cgtggagcgg gcgctgaatc accgttcagc cgcccccctc ccctcctccc cgaccggtgc  480
   ccgcagtccc cgcctcctcg gccgccgcct ccacggggcg gggccctggc ccgggaccag  540
   cgccgcggct ataaatgggc tgcggcgagg ccggcagaac gctgtgacag ccacacgccc  600
   caaggcctcc aagatgagct acacgttgga ctcgctgggc aacccgtccg cctaccggcg  660
   ggtaaccgag acccgctcga gcttcagccg cgtcagcggc tccccgtcca gtggcttccg  720
   ctcgcagtcg tggtcccgcg gctcgcccag caccgtgtcc tcctcctata agcgcagcat  780
   gctcgccccg cgcctcgctt acagctcggc catgctcagc tccgccgaga gcagccttga  840
   cttcagccag tcctcgtccc tgctcaacgg cggctccgga cccggcggcg actacaagct  900
   gtcccgctcc aacgagaagg agcagctgca ggggctgaac gaccgctttg ccggctacat  960
   agagaaggtg cactacctgg agcagcagaa taaggagatt gaggcggaga tccaggcgct 1020
   gcggcagaag caggcctcgc acgcccagct gggcgacgcg tacgaccagg agatccgcga 1080
   gctgcgcgcc accctggaga tggtgaacca cgagaaggct caggtgcagc tggactcgga 1140
   ccacctggag gaagacatcc accggctcaa ggagcgcttt gaggaggagg cgcggttgcg 1200
   cgacgacact gaggcggcca tccgcgcgct gcgcaaagac atcgaggagg cgtcgctggt 1260
   caaggtggag ctggacaaga aggtgcagtc gctgcaggat gaggtggcct tcctgcggag 1320
   caaccacgag gaggaggtgg ccgaccttct ggcccagatc caggcatcgc acatcacggt 1380
   ggagcgcaaa gactacctga agacagacat ctcgacggcg ctgaaggaaa tccgctccca 1440
   gctcgaaagc cactcagacc agaatatgca ccaggccgaa gagtggttca aatgccgcta 1500
   cgccaagctc accgaggcgg ccgagcagaa caaggaggcc atccgctccg ccaaggaaga 1560
   gatcgccgag taccggcgcc agctgcagtc caagagcatc gagctagagt cggtgcgcgg 1620
   caccaaggag tccctggagc ggcagctcag cgacatcgag gagcgccaca accacgacct 1680
   cagcagctac caggtaggaa ccgcggctgc gcggccagcc tgcgccagcg ccagcgccgc 1740
   gcgcccccga cacttgggct cgtgcccagg cgccctctcc gccgcgctcc ctggtggccg 1800
   ctcgctagag cacgcgcgcc gcagacctag ggtatttgcg gatcagcgtc ctcgcccatc 1860
   tcatcctcca cactccgccc ccacccacct gccccagctg ctaagggtct tgaccttttt 1920
   cagaaacgtg catcttttcc agttctaatt ttgcacgctt gcacgtttaa agcaggaggg 1980
   atgaattcgg tagtggataa                                             2000
<210> 13
   <211> 2300
   <212> DNA
   <213> Homo sapiens
<400> 13
   tcagattgtc attgggaggg tgaataaatg aatgcttgca ttatgagagt ttgggggcag   60
   aaatatgcca cagactctta tctgaagcca tcagatttag tggctgcgaa cccaccgaag  120
   tcagggattt acatttttta cagcaacgag agaaaacttc ccctttcctc tgcagaagtc  180
   aggactggat ctcaaaaata gaaatgtgtc ctcctaaatg tgtgcccatc cccgtggttg  240
   acaaacaacg gatttcccaa gatagctgcc acacacttgg tttctaatct ctgtattgct  300
   tccccgccag aatgtcgaag tccttcccga atatgcccag tcatactttc tgaacttttg  360
   agcaaacacc gtccggcttc ttgtgctttc ctcaaagacc ccaggcaccg gcagggagga  420
   cacaggccgg ggcagagcgc ccctgcgcgg gggattcctg ccactccgcg ccagcctgcg  480
   gcgcaaacgc tcttctcagc cgcagtccca cccgctgctg gcaatctgaa tgaggagccg  540
   cgctattttt acctccccgg ctgcaatcct ttatatttac atgcaggaag caaatatata  600
   agggattaag aaggagatgc gtggccttag tttatccaga gcaggaagag gttggaatag  660
   gagagggtat gtgaagtctg gggtggtgga aaaggcaggt ggacttcggc tggttgtttt  720
   ctcccgatca tccctgtctc tggcctggaa acccccgtac tctctttctt ctggcttatc  780
   cgtgactgcc ggctccccct ccaccgcccc catcttttga ggtaccaccc gtcacctccg  840
   atgctgcttg ggctgctgca tcactctgct gctttacccc cttccccgcc ccccaacaaa  900
   gcatgcgcag tgcgttccgg gccaggcaac agcagcagca cagcatccag caacagcatc  960
   agcacccgaa gccccgctcg ggcgcgctct cggggggcgg ggcgcacgcc cgctccgcgc 1020
   gtccccgcgc cgctcgctcc cgcgcgtccc cgcgccgctc gctcccgcgc gccgcctcag 1080
   catcctcagg cccggcggca gcccccgcag tcgctgaagc ggccgcgccc gccgggggag 1140
   ggagtagccg ctggggaggc tccaagttgg cggagcggcg aggacccctg gactcctctg 1200
   cgtcccgccc cgggagtggc tgcgaggcta ggcgagccgg gaaagggggc gccgcccagc 1260
   cccgagcccc gcgccccgtg ccccgagccc ggagccccct gcccgccgcg gcaccatgcg 1320
   cgccgagccg gcgtgaccgg ctccgcccgc ggccgccccg cagctagccc ggcgctctcg 1380
   ccggccacac ggagcggcgc ccgggagcta tgagccatga agccgcccgg cagcagctcg 1440
   cggcagccgc ccctggcggg ctgcagcctt gccggcgctt cctgcggccc ccaacgcggc 1500
   cccgccggct cggtgcctgc cagcgccccg gcccgcacgc cgccctgccg cctgcttctc 1560
   gtccttctcc tgctgcctcc gctcgccgcc tcgtcccggc cccgcgcctg gggggctgct 1620
   gcgcccagcg gtgggtatgg ccccgtgccc tttgcgttgg ctttcccgcg gggccctgca 1680
   gaggaaagcg aagggcgcgc gggtccgtgt gctccgggct tgtccccggc tcggcctttc 1740
   cttccctccc tgcctgtctt tccacccttc tcgttcccaa acccccattc atcccagttc 1800
   acttttggaa gtccatttct gttgcattcg cgaaaaaccc attccaattc ttgttggttc 1860
   cactgggagg tgtttagtgg atcctgggtc cctcagcgat ctctgtgcaa cttgcggagg 1920
   ggcaaccagt ggatgggaaa tacagcgagg gagcaagttg ctacttgcgt ggtggaacct 1980
   taatgtgaat gcggggagga tgtagtgata atagtggtaa tgggctgttt cctcaaattt 2040
   cgtatccggc gcattcagtg cggttggaat taaggtgggg gaggcacact tcggggacca 2100
   aagaattaag gtgctgaaga catacttcat gcacgacctt tggttctgat ttctcaaagt 2160
   gcttgtcatt ataatgaaca attaatataa taccatcttc tatatattga tgattggaag 2220
   tcactgaaag cagaaagctg gctttgtcag gaaaataaaa agaaattggg aagctgccag 2280
   catctgtatc cctacatggc                                              2300
<210> 14
   <211> 3000
   <212> DNA
   <213> Homo sapiens
<400> 14
   tactgccgac tttaggtctc tctggatctc aggccccctt ctctaagatg catcctagag   60
   gaccaaaaat acactttatt tgggcttcgc ctgcttttgt ggaagggtag tttactagag  120
   gatataatct cgtgttttaa tttgctctct ctcctaaagg aaatgtggag aaaaaaaaaa  180
   agcagaaatt ggaaataacc aatatttagt ttatttcatt cgattcttag gggaactggt   240
   gaggagccta agatgatttt cccttcctag agaaagaatc caaagtccag ggaaatagcg  300
   acaggggagt tcaagactgc ccctgctagt ccttccttgg ctactctccg ctgcgatcgc  360
   aggatagctc tcattagcag gagaatcggg caagtgtgtg gataagtaga gagtgtgttg  420
   aacaacttgt aacgttttat gaaatacgca ttgtcatggt tccctaaaag gctttgcgga  480
   agccgtttgt ctttactaat caagtcttta cttacacaaa agtagaagta gaagtagttt  540
   tagaaaacat actaacaatc ttctatcccc ttgaagacca gagtagcaga aaacaggtga  600
   tttgcattat aaaattgcac tcactttttc ctcctttcag atttcacatt acattagccc  660
   atttgtgtta cggtgtataa aaaatggaac aggcgcctcc actgcattgt tctcctttaa 720
   aaatagatca cttacaccct aactttgttt tccttaaatt cgattcttaa caggagagct 780
   ttctattatt tcagatggag tgaggttgca cgactgggat ggaagaaagg aatcccttaa 840
   atttggggga atttctgttc tctgttccaa gaccatttta cttggggtgt gggggtgggc 900
   gcggcggtca gggcagtgga acgcagtcgc ggctgcgcca tccctgcact tccaggcgcg 960
   cgggagggac cggcggggac gcgagctgcg gactctggcg aactcggggg aggcagacag 1020
   ggggaggcgg acacccagcc ggcaggcgtc tcagcctccc cgcagccggc gggcttttct 1080
   cctgacagct ccaggaaagg cagacccctt ccccagccag ccaggtaagg taaagactgc 1140
   tgttgagctt gctgttactg agggcgcaca gaccctgggg agaccgaagc ttgccactgc 1200
   gggattctgt ggggtaacct gggtctacgg aagtttcctg aaagagggga gaagggtttg 1260
   catttttcct atggaggatt cttctctctc tagcatttcg tttgatgtat tcaactggta 1320
   gaagtgagat ttcaacaggt agcagagagc gctcacgtgg aggaggtttg gggcgccgcg 1380
   gcgccacccc cacccctcct cgggaccgcg cctatttcta aagttacacg tcgacgaact 1440
   aacctatgct ttaaattcct ctttccagcc ccgtgagtcc gcggcgacat tgggccgtgg 1500
   ggtggctggg aacggtcccc tcctccggaa aaaccagaga acggcttgga gagctgaaac 1560
   gagcgtccgc gagcaggtcc gtgcagaacc gggcttcagg accgctgagc tccgtagggc 1620
   gtccttgggg gacgccaggt cgccggctcc tctgccctcg ttgagatgga caacgcctcg 1680
   ttctcggagc cctggcccgc caacgcatcg ggcccggacc cggcgctgag ctgctccaac 1740
   gcgtcgactc tggcgccgct gccggcgccg ctggcggtgg ctgtaccagt tgtctacgcg 1800
   gtgatctgcg ccgtgggtct ggcgggcaac tccgccgtgc tgtacgtgtt gctgcgggcg 1860
   ccccgcatga agaccgtcac caacctgttc atcctcaacc tggccatcgc cgacgagctc 1920
   ttcacgctgg tgctgcccat caacatcgcc gacttcctgc tgcggcagtg gcccttcggg 1980
   gagctcatgt gcaagctcat cgtggctatc gaccagtaca acaccttctc cagcctctac 2040
   ttcctcaccg tcatgagcgc cgaccgctac ctggtggtgt tggccactgc ggagtcgcgc 2100
   cgggtggccg gccgcaccta cagcgccgcg cgcgcggtga gcctggccgt gtgggggatc 2160
   gtcacactcg tcgtgctgcc cttcgcagtc ttcgcccggc tagacgacga gcagggccgg 2220
   cgccagtgcg tgctagtctt tccgcagccc gaggccttct ggtggcgcgc gagccgcctc 2280
   tacacgctcg tgctgggctt cgccatcccc gtgtccacca tctgtgtcct ctataccacc 2340
   ctgctgtgcc ggctgcatgc catgcggctg gacagccacg ccaaggccct ggagcgcgcc 2400
   aagaagcggg tgaccttcct ggtggtggca atcctggcgg tgtgcctcct ctgctggacg 2460
   ccctaccacc tgagcaccgt ggtggcgctc accaccgacc tcccgcagac gccgctggtc  2520
   atcgctatct cctacttcat caccagcctg agctacgcca acagctgcct caaccccttc  2580
   ctctacgcct tcctggacgc cagcttccgc aggaacctcc gccagctgat aacttgccgc 2640
   gcggcagcct gactccccca gcgtccggct ccgcaactgc ccgccactcc tggccagcga 2700
   gggaggagcc ggcgccagag tgcgggacca gacaggccgc ctaggcctcc tggggaaacc 2760
   gactcgcgcc ccatacccga cctagcagat cggaagcgct gcgactgtgc ccgcaggttg 2820
   accttgccaa gccctccagg tgatgcgcgg ccatgccggg tgaggagaac tgaggctgag 2880
   atcgccacac tgagggctcc ctaaagccga ggtggaggaa gaggagggta gaggaggagg 2940
   gcggtattgc tgggaaccgc cccctccctg ccctgctccc tgctgcccca cccgagccct 3000
<210> 15
   <211> 3000
   <212> DNA
   <213> Homo sapiens
<400> 15
   gaatacatta aagtaggggc aacccttgag cccagacttc tgccatgtga agaccctttg   60
   aaaatcctga caaacacagg tactgcgtaa gtggtcagct aattaaagag gggaggtgga  120
   gctgtccttt gtgtatccaa taagtaccca ttatctcatt tgagcatgaa aagaggccac  180
   tgttattact ttcaagaagg aaagtaagca ggatagctca tatttttaga accattcctc  240
   accaaatgga ataattccgg tgaaaagtgg gagtgaggaa gaaagaaaaa aaaaacttct  300
   aatcataatg tttgggaata agaaaggaag aagaaactca cgtcaaagcc gactttctcc  360
   tgcagctgta aaataaactc ttaagaccct tcctgctgaa actctggaga ggaaaactgg  420
   agtggcgggt gggctttgcc tgcagctcaa ctctccctcg cggcgcgggc gcggctgggt  480
   tcagcacctc ggaaagcgcc cctcgcggcg ccccgggatt acgcatgctc cttggggccc  540
   gccgccttgg ccgtgcaagt gccaccgtaa ctggtgagag ccgctggcaa cccacccgga  600
   gttgacaacc gcggagagac gcagacaccc actgacctcc aggaagctga gcgtggtgga  660
   tggaactcta cgatctcttt ctctccaagg acggaaacct catccaagca gtcccagagg  720
   aaacggataa aggtatttga aagggagcga gcggccccaa atcgcacaat tgagcggctg  780
   ggggagttat gcgccagtgc cccagtgacc gcgggacacg gagaggggaa gtctgcgttg  840
   tacataagga cctagggact ccgagcttgg cctgagaacc cttggacgcc gagtgcttgc  900
   cttacgggct gcactcctca actctgctcc aaagcagccg ctgagctcaa ctcctgcgtc  960
   cagggcgttc gctgcgcgcc aggacgcgct tagtacccag ttcctgggct ctctcttcag 1020
   tagctgcttt gaaagctccc acgcacgtcc cgcaggctag cctggcaaca aaactggggt 1080
   aaaccgtgtt atcttaggtc ttgtccccca gaacatgacc tagaggtacc tgcgcatgca 1140
   gatggccgat gcagccacga tagccaccat gaataaggca gcaggcgggg acaagctagc 1200
   agaactcttc agtctggtcc cggaccttct ggaggcggcc aacacgagtg gtaacgcgtc 1260
   gctgcagctt ccggacttgt ggtgggagct ggggctggag ttgccggacg gcgcgccgcc 1320
   aggacatccc ccgggcagcg gcggggcaga gagcgcggac acagaggccc gggtgcggat 1380
   tctcatcagc gtggtgtact gggtggtgtg cgccctgggg ttggcgggca acctgctggt 1440
   tctctacctg atgaagagca tgcagggctg gcgcaagtcc tctatcaacc tcttcgtcac 1500
   caacctggcg ctgacggact ttcagtttgt gctcaccctg cccttctggg cggtggagaa 1560
   cgctcttgac ttcaaatggc ccttcggcaa ggccatgtgt aagatcgtgt ccatggtgac 1620
   gtccatgaac atgtacgcca gcgtgttctt cctcactgcc atgagtgtga cgcgctacca 1680
   ttcggtggcc tcggctctga agagccaccg gacccgagga cacggccggg gcgactgctg 1740
   cggccggagc ctgggggaca gctgctgctt ctcggccaag gcgctgtgtg tgtggatctg 1800
   ggctttggcc gcgctggcct cgctgcccag tgccattttc tccaccacgg tcaaggtgat 1860
   gggcgaggag ctgtgcctgg tgcgtttccc ggacaagttg ctgggccgcg acaggcagtt 1920
   ctggctgggc ctctaccact cgcagaaggt gctgctgggc ttcgtgctgc cgctgggcat 1980
   cattatcttg tgctacctgc tgctggtgcg cttcatcgcc gaccgccgcg cggcggggac 2040
   caaaggaggg gccgcggtag ccggaggacg cccgaccgga gccagcgccc ggagactgtc 2100
   gaaggtcacc aaatcagtga ccatcgttgt cctgtccttc ttcctgtgtt ggctgcccaa 2160
   ccaggcgctc accacctgga gcatcctcat caagttcaac gcggtgccct tcagccagga 2220
   gtatttcctg tgccaggtat acgcgttccc tgtgagcgtg tgcctagcgc actccaacag 2280
   ctgcctcaac cccgtcctct actgcctcgt gcgccgcgag ttccgcaagg cgctcaagag 2340
   cctgctgtgg cgcatcgcgt ctccttcgat caccagcatg cgccccttca ccgccactac 2400
   caagccggag cacgaggatc aggggctgca ggccccggcg ccgccccacg cggccgcgga 2460
   gccggacctg ctctactacc cacctggcgt cgtggtctac agcggggggc gctacgacct 2520
   gctgcccagc agctctgcct actgacgcag gcctcaggcc cagggcgcgc cgtcggggca 2580
   aggtggcctt ccccgggcgg taaagaggtg aaaggatgaa ggagggctgg ggggggcccc 2640
   atttaagaag taggtgggag gaggatgggc agagcatgga ggaggagcct gtggataggc 2700
   cgaggacctt ctctggagag gagatgcttc gaaatcaggt ggagagagga aattggcaaa 2760
   gggatagaga cgagccccac gggccagaca gccaacctcc gctccgcacc ccacagcctc 2820
   tccttactct tcccacgctg agtagtgtgg gggcgcccag aagcgaagac aagcagcaaa 2880
   aatgtagaga aattggcacg gggagcgggg cttagccaaa tgatgcacag acaattgtgc 2940
   ccgtttattc cagcgacttc tgcggagagg gcagccgtcg gcacaaacac tcctttgcgt 3000
<210> 16
   <211> 2200
   <212> DNA
   <213> Homo sapiens
<400> 16
   gtcccccgat tccctcaccc atcatataac gtgtgtattt attatgtttc ccgtttcctc   60
   tgtctccgcc agcagaatgt aaactccatg aggtcaggaa tctccgagtt atgttgcgcc  120
   agtgtaatcc aagagcccgg aacagtgcct ggcacacagc gggcatatgg aagaacaaat  180
   gtgtgaaggt gtgaatgaat gaataattga aagaataaat agtagttctc agcctcacag  240
   aacacgggtc acaacctcaa atgacctgct accctgccca taaataacag agatgcagga  300
   gtaagtgctg ggctgtgacc tgtcaacatg ctaagccgct caaacaaaac tgcccaacag  360
   cccgctggcc gcctatttgc agcactgggc cctgagccgc acattcccat ttcgttgata  420
   aagaaactga ccagatagtt taagtggcct gctgcggaag acagagctgg tgctgcaccg  480
   gtcgctgctt ccccagtcct tttttggcct cctttctgac gcgacgcaga ccccagttct  540
   ggagagtctg tcactcgctc cccgtggtgg gagatcagag gcctggtgtc cttgggagcg  600
   gcgagcggtg ctcggcgcag gatagaaagg gagtgcgcgc ccgagtcccc cagatccctg  660
   ggaacccgcg ccaccctccc gcccctgccc atccccggcc gcgctgtcag tctccattag  720
   cgctaacagg ctccagacgg agcgggccgg gcgctgggtt aatgcaatcg gcgcgttacc  780
   tggggcgcag gctacattac cagcccggcc cccgccaggc acggccagaa ccagtcagcc  840
   cgcgccctgc cggccgcccc gcgcctccag ctcttccccg gccccgcccg aacgccacac  900
   ggcggagccc agccccagcc cgcgccctag agcctgccaa ggcgccgccg gtcgggggcc  960
   ggcagggcgc aaggcaccag ggatcccctc gccgccggac acgtgagtgc gccctgagcg 1020
   cgggacaggg ctaggtctgc ctgggaggcc cgggccgaga cgcgccagca gagggctagc 1080
   gagtttgtag tgcagtgacg ttaagtgtcc gagaaggctc ctgtggctgt tgaagtgtcg 1140
   cggacctgag ctggggaggg ggtcggcacg ctgccctcag cctcggtgag ttcaatccca 1200
   gccatttggg gcaggcgaga gtgggtgaac gaggaaaagt gctgcagggt cttcagccgc 1260
   ccccagaggg ctgtcagaag tctccaactc ttgagttccg gcgtgcccca acctctgttt 1320
   ccaaattttt ccagcggacg cgcgctcttt tctgggaacc ctgcgtccgc tcagcgcgcg 1380
   ctcatcccag tgtctaaggc gctcccgggt ggtcttggga gttgcaagta gggaggaacg 1440
   gccgggtaac cacctctttt ccctttatcc aagcagagcc tcggcgtgcc cccaggaccg 1500
   gtaaagttcc tctcgccagc cgcatccatg cttctggcgc ggatgaaccc gcaggtgcag 1560
   cccgagaaca acggggcgga cacgggtcca gagcagcccc ttcgggcgcg caaaactgcg 1620
   gagctgctgg tggtgaagga gcgcaacggc gtccagtgcc tgctggcgcc ccgcgacggc 1680
   gacgcgcagc cccgggagac ctggggcaag aagatcgact tcctgctgtc cgtagtcggc 1740
   ttcgcagtgg acctggccaa cgtgtggcgc ttcccctacc tctgctacaa gaacggcggc 1800
   ggtgagcgtg gggtcgggct gggaatttga atctgggagg tccactgtct gcagcggtgg 1860
   ctgggacagg agctggaata cacacggaag ggaggcgagg agacaggggc aaatctgggg 1920
   cgcagaaaga actggacagg gctaacggga aaaaaaaaag attggagtcc tctggaaggt 1980
   catttcccca ggctctttgc agagtacctc gagctcattc cagcggaagt gtcaggattg 2040
   ggcaccctgg aagcaaaaca gcagaagagt gaaatcgagt catgacccta aagtcatggt 2100
   aggggtatgg atggaaagga cagaatctgg ggtgccaggt tgggtggggg agcctgacct 2160
   tttgatggtc tgctggaagg gaggtggaga ttccaagagc                       2200
<210> 17
   <211> 98
   <212> DNA
   <213> Artificial Sequence
<220>
   <221> modified_base
   <222> 20,23,31,46,51,53,55
   <223> Designed oligonucleotide consisting of objective DNA domain (GPR7-2079-2176), n=m5c
<400> 17
   gttggccact gcggagtcgn gcngggtggc nggccgcacc tacagngccg ngngngcggt   60
   gagcctggcc gtgtggggga tcgtcacact cgtcgtgc                           98
<210> 18
   <211> 98
   <212> DNA
   <213> Artificial Sequence
<220>
   <221> modified_base
   <222> 31, 46, 51, 53, 55
   <223> Designed oligonucleotide consisting of objective DNA domain (GPR7-2079-2176),n=m5c
<400> 18
   gttggccact gcggagtcgc gccgggtggc nggccgcacc tacagngccg ngngngcggt   60
   gagcctggcc gtgtggggga tcgtcacact cgtcgtgc                            98
<210> 19
   <211> 98
   <212> DNA
   <213> Artificial Sequence
<220>
   <223> Designed oligonucleotide consisting of objective DNA domain (GPR7-2079-2176)
<400> 19
   gttggccact gcggagtcgc gccgggtggc cggccgcacc tacagcgccg cgcgcgcggt   60
   gagcctggcc gtgtggggga tcgtcacact cgtcgtgc                            98
<210> 20
   <211> 14
   <212> DNA
   <213> Artificial Sequence
<220>
   <223> Designed oligonucleotide for experiment
<400> 20
   gccacccggc gcga 14
<210> 21
   <211> 19
   <212> DNA
   <213> Artificial Sequence
<220>
   <223> Designed oligonucleotide primer for PCR
<400> 21
   gttggccact gcggagtcg 19
<210> 22
   <211> 20
   <212> DNA
   <213> Artificial Sequence
<220>
   <223> Designed oligonucleotide primer for PCR
<400> 22
   gcacgacgag tgtgacgatc 20
<210> 23
   <211> 98
   <212> DNA
   <213> Artificial Sequence
<220>
   <223> Designed oligonucleotide consisting of objective DNA domain (GPR7-2079-2176)
<400> 23
   gttggccact gcggagtcgc gccgggtggc cggccgcacc tacagcgccg cgcgcgcggt    60
   gagcctggcc gtgtggggga tcgtcacact cgtcgtgc                            98
<210> 24
   <211> 18
   <212> DNA
   <213> Artificial Sequence
<220>
   <223> Designed oligonucleotide primer for PCR
<400> 24
   ctcagcaccc aggcggcc 18
<210> 25
   <211> 20
   <212> DNA
   <213> Artificial Sequence
<220>
   <223> Designed oligonucleotide primer for PCR
<400> 25
   ctggccaaac tggagatcgc 20
<210> 26
   <211> 386
   <212> DNA
   <213> Artificial Sequence
<220>
   <223> Amplified oligonucleotide consisting of objective DNA domain ( Genbank
   Accession No.NT_029419 25687390-25687775 Homo sapiens )
<400> 26
   ctcagcaccc aggcggccgc gatcatgagg cgcgagcggc gcgcgggctg ttgcagagtc    60
   ttgagcgggt ggcacaccgc gatgtagcgg tcggctgtca tgactaccag catgtaggcc   120
   gacgcaaaca tgccgaacac ctgcaggtgc ttcaccacgc ggcacagcca gtcggggccg   180
   cggaagcggt aggtgatgtc ccagcacatt tgcggcagca cctggaagaa tgccacggcc   240
   aggtcggcca ggctgaggtg tcggatgaag aggtgcatgc gggacgtctt gcgcggcgtc   300
   cggtgcagag ccagcagtac gctgctgttg cccagcacgg ccaccgcgaa agtcaccgcc   360
   agcacggcga tctccagttt ggccag                                        386
<210> 27
   <211> 386
   <212> DNA
   <213> Artificial Sequence
<220>
   <221> modified_base
   <222> 14,18,20,31,33,38,40,42,44,66,78,80,88,92,120,123,134,160,173,179,181,187,20 3,237,245,262,280,285,292,294,297,301,320,338,346,357,365,368
   <223> Designed oligonucleotide consisting of objective DNA domain ( Genbank Accession No.NT_029419 25687390-25687775 Homo sapiens ), n=m5c
<400> 27
   ctcagcaccc aggnggcngn gatcatgagg ngngagnggn gngngggctg ttgcagagtc   60
   ttgagngggt ggcacacngn gatgtagngg tnggctgtca tgactaccag catgtaggcn  120
   gangcaaaca tgcngaacac ctgcaggtgc ttcaccangn ggcacagcca gtnggggcng  180
   nggaagnggt aggtgatgtc ccagcacatt tgnggcagca cctggaagaa tgccanggcc  240
   aggtnggcca ggctgaggtg tnggatgaag aggtgcatgn gggangtctt gngnggngtc  300
   nggtgcagag ccagcagtan gctgctgttg cccagcangg ccacngngaa agtcacngcc  360
   agcanggnga tctccagttt ggccag                                         386
<210> 28
   <211> 386
   <212> DNA
   <213> Artificial Sequence
<220>
   <223> Designed oligonucleotide consisting of objective DNA domain ( Genbank
   Accession No.NT_029419 25687390-25687775 Homo sapiens )
<400> 28
   ctcagcaccc aggcggccgc gatcatgagg cgcgagcggc gcgcgggctg ttgcagagtc   60
   ttgagcgggt ggcacaccgc gatgtagcgg tcggctgtca tgactaccag catgtaggcc  120
   gacgcaaaca tgccgaacac ctgcaggtgc ttcaccacgc ggcacagcca gtcggggccg  180
   cggaagcggt aggtgatgtc ccagcacatt tgcggcagca cctggaagaa tgccacggcc  240
   aggtcggcca ggctgaggtg tcggatgaag aggtgcatgc gggacgtctt gcgcggcgtc  300
   cggtgcagag ccagcagtac gctgctgttg cccagcacgg ccaccgcgaa agtcaccgcc  360
   agcacggcga tctccagttt ggccag                                         386
<210> 29
   <211> 30
   <212> DNA
   <213> Artificial Sequence
<220>
   <223> Designed counter oligonucleotide for making an objective DNA domain a
   single strand DNA
<400> 29
   gccaccgcga aagtcaccgc cagcacggcg 30
<210> 30
   <211> 30
   <212> DNA
   <213> Artificial Sequence
<220>
   <223> Designed counter oligonucleotide for making an objective DNA domain a
   single strand DNA
<400> 30
   gccagcagta cgctgctgtt gcccagcacg 30
<210> 31
   <211> 30
   <212> DNA
   <213> Artificial Sequence
<220>
   <223> Designed counter oligonucleotide for making an objective DNA domain a
   single strand DNA
<400> 31
   cgggacgtct tgcgcggcgt ccggtgcaga 30
<210> 32
   <211> 30
   <212> DNA
   <213> Artificial Sequence
<220>
   <223> Designed counter oligonucleotide for making an objective DNA domain a
   single strand DNA
<400> 32
   aggctgaggt gtcggatgaa gaggtgcatg 30
<210> 33
   <211> 30
   <212> DNA
   <213> Artificial Sequence
<220>
   <223> Designed counter oligonucleotide for making an objective DNA domain a
   single strand DNA
<400> 33
   acctggaaga atgccacggc caggtcggcc 30
<210> 34
   <211> 30
   <212> DNA
   <213> Artificial Sequence
<220>
   <223> Designed counter oligonucleotide for making an objective DNA domain a
   single strand DNA
<400> 34
   taggtgatgt cccagcacat ttgcggcagc 30
<210> 35
   <211> 30
   <212> DNA
   <213> Artificial Sequence
<220>
   <223> Designed counter oligonucleotide for making an objective DNA domain a
   single strand DNA
<400> 35
   cggcacagcc agtcggggcc gcggaagcgg 30
<210> 36
   <211> 30
   <212> DNA
   <213> Artificial Sequence
<220>
   <223> Designed counter oligonucleotide for making an objective DNA domain a
   single strand DNA
<400> 36
   atgccgaaca cctgcaggtg cttcaccacg 30
<210> 37
   <211> 30
   <212> DNA
   <213> Artificial Sequence
<220>
   <223> Designed counter oligonucleotide for making an objective DNA domain a
   single strand DNA
<400> 37
   atgactacca gcatgtaggc cgacgcaaac 30
<210> 38
   <211> 30
   <212> DNA
   <213> Artificial Sequence
<220>
   <223> Designed counter oligonucleotide for making an objective DNA domain a
   single strand DNA
<400> 38
   tggcacaccg cgatgtagcg gtcggctgtc 30
<210> 39
   <211> 30
   <212> DNA
   <213> Artificial Sequence
<220>
   <223> Designed counter oligonucleotide for making an objective DNA domain a
   single strand DNA
<400> 39
   cgcgcgggct gttgcagagt cttgagcggg 30
<210> 40
   <211> 30
   <212> DNA
   <213> Artificial Sequence
<220>
   <223> Designed counter oligonucleotide for making an objective DNA domain a
   single strand DNA
<400> 40
   caggcggccg cgatcatgag gcgcgagcgg 30
<210> 41
   <211> 19
   <212> DNA
   <213> Artificial Sequence
<220>
   <223> Designed oligonucleotide primer for PCR
<400> 41
   tgagctccgt agggcgtcc 19
<210> 42
   <211> 17
   <212> DNA
   <213> Artificial Sequence
<220>
   <223> Designed oligonucleotide primer for PCR
<400> 42
   gcgccgggtc cgggccc 17
<210> 43
   <211> 121
   <212> DNA
   <213> Artificial Sequence
<220>
   <223> Amplified oligonucleotide consisting of objective DNA domain ( Genbank
   Accession No.AC009800 76606-76726 Homo sapiens )
<220>
<400> 43
   gcgccgggtc cgggcccgat gcgttggcgg gccagggctc cgagaacgag gcgttgtcca 60
   tctcaacgag ggcagaggag ccggcgacct ggcgtccccc aaggacgccc tacggagctc 120
   a 121
<210> 44
   <211> 121
   <212> DNA
   <213> Artificial Sequence
<220>
   <221> modified base
   <222> 2,5,11,17,22,28,41,47,52,67,82,85,93,106,113
   <223> Designed oligonucleotide consisting of objective DNA domain ( Genbank
   Accession No.AC009800 76606-76726 Homo sapiens ) n=m5c
<400> 44
   gngcngggtc ngggccngat gngttggngg gccagggctc ngagaangag gngttgtcca 60
   tctcaangag ggcagaggag cnggngacct ggngtccccc aaggangccc tanggagctc 120
   a 121
<210> 45
   <211> 121
   <212> DNA
   <213> Artificial Sequence
<220>
   <223> Designed oligonucleotide consisting of objective DNA domain ( Genbank
   Accession No.AC009800 76606-76726 Homo sapiens)
<400> 45
   gcgccgggtc cgggcccgat gcgttggcgg gccagggctc cgagaacgag gcgttgtcca    60
   tctcaacgag ggcagaggag ccggcgacct ggcgtccccc aaggacgccc tacggagctc   120
   a                                                                   121
<210> 46
   <211> 30
   <212> DNA
   <213> Artificial Sequence
<220>
   <223> Designed counter oligonucleotide for making an objective DNA domain a
   single strand DNA
<400> 46
   gcgtccccca aggacgccct acggagctca 30
<210> 47
   <211> 30
   <212> DNA
   <213> Artificial Sequence
<220>
   <223> Designed counter oligonucleotide for making an objective DNA domain a
   single strand DNA
<400> 47
   ctcaacgagg gcagaggagc cggcgacctg 30
<210> 48
   <211> 30
   <212> DNA
   <213> Artificial Sequence
<220>
   <223> Designed counter oligonucleotide for making an objective DNA domain a
   single strand DNA
<400> 48
   cgccgggtcc gggcccgatg cgttggcggg 30
<210> 49
   <211> 21
   <212> DNA
   <213> Artificial Sequence
<220>
   <223> Designed oligonucleotide primer for PCR
<400> 49
   ggacctgtgt ttgacgggta t 21
<210> 50
   <211> 21
   <212> DNA
   <213> Artificial Sequence
<220>
   <223> Designed oligonucleotide primer for PCR
<400> 50
   agtacagatc tggcgttctc g 21
<210> 51
   <211> 117
   <212> DNA
   <213> Artificial Sequence
<220>
   <223> Amplified oligonucleotide consisting of objective DNA domain (Genbank
   Accession No.NC001139 384569-384685 Saccharomyces cereviciae chromosome VII)
<400> 51
   ggacctgtgt ttgacgggta taacactaag ttgcgcaatt tgctgtattg cgaaatccgc    60
   ccggacgata tcactcttga gcgcatgtgc cgtttccgag aacgccagat ctgtact      117
<210> 52
   <211> 117
   <212> DNA
   <213> Artificial Sequence
<220>
   <221> modified base
   <222> 15,34,51,58,61,66,82,91,97,lD3
   <223> Designed oligonucleotide consisting of objective DNA domain (Genbank Accession No.NC001139 384569-384685 Saccharomyces cereviciae chromosome VII) n=m5c
<400> 52
   ggacctgtgt ttgangggta taacactaag ttgngcaatt tgctgtattg ngaaatcngc    60
   cnggangata tcactcttga gngcatgtgc ngtttcngag aangccagat ctgtact      117
<210> 53
   <211> 117
   <212> DNA
   <213> Artificial Sequence
<220>
   <223> Designed oligonucleotide consisting of objective DNA domain (Genbank
   Accession No.NC001139 384569-384685 Saccharomyces cereviciae chromosome VII)
<400> 53
   ggacctgtgt ttgacgggta taacactaag ttgcgcaatt tgctgtattg cgaaatccgc    60
   ccggacgata tcactcttga gcgcatgtgc cgtttccgag aacgccagat ctgtact      117
<210> 54
   <211> 21
   <212> DNA
   <213> Artificial Sequence
<220>
   <223> Designed counter oligonucleotide for making an objective DNA domain a
   single strand DNA
<400> 54
   ggacctgtgt ttgacgggta t 21
<210> 55
   <211> 25
   <212> DNA
   <213> Artificial Sequence
<220>
   <223> Designed counter oligonucleotide for making an objective DNA domain a
   single strand DNA
<400> 55
   aacactaagt tgcgcaattt gctgt 25
<210> 56
   <211> 25
   <212> DNA
   <213> Artificial Sequence
<220>
   <223> Designed counter oligonucleotide for making an objective DNA domain a
   single strand DNA
<400> 56
   attgcgaaat ccgcccggac gatat 25
<210> 57
   <211> 25
   <212> DNA
   <213> Artificial Sequence
<220>
   <223> Designed counter oligonucleotide for making an objective DNA domain a
   single strand DNA
<400> 57
   cactcttgag cgcatgtgcc gtttc 25
<210> 58
   <211> 244
   <212> DNA
   <213> Artificial Sequence
<220>
   <223> Designed oligonucleotide primer for PCR
<400> 58
   taggaaatac attccgaggg cgcccgcaca aggcctatta ttagagggac ctgtgtttga 60
   cgggtataac actaagttgc gcaatttgct gtattgcgaa atccgcccgg acgatatcac 120
   tcttgagcgc atgtgccgtt tccgagaacg ccagatctgt actgcgatcg cacacgagga 180
   gacacagcgt cacgtgtttt gccattttgt acgacaaatg aaccgcctgg ccacgcctct 240
   aatc 244
<210> 59
   <211> 30
   <212> DNA
   <213> Artificial Sequence
<220>
   <223> Designed counter oligonucleotide for making an objective DNA domain a
   single strand DNA
<400> 59
   aatacattcc gagggcgccc gcacaaggcc 30
<210> 60
   <211> 21
   <212> DNA
   <213> Artificial Sequence
<220>
   <223> Designed counter oligonucleotide for making an objective DNA domain a
   single strand DNA
<400> 60
   gcgatcgcac acgaggagac a 21
<210> 61
   <211> 30
   <212> DNA
   <213> Artificial Sequence
<220>
   <223> Designed counter oligonucleotide for making an objective DNA domain a
   single strand DNA
<400> 61
   agcgtcacgt gttttgccat tttgtacgac 30
<210> 62
   <211> 29
   <212> DNA
   <213> Artificial Sequence
<220>
   <223> Designed counter oligonucleotide for making an objective DNA domain a
   single strand DNA
<400> 62
   aaatgaaccg cctggccacg cctctaatc 29

## Description

### TECHNICAL FIELD

The present invention relates to a method of measuring the content of methylated DNA in a DNA region of interest in a genomic DNA contained in a biological specimen, and so on.

### BACKGROUND ART

As a method for evaluating the methylation state of DNA in an objective DNA region in a genomic DNA contained in a biological specimen, for example, there is known a method of measuring the content of methylated DNA in an objective DNA region in a genomic DNA (see, for example, Nucleic Acids Res., 1994, Aug 11; 22(15): 2990-7, and Proc. Natl. Acad. Sci. U.S.A., 1997, Mar 18; 94 (6) : 2284-9 for reference). In such a measuring method, first, it is necessary to extract DNA containing the objective DNA region from a DNA sample derived from a genomic DNA, and the extracting operation is complicated.

As a method of measuring the content of methylated DNA in an objective region of extracted DNA, for example, (1) a method of amplifying an objective region by subjecting the DNA to a chain reaction for DNA synthesis by DNA polymerase after modification of the DNA with a sulfite or the like (Polymerase Chain Reaction; hereinafter also referred to as PCR), and (2) a method of amplifying an objective region by subjecting the DNA to PCR after digestion of the DNA using a methylation sensitive restriction enzyme are known. Both of these methods require time and labor for DNA modification for detection of methylation, subsequent purification of the product, preparation of a reaction system for PCR, and checking of DNA amplification.

WO 2007/081791 relates to a method of detecting DNA methylation.

### DISCLOSURE OF THE INVENTION

It is an object of the present invention to provide a method of measuring the content of methylated DNA in an objective DNA region in a genomic DNA contained in a biological specimen in a simple and convenient manner.

That is, the present invention provides the following:
[1] A method of measuring the content of methylated DNA in a objective DNA region in a genomic DNA contained in a biological specimen, the objective DNA region having a recognition site of at least one kind of methylation sensitive restriction enzyme, comprising:
   (1) First step of mixing a DNA sample comprising a single-stranded DNA (plus strand) containing the objective DNA region and a masking oligonucleotide comprising a nucleotide sequence complementary to a nucleotide sequence of a recognition site for the at least one kind of methylation-sensitive restriction enzyme, thereby obtaining a partially single-stranded DNA in which the recognition site for the methylation-sensitive restriction enzyme forms double strand by complementary base-pairing with the masking oligonucleotide, followed by digesting the DNA sample with the methylation-sensitive restriction enzyme wherein said enzyme digests a recognition site containing unmethylated cytosine and does not digest a recognition site containing methylated cytosine;
   (2) Second step of obtaining methylated single-stranded DNA from the DNA sample that has been subjected to the digestion treatment in First step, by separating methylated double-stranded DNA contained in the DNA sample into methylated single-stranded DNA, and binding the methylated single-stranded DNA to an immobilized methylated DNA antibody, thereby selecting the single-stranded DNA; and
   (3) Third step of amplifying the methylated DNA in the objective DNA region from the selected single-stranded DNA to a detectable level by a polymerase chain reaction (PCR) and quantifying the amount of the amplified DNA.
[2] The method of [1], wherein the immobilized immobilized methylated DNA antibody is a methylcytosine antibody.
[3] The method of [1] or [2], wherein the biological specimen is blood, a bodily fluid, serum, plasma, a cell lysate, or a tissue lysate from a mammal.
[4] The method of any one of [1] to [3], wherein the DNA sample derived from the genomic DNA contained in the biological specimen is a DNA sample digested in advance with a restriction enzyme for which a recognition cleavage site is not present in the objective DNA region of the genomic DNA, or a DNA sample purified in advance.
[5] The method of any one of [1] to [4], wherein the methylation-sensitive restriction enzyme is HhaI.
[6] The method of any one of [1] to [5], wherein a counter oligonucleotide is added when separating the methylated double-stranded DNA into the methylated single-stranded DNA in Second step.

### BRIEF DESCRIPTION OF THE DRAWINGS

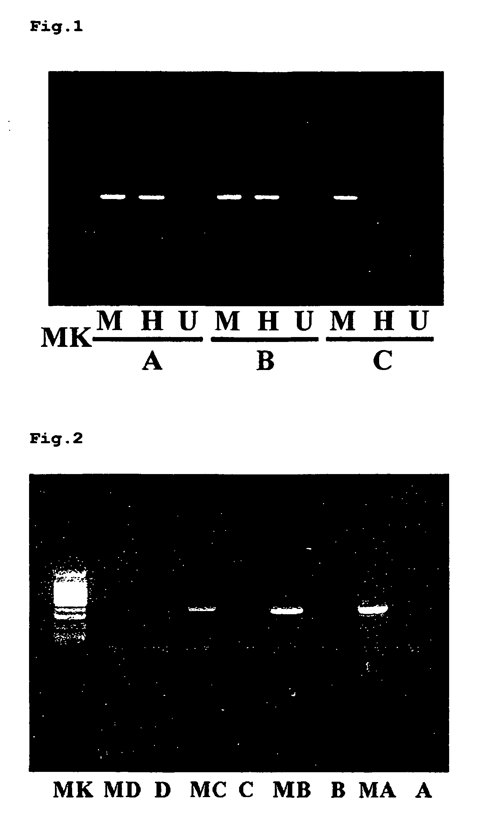

Fig. 1 shows results of 2% agarose gel electrophoresis of amplification products obtained by amplifying methylated DNA in the region comprising the nucleotide sequence of SEQ ID NO: 23 by PCR from a prepared sample in Example 1.
   From the leftmost lane in the drawing, results in a DNA marker "MK", a sample "M" of a solution of a partially methylated oligonucleotide GPR7-2079-2176/98 mer-M(7) in which the recognition sequence of HpaII is methylated, subjected to an "A" treatment, a sample "H" of a solution of a partially methylated oligonucleotide GPR7-2079-2176/98 mer-HM(5) in which part of the recognition sequence of HpaII is not methylated, subjected to an "A" treatment, a sample "U" of a solution of an unmethylated oligonucleotide GPR7-2079-2176/98 mer-UM, subjected to an "A" treatment, a sample "M" of a solution of a partially methylated oligonucleotide GPR7-2079-2176/98 mer-M(7) in which the recognition sequence of HpaII is methylated, subjected to a "B" treatment, a sample "H" of a solution of a partially methylated oligonucleotide GPR7-2079-2176/98 mer-HM(5) in which part of the recognition sequence of HpaII is not methylated, subjected to a "B" treatment, a sample "U" of a solution of an unmethylated oligonucleotide GPR7-2079-2176/98 mer-UM, subjected to a "B" treatment, a sample "M" of a solution of a partially methylated oligonucleotide GPR7-2079-2176/98 mer-M(7) in which the recognition sequence of HpaII is methylated, subjected to a "C" treatment, a sample "H" of a solution of a partially methylated oligonucleotide GPR7-2079-2176/98 mer-HM(5) in which part of the recognition sequence of HpaII is not methylated, subjected to a "C" treatment, and a sample "U" of a solution of an unmethylated oligonucleotide GPR7-2079-2176/98 mer-UM, subjected to a "C" treatment are shown.
Fig. 2 shows results of 1.5% agarose gel electrophoresis of amplification products obtained by amplifying methylated DNA in the target DNA region comprising the nucleotide sequence of SEQ ID NO: 28 by PCR from a prepared sample in Example 2. From the leftmost lane in the drawing, results in a DNA marker "MK", a solution "MD" of a methylated DNA fragment MX (negative control), a solution "D" of an unmethylated DNA fragment X (negative control), a solution "MC" of a methylated DNA fragment MX, a solution "C" of an unmethylated DNA fragment X, a solution "MB" of a methylated DNA fragment MX, a solution "B" of an unmethylated DNA fragment X, a solution "MA" of a methylated DNA fragment MX, and a solution "A" of an unmethylated DNA fragment X are shown.

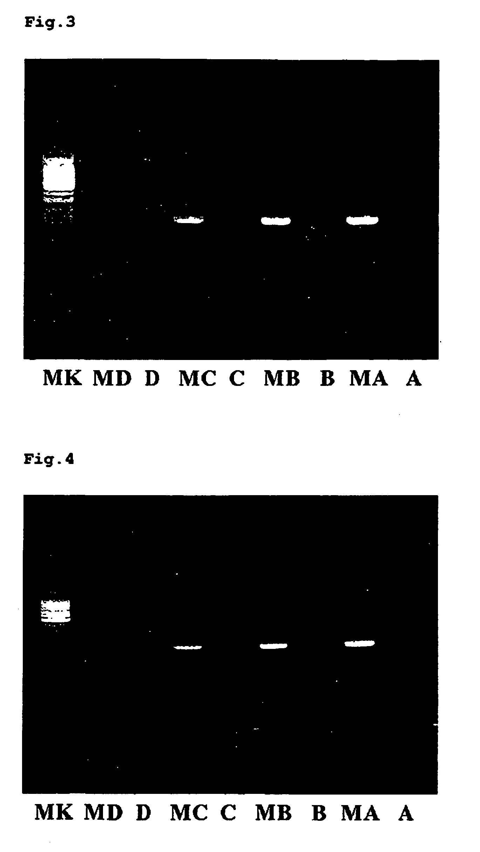

Fig. 3 shows results of 1.5% agarose gel electrophoresis of amplification products obtained by amplifying methylated DNA in the target DNA region comprising the nucleotide sequence of SEQ ID NO: 45 by PCR from a prepared sample in Example 3. From the leftmost lane in the drawing, results in a DNA marker "MK", a solution "MD" of a methylated DNA fragment MY (negative control), a solution "D" of an unmethylated DNA fragment Y (negative control), a solution "MC" of a methylated DNA fragment MY, a solution "C" of an unmethylated DNA fragment X, a solution "MB" of a methylated DNA fragment MY, a solution "B" of an unmethylated DNA fragment Y, a solution "MA" of a methylated DNA fragment MA, and a solution "A" of an unmethylated DNA fragment Y are shown.
Fig. 4 shows results of 1.5% agarose gel electrophoresis of amplification products obtained by amplifying methylated DNA in the target DNA region comprising the nucleotide sequence of SEQ ID NO: 53 by PCR from a prepared sample in Example 4. From the leftmost lane in the drawing, results in a DNA marker "MK", a solution "MD" of a methylated DNA fragment MT (negative control), a solution "D" of an unmethylated DNA fragment T (negative control), a solution "MC" of a methylated DNA fragment MT, a solution "C" of an unmethylated DNA fragment T, a solution "MB" of a methylated DNA fragment MT, a solution "B" of an unmethylated DNA fragment T, a solution "MA" of a methylated DNA fragment MT, and a solution "A" of an unmethylated DNA fragment T are shown.

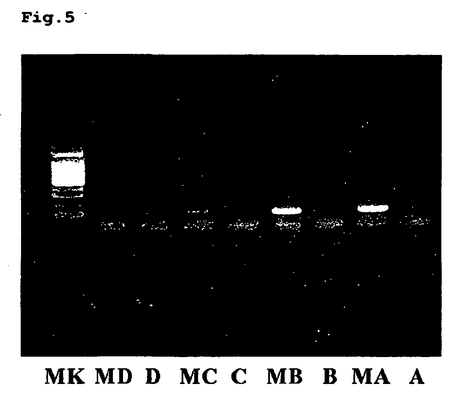

Fig. 5 shows results of 1.5% agarose gel electrophoresis of amplification products obtained by amplifying methylated DNA in the target DNA region comprising the nucleotide sequence of SEQ ID NO: 53 by PCR from a prepared sample in Example 5. From the leftmost lane in the drawing, results in a DNA marker "MK", a solution "MD" of a methylated yeast genomic DNA (negative control), a solution "D" of an unmethylated yeast genomic DNA (negative control), a solution "MC" of a methylated yeast genomic DNA, a solution "C" of an unmethylated yeast genomic DNA, a solution "MB" of a methylated yeast genomic DNA, a solution "B" of an unmethylated yeast genomic DNA, a solution "MA" of a methylated yeast genomic DNA, and a solution "A" of an unmethylated yeast genomic DNA are shown.

### MODE FOR CARRYING OUT THE INVENTION

As the "biological specimen" in the present invention, for example, a cell lysate, a tissue lysate (here the term "tissue" is used in a broad sense including blood, lymph node and so on) or biological samples including bodily sections such as plasma, serum and lymph, bodily secretions (urine, milk and so on) and the like and a genomic DNA obtained by extracting these biological samples, in mammals can be recited. As a biological specimen, for example, samples derived from microorganisms, viruses and the like can be recited, and in such a case, "a genomic DNA" in the present measuring method also means genomic DNA of microorganisms, viruses and the like.

When the specimen derived from a mammal is blood, use of the present measuring method in a regular health check or a simple examination is expected.

For obtaining a genomic DNA from a specimen derived from a mammal, for example, DNA may be extracted using a commercially available DNA extraction kit.

When blood is used as a specimen, plasma or serum is prepared from blood in accordance with a commonly used method, and using the prepared plasma or serum as a specimen, free DNA (including DNA derived from cancer cells such as gastric cancer cells) contained in the specimen is analyzed. This enables analysis of DNA derived from cancer cells such as gastric cancer cells while avoiding DNA derived from hemocytes, and improves the sensitivity of detection of cancer cells such as gastric cancer cells and a tissue containing the same.

The DNA sample derived from genomic DNA may be a DNA sample digested in advance with a restriction enzyme recognition cleavage site for which is not present in the objective DNA region of the genomic DNA, or a DNA sample purified in advance by a prescribed method.

Usually, a gene (a genomic DNA) consists of four kinds of bases. In these bases, such a phenomenon is known that only cytosine is methylated, and such methylation modification of DNA is limited to cytosine in a nucleotide sequence represented by 5'-CG-3' (C represents cytosine, and G represents guanine. Hereinafter, the nucleotide sequence is also referred to as "CpG"). The site to be methylated in cytosine is its position 5. In DNA replication prior to cell division, only cytosine in "CpG" of a template chain is methylated immediately after replication, however, cytosine in "CpG" of a newly-generated strand is immediately methylated by the action of methyltransferase. Therefore, the methylation state of DNA will be passed to new two sets of DNA even after DNA replication. The term "methylated DNA" in the present invention means DNA occurring by such methylation modification.

The term "CpG pair" in the present invention means double-stranded oligonucleotide in which a nucleotide sequence represented by CpG and a CpG that is complement with this are base-paired.

The term "objective DNA region" (hereinafter, also referred to as an "objective region") used in the present invention means a DNA region for which presence or absence of methylation of cytosine included in the region is to be examined, and has a recognition site of at least one kind of methylation sensitive restriction enzyme. A DNA region containing at least one cytosine in a nucleotide sequence represented by CpG which is present in a nucleotide sequence of a promoter region, an untranslated region, or a translated region (coding region) of a useful protein gene such as Lysyl oxidase, HRAS-like suppressor, bA305P22.2.1, Gamma filamin, HAND1, Homologue of RIKEN 2210016F16, FLJ32130, PPARG angiopoietin-related protein, Thrombomodulin, p53-responsive gene 2, Fibrillin2, Neurofilament3, disintegrin and metalloproteinase domain 23, G protein-coupled receptor 7, G-protein coupled somatostatin and angiotensin-like peptide receptor, Solute carrier family 6 neurotransmitter transporter noradrenalin member 2 and so on can be recited.

To be more specific, when the useful protein gene is a Lysyl oxidase gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Lysyl oxidase gene derived from human, and a promoter region located 5' upstream of the same can be recited, and more concretely, the nucleotide sequence of SEQ ID NO: 1 (corresponding to a nucleotide sequence represented by base No. 16001 to 18661 in the nucleotide sequence described in Genbank Accession No. AF270645) can be recited. In the nucleotide sequence of SEQ ID NO: 1, ATG codon encoding methionine at amino terminal of Lysyl oxidase protein derived from human is represented in base No. 2031 to 2033, and a nucleotide sequence of the above exon 1 is represented in base No. 1957 to 2661. Cytosine in the nucleotide sequence represented by CpG which is present in the nucleotide sequence of SEQ ID NO: 1, in particular, cytosine in CpG which is present in a region where CpGs are densely present in the nucleotide sequence of SEQ ID NO: 1 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1560, 1574, 1600, 1623, 1635, 1644, 1654, 1661, 1682, 1686, 1696, 1717, 1767, 1774, 1783, 1785, 1787, 1795 and so on in the nucleotide sequence of SEQ ID NO: 1 can be recited.

To be more specific, when the useful protein gene is a HRAS-like suppressor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HRAS-like suppressor gene derived from human, and a promoter region located 5' upstream of the same can be recited, and more concretely, the nucleotide sequence of SEQ ID NO: 2 (corresponding to a nucleotide sequence represented by base No. 172001 to 173953 in the nucleotide sequence described in Genbank Accession No. AC068162) can be recited. In the nucleotide sequence of SEQ ID NO: 2, the nucleotide sequence of exon 1 of a HRAS-like suppressor gene derived from human is represented in base No. 1743 to 1953. Cytosine in the nucleotide sequence represented by CpG which is present in the nucleotide sequence of SEQ ID NO: 2, in particular, cytosine in CpG which is present in a region where CpGs are densely present in the nucleotide sequence of SEQ ID NO: 2 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1316, 1341, 1357, 1359, 1362, 1374, 1390, 1399, 1405, 1409, 1414, 1416, 1422, 1428, 1434, 1449, 1451, 1454, 1463, 1469, 1477, 1479, 1483, 1488, 1492, 1494, 1496, 1498, 1504, 1510, 1513, 1518, 1520 and so on in the nucleotide sequence of SEQ ID NO: 2 can be recited.

To be more specific, when the useful protein gene is a bA305P22.2.1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a bA305P22.2.1 gene derived from human, and a promoter region located 5' upstream of the same can be recited, and more concretely, the nucleotide sequence of SEQ ID NO: 3 (corresponding to a nucleotide sequence represented by base No. 13001 to 13889 in the nucleotide sequence described in Genbank Accession No. AL121673) can be recited. In the nucleotide sequence of SEQ ID NO: 3, ATG codon encoding methionine at amino terminal of bA305P22.2.1 protein derived from human is represented in base No. 849 to 851, and a nucleotide sequence of the above exon 1 is represented in base No. 663 to 889. Cytosine in the nucleotide sequence represented by CpG which is present in the nucleotide sequence of SEQ ID NO: 3, in particular, cytosine in CpG which is present in a region where CpGs are densely present in the nucleotide sequence of SEQ ID NO: 3 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 335, 337, 351, 363, 373, 405, 424, 427, 446, 465, 472, 486 and so on in the nucleotide sequence of SEQ ID NO: 3 can be recited.

To be more specific, when the useful protein gene is a Gamma filamin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Gamma filamin gene derived from human, and a promoter region located 5' upstream of the same can be recited, and more concretely, the nucleotide sequence of SEQ ID NO: 4 (corresponding to a complementary sequence to a nucleotide sequence represented by base No. 63528 to 64390 in the nucleotide sequence described in Genbank Accession No. AC074373) can be recited. In the nucleotide sequence of SEQ ID NO: 4, ATG codon encoding methionine at amino terminal of Gamma filamin protein derived from human is represented in base No. 572 to 574, and a nucleotide sequence of the above exon 1 is represented in base No. 463 to 863. Cytosine in the nucleotide sequence represented by CpG which is present in the nucleotide sequence of SEQ ID NO: 4, in particular, cytosine in CpG which is present in a region where CpGs are densely present in the nucleotide sequence of SEQ ID NO: 4 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 329, 333, 337, 350, 353, 360, 363, 370, 379, 382, 384, 409, 414, 419, 426, 432, 434, 445, 449, 459, 472, 474, 486, 490, 503, 505 and so on in the nucleotide sequence of SEQ ID NO: 4 can be recited.

To be more specific, when the useful protein gene is a HAND1 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a HAND1 gene derived from human, and a promoter region located 5' upstream of the same can be recited, and more concretely, the nucleotide sequence of SEQ ID NO: 5 (corresponding to a complementary sequence to a nucleotide sequence represented by base No. 24303 to 26500 in the nucleotide sequence described in Genbank Accession No. AC026688) can be recited. In the nucleotide sequence of SEQ ID NO: 5, ATG codon encoding methionine at amino terminal of HAND1 protein derived from human is represented in base No. 1656 to 1658, and a nucleotide sequence of the above exon 1 is represented in base No. 1400 to 2198. Cytosine in the nucleotide sequence represented by CpG which is present in the nucleotide sequence of SEQ ID NO: 5, in particular, cytosine in CpG which is present in a region where CpGs are densely present in the nucleotide sequence of SEQ ID NO: 5 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1153, 1160, 1178, 1187, 1193, 1218, 1232, 1266, 1272, 1292, 1305, 1307, 1316, 1356, 1377, 1399, 1401, 1422, 1434 and so on in the nucleotide sequence of SEQ ID NO: 5 can be recited.

To be more specific, when the useful protein gene is a Homologue of RIKEN 2210016F16 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Homologue of RIKEN 2210016F16 gene derived from human, and a promoter region located 5' upstream of the same can be recited, and more concretely, the nucleotide sequence of SEQ ID NO: 6 (corresponding to a complementary nucleotide sequence to a nucleotide sequence represented by base No. 157056 to 159000 in the nucleotide sequence described in Genbank Accession No. AL354733) can be recited. In the nucleotide sequence of SEQ ID NO: 6, a nucleotide sequence of exon 1 of a Homologue of a RIKEN 2210016F16 gene derived from human is represented in base No. 1392 to 1945. Cytosine in the nucleotide sequence represented by CpG which is present in the nucleotide sequence of SEQ ID NO: 6, in particular, cytosine in CpG which is present in a region where CpGs are densely present in the nucleotide sequence of SEQ ID NO: 6 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1172, 1175, 1180, 1183, 1189, 1204, 1209, 1267, 1271, 1278, 1281, 1313, 1319, 1332, 1334, 1338, 1346, 1352, 1358, 1366, 1378, 1392, 1402, 1433, 1436, 1438 and so on in the nucleotide sequence of SEQ ID NO: 6 can be recited.

To be more specific, when the useful protein gene is a FLJ32130 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a FLJ32130 gene derived from human, and a promoter region located 5' upstream of the same can be recited, and more concretely, the nucleotide sequence of SEQ ID NO: 7 (corresponding to a complementary nucleotide sequence to a nucleotide sequence represented by base No. 1 to 2379 in the nucleotide sequence described in Genbank Accession No. AC002310) can be recited. In the nucleotide sequence of SEQ ID NO: 7, ATG codon encoding methionine at amino terminal of FLJ32130 protein derived from human is represented in base No. 2136 to 2138, and a nucleotide sequence assumed to be the above exon 1 is represented in base No. 2136 to 2379. Cytosine in the nucleotide sequence represented by CpG which is present in the nucleotide sequence of SEQ ID NO: 7, in particular, cytosine in CpG which is present in a region where CpGs are densely present in the nucleotide sequence of SEQ ID NO: 7 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1714, 1716, 1749, 1753, 1762, 1795, 1814, 1894, 1911, 1915, 1925, 1940, 1955, 1968 and so on in the nucleotide sequence of SEQ ID NO: 7 can be recited.

To be more specific, when the useful protein gene is a PPARG angiopoietin-related protein gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a PPARG angiopoietin-related protein gene derived from human, and a promoter region located 5' upstream of the same can be recited, and more concretely, the nucleotide sequence of SEQ ID NO: 8 can be recited. In the nucleotide sequence of SEQ ID NO: 8, ATG codon encoding methionine at amino terminal of PPARG angiopoietin-related protein derived from human is represented in base No. 717 to 719, and a nucleotide sequence of the 5' side part of the above exon 1 is represented in base No. 1957 to 2661. Cytosine in the nucleotide sequence represented by CpG which is present in the nucleotide sequence of SEQ ID NO: 8, in particular, cytosine in CpG which is present in a region where CpGs are densely present in the nucleotide sequence of SEQ ID NO: 8 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 35, 43, 51, 54, 75, 85, 107, 127, 129, 143, 184, 194, 223, 227, 236, 251, 258 and so on in the nucleotide sequence of SEQ ID NO: 8 can be recited.

To be more specific, when the useful protein gene is a Thrombomodulin gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Thrombomodulin gene derived from human, and a promoter region located 5' upstream of the same can be recited, and more concretely, the nucleotide sequence of SEQ ID NO: 9 (corresponding to a nucleotide sequence represented by base No. 1 to 6096 in the nucleotide sequence described in Genbank Accession No. AF495471) can be recited. In the nucleotide sequence of SEQ ID NO: 9, ATG codon encoding methionine at amino terminal of Thrombomodulin protein derived from human is represented in base No. 2590 to 2592, and a nucleotide sequence of the above exon 1 is represented in base No. 2048 to 6096. Cytosine in the nucleotide sequence represented by CpG which is present in the nucleotide sequence of SEQ ID NO: 9, in particular, cytosine in CpG which is present in a region where CpGs are densely present in the nucleotide sequence of SEQ ID NO: 9 exhibits high methylation frequency (namely, a high methylation state (hypermethylation)) in, for example, cancer cells such as gastric cancer cells. More concretely, as cytosine exhibiting high methylation frequency in gastric cancer cells, for example, cytosines represented by base Nos. 1539, 1551, 1571, 1579, 1581, 1585, 1595, 1598, 1601, 1621, 1632, 1638, 1645, 1648, 1665, 1667, 1680, 1698, 1710, 1724, 1726, 1756 and so on in the nucleotide sequence of SEQ ID NO: 9 can be recited.

To be more specific, when the useful protein gene is a p53-responsive gene 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a p53-responsive gene 2 gene derived from human, and a promoter region located 5' upstream of the same can be recited, and more concretely, the nucleotide sequence of SEQ ID NO: 10 (corresponding to a complementary sequence to a nucleotide sequence represented by base No. 113501 to 116000 in the nucleotide sequence described in Genbank Accession No. AC009471) can be recited. In the nucleotide sequence of SEQ ID NO: 10, a nucleotide sequence of exon 1 of a p53-responsive gene 2 gene derived from human is represented in base No. 1558 to 1808. Cytosine in the nucleotide sequence represented by CpG which is present in the nucleotide sequence of SEQ ID NO: 10 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1282, 1284, 1301, 1308, 1315, 1319, 1349, 1351, 1357, 1361, 1365, 1378, 1383 and so on in the nucleotide sequence of SEQ ID NO: 10 can be recited.

To be more specific, when the useful protein gene is a Fibrillin2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Fibrillin2 gene derived from human, and a promoter region located 5' upstream of the same can be recited, and more concretely, the nucleotide sequence of SEQ ID NO: 11 (corresponding to a complementary sequence to a nucleotide sequence represented by base No. 118801 to 121000 in the nucleotide sequence described in Genbank Accession No. AC113387) can be recited. In the nucleotide sequence of SEQ ID NO: 11, a nucleotide sequence of exon 1 of a Fibrillin2 gene derived from human is represented in base No. 1091 to 1345. Cytosine in the nucleotide sequence represented by CpG which is present in the nucleotide sequence of SEQ ID NO: 11 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 679, 687, 690, 699, 746, 773, 777, 783, 795, 799, 812, 823, 830, 834, 843 and so on in the nucleotide sequence of SEQ ID NO: 11 can be recited.

To be more specific, when the useful protein gene is a Neurofilament3 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Neurofilament3 gene derived from human, and a promoter region located 5' upstream of the same can be recited, and more concretely, the nucleotide sequence of SEQ ID NO: 12 (corresponding to a complementary sequence to a nucleotide sequence represented by base No. 28001 to 30000 in the nucleotide sequence described in Genbank Accession No. AF106564) can be recited. In the nucleotide sequence of SEQ ID NO: 12, a nucleotide sequence of exon 1 of a Neurofilament3 gene derived from human is represented in base No. 614 to 1694. Cytosine in the nucleotide sequence represented by CpG which is present in the nucleotide sequence of SEQ ID NO: 12 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 428, 432, 443, 451, 471, 475, 482, 491, 499, 503, 506, 514, 519, 532, 541, 544, 546, 563, 566, 572, 580 and so on in the nucleotide sequence of SEQ ID NO: 12 can be recited.

To be more specific, when the useful protein gene is a disintegrin and metalloproteinase domain 23 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a disintegrin and metalloproteinase domain 23 gene derived from human, and a promoter region located 5' upstream of the same can be recited, and more concretely, the nucleotide sequence of SEQ ID NO: 13 (corresponding to a nucleotide sequence represented by base No. 21001 to 23300 in the nucleotide sequence described in Genbank Accession No. AC009225) can be recited. In the nucleotide sequence of SEQ ID NO: 13, a nucleotide sequence of exon 1 of a disintegrin and metalloproteinase domain 23 gene derived from human is represented in base No. 1194 to 1630. Cytosine in the nucleotide sequence represented by CpG which is present in the nucleotide sequence of SEQ ID NO: 13 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 998, 1003, 1007, 1011, 1016, 1018, 1020, 1026, 1028, 1031, 1035, 1041, 1043, 1045, 1051, 1053, 1056, 1060, 1066, 1068, 1070, 1073, 1093, 1096, 1106, 1112, 1120, 1124, 1126 and so on in the nucleotide sequence of SEQ ID NO: 13 can be recited.

To be more specific, when the useful protein gene is a G protein-coupled receptor 7 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G protein-coupled receptor 7 gene derived from human, and a promoter region located 5' upstream of the same can be recited, and more concretely, the nucleotide sequence of SEQ ID NO: 14 (corresponding to a nucleotide sequence represented by base No. 75001 to 78000 in the nucleotide sequence described in Genbank Accession No. AC009800) can be recited. In the nucleotide sequence of SEQ ID NO: 14, a nucleotide sequence of exon 1 of a G protein-coupled receptor 7 gene derived from human is represented in base No. 1666 to 2652. Cytosine in the nucleotide sequence represented by CpG which is present in the nucleotide sequence of SEQ ID NO: 14 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1480, 1482, 1485, 1496, 1513, 1526, 1542, 1560, 1564, 1568, 1570, 1580, 1590, 1603, 1613, 1620 and so on in the nucleotide sequence of SEQ ID NO: 14 can be recited.

To be more specific, when the useful protein gene is a G-protein coupled somatostatin and angiotensin-like peptide receptor gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a G-protein coupled somatostatin and angiotensin-like peptide receptor gene derived from human, and a promoter region located 5' upstream of the same can be recited, and more concretely, the nucleotide sequence of SEQ ID NO: 15 (corresponding to a complementary sequence to a nucleotide sequence represented by base No. 57001 to 60000 in the nucleotide sequence described in Genbank Accession No. AC008971) can be recited. In the nucleotide sequence of SEQ ID NO: 15, a nucleotide sequence of exon 1 of a G-protein coupled somatostatin and angiotensin-like peptide receptor gene derived from human is represented in base No. 776 to 2632. Cytosine in the nucleotide sequence represented by CpG which is present in the nucleotide sequence of SEQ ID NO: 15 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 470, 472, 490, 497, 504, 506, 509, 514, 522, 540, 543, 552, 566, 582, 597, 610, 612 and so on in the nucleotide sequence of SEQ ID NO: 15 can be recited.

To be more specific, when the useful protein gene is a Solute carrier family 6 neurotransmitter transporter noradrenalin member 2 gene, as a nucleotide sequence that includes at least one nucleotide sequence represented by CpG present in a nucleotide sequence of its promoter region, untranslated region or translated region (coding region), a nucleotide sequence of a genomic DNA containing exon 1 of a Solute carrier family 6 neurotransmitter transporter noradrenalin member 2 gene derived from human, and a promoter region located 5' upstream of the same can be recited, and more concretely, the nucleotide sequence of SEQ ID NO: 16 (corresponding to a complementary sequence to a nucleotide sequence represented by base No. 78801 to 81000 in the nucleotide sequence described in Genbank Accession No. AC026802) can be recited. In the nucleotide sequence of SEQ ID NO: 16, a nucleotide sequence of exon 1 of a Solute carrier family 6 neurotransmitter transporter noradrenalin member 2 gene derived from human is represented in base No. 1479 to 1804. Cytosine in the nucleotide sequence represented by CpG which is present in the nucleotide sequence of SEQ ID NO: 16 exhibits high methylation frequency (namely, a high methylation state (hypermethylation)) in, for example, cancer cells such as pancreas cancer cells. More concretely, as cytosine exhibiting high methylation frequency in pancreas cancer cells, for example, cytosines represented by base Nos. 1002, 1010, 1019, 1021, 1051, 1056, 1061, 1063, 1080, 1099, 1110, 1139, 1141, 1164, 1169, 1184 and so on in the nucleotide sequence of SEQ ID NO: 16 can be recited.

The term "methylated DNA antibody" means an antibody that binds to a methylated base in DNA as its antigen. Concretely, it may be a methylcytosine antibody, and an antibody having a property of recognizing and binding to cytosine methylated at position 5 in single-stranded DNA can be recited. Also a commercially available methylated DNA antibody may be applicable as far as it specifically recognizes and specifically binds to DNA in a methylated state according to the present invention.

A methylated DNA antibody can be prepared by a conventional immunological technique from a methylated base, methylated DNA or the like as an antigen. Concretely, a methylcytosine antibody can be obtained by selecting from antibodies prepared against an antigen such as 5-methylcytidine, 5-methylcytosine or DNA containing 5-methylcytosine according to specific binding to methylcytosine in DNA as an index.

As an antibody obtainable by immunizing an animal against an antigen, after immunizing with a purified antigen, an antibody of an IgG fraction (polyclonal antibody), and an antibody produced by a single clone (monoclonal antibody) can be used. In the present invention, since an antibody capable of specifically recognizing methylated DNA or methylcytosine is desired, it is preferable to use a monoclonal antibody.

As a method of preparing a monoclonal antibody, a procedure based on a cell fusion method can be recited. For example, in the cell fusion method, a hybridoma is prepared by allowing cell fusion between a pancreatic cell (B cell) derived from an immunized mouse and a myeloma cell, and an antibody produced by the hybridoma is selected, and thus a methylcytosine antibody (monoclonal antibody) is prepared. When a monoclonal antibody is prepared by a cell fusion method, it is not necessary to purify an antigen, and for example, a mixture of 5-methyl cytidine, 5-methylcytosine or DNA or the like containing 5-methylcytosine may be administered as an antigen to an animal used for immunization. As an administration method, 5-methyl cytidine, 5-methylcytosine or DNA or the like containing 5-methylcytosine is directly administered to a mouse for production of an antibody. When an antibody is difficult to be produced, an antigen bound to a support may be used for immunization.

Also, by thoroughly mixing an adjuvant solution (prepared, for example, by mixing liquid paraffin and Aracel A, and mixing killed tubercle bacilli as an adjuvant) and an antigen, and immunizing via liposome incorporating the same, immunity of an antigen can be improved. Also a method involving adding equivalent amounts of a solution containing an antigen and an adjuvant solution, fully emulsifying them, and subcutaneously or intraperitoneally injecting the resultant mixture to a mouse, and a method of adding killed Bordetella pertussis as an adjuvant after mixing well with alum water are known. A mouse may be boosted intraperitoneally or intravenously after an appropriate term from initial immunization. When the amount of an antigen is small, a solution in which the antigen is suspended may be directly injected into a mouse spleen to effect immunization.

After exenterating a spleen and peeling an adipose tissue off after several days from the final immunization, a spleen cell suspension is prepared. The spleen cell is fused, for example, with an HGPRT-deficient myeloma cell to prepare a hybridoma. As a cell fusion agent, any means capable of efficiently fusing a spleen cell (B cell) and a myeloma cell is applicable, and for example, a method of using a hemagglutinating virus of Japan (HVJ), polyethyleneglycol (PEG) and the like are recited. Cell fusion may be conducted by a method using a high voltage pulse.

After the cell fusion operation, cells are cultured in an HAT medium, a clone of a hybridoma in which a spleen cell and a myeloma cell are fused is selected, and the cell is allowed to grow until screening becomes possible. In a method of detecting an antibody for selecting a hybridoma that produces an intended antibody, or a method of measuring a titer of an antibody, an antigen-antibody reaction system may be used. Concretely, as a method of measuring an antibody against a soluble antigen, a radioisotope immune assay (RIA), an enzyme-linked immunosorbent assay (ELISA) and the like can be recited.

Single-stranded DNA is able to bind with an anti methylation antibody as far as at least one position of a CpG existing therein is methylated. The term "methylated single-stranded DNA" in the present invention means single-stranded DNA in which at least one potision of a CpG existing in single-stranded DNA is methylated, rather than meaning exclusively single-stranded DNA in which every CpG existing in single-stranded DNA is methylated.

The expression "an amount of amplified DNA obtained by (amplifying methylated DNA in a target DNA region to a detectable level"" means an amount itself after amplification of methylated DNA in a target region comprised by genomic DNA contained in a biological specimen, namely, an amount determined in Third step of the present invention as described below. For example, when the biological specimen is 1 mL of serum, it means an amount of DNA amplified based on the methylated DNA contained in 1 mL of serum.

In First step, a DNA sample derived from genomic DNA contained in a biological specimen is subjected to a digestion treatment with a methylation-sensitive restriction enzyme.

The "methylation-sensitive restriction enzyme" in the present invention does not digest a recognition sequence containing methylated cytosine, but digests only a recognition sequence containing unmethylated cytosine. In other words, in the case of DNA wherein cytosine contained in a recognition sequence inherently recognizable by the methylation sensitive restriction enzyme is methylated, the DNA will not be cleaved even when the methylation sensitive restriction enzyme is caused to act on the DNA. On the other hand, in the case of DNA wherein cytosine contained in a recognition sequence inherently recognizable by the methylation sensitive restriction enzyme is not methylated, the DNA will be cleaved when the methylation sensitive restriction enzyme is caused to act on the DNA. Concrete examples of such methylation sensitive restriction enzymes include HpaII, BstUI, NarI, SacII, and HhaI which are restriction enzymes recognition cleavage site for which is present in the objective DNA region of the genomic DNA contained in a biological specimen. The aforementioned methylation sensitive restriction enzymes have already been revealed by Gruenbaum et al. (Nucleic Acid Research, 9, 2509-2515).

As a method of examining whether or not digestion by the methylation sensitive restriction enzyme occurs, concretely, for example, a method of conducting PCR using a pair of primers capable of amplifying DNA containing cytosine which is a target of analysis in a recognition sequence while using the DNA as a template, and examining whether or not the DNA is amplified (amplified product) can be recited. When the cytosine which is a target of analysis is methylated, an amplified product is obtained. On the other hand, when the cytosine which is a target of analysis is not methylated, an amplified product is not obtained. In this manner, by comparing the amounts of amplified DNA, it is possible to measure the methylated rate of the cytosine which is a target of analysis. In brief, when genomic DNA contained in the biological specimen is methylated, it is possible to distinguish whether or not cytosine in CpG pair existing in the recognition site of the methylation sensitive restriction enzyme in genomic DNA contained in the biological specimen is methylated by utilizing the characteristic that the methylation sensitive restriction enzyme fails to cleave methylated DNA. In other words, when cytosine in at least one CpG pair existing in the recognition site of the methylation sensitive restriction enzyme in genomic DNA contained in the biological specimen is not methylated, the DNA having such a recognition site will be cleaved by the methylation sensitive restriction enzyme when it is subjected to a digestion treatment with the methylation sensitive restriction enzyme. Further, when cytosine in every CpG pair existing in the recognition site of the methylation sensitive restriction enzyme in genomic DNA contained in the biological specimen is methylated, the DNA having such a recognition site will not be cleaved by the methylation sensitive restriction enzyme. Therefore, by conducting PCR using a pair of primers capable of amplifying the objective DNA region as will be described later after executing the digestion treatment, an amplified product by PCR will not be obtained when cytosine in at least one CpG pair existing in the recognition site of the methylation sensitive restriction enzyme in genomic DNA contained in the biological specimen is not methylated, and on the other hand, an amplified product by PCR will be obtained when cytosine in every CpG pair existing in the recognition site of the methylation sensitive restriction enzyme in genomic DNA contained in the biological specimen is methylated.

Concretely, First step may be executed, for example, in the following manner when genomic DNA contained in the biological specimen is genomic DNA from mammals. Genomic DNA from mammals is added with 3 µL of an optimum 10 x buffer (330 mM Tris-Acetate pH 7.9, 660 mM KOAc, 100 mM MgOAc₂, 5 mM Dithiothreitol), 3 µL of 1 mg/mL BSA aqueous solution, each 1.5 µL of a methylation sensitive restriction enzyme such as HpaII or HhaI (10 U/µL), and the resultant mixture is added with sterilized ultrapure water to make the liquid amount 30 µL, and incubated at 37°C for one to three hours.

The treatment with a methylation-sensitive restriction enzyme in First step includes addition of a masking oligonucleotide. In particular, the treatment comprises

First (A) step of mixing a single-stranded DNA (plus strand) containing the objective DNA region and a masking oligonucleotide comprising a nucleotide sequence complementary to a nucleotide sequence of a recognition site for a methylation-sensitive restriction enzyme, thereby obtaining a partially single-stranded DNA in which the recognition site for the methylation-sensitive restriction enzyme forms double strand by complementary base-pairing with the masking oligonucleotide; followed by First (B) step of digesting the single-stranded DNA selected in First (A) step with the methylation-sensitive restriction enzyme. In particular, therefore, a masking oligonucleotide is added to a solution for the treatment with a methylation-sensitive restriction enzyme.

By undergoing First (A) and First (B) steps, any DNA sample derived from a genomic DNA contained in a biological specimen may be digested with a methylation-sensitive restriction enzyme with double-stranded DNA exclusively as a substrate, even if the DNA sample is single-stranded DNA.

The term "masking oligonucleotide" means oligonucleotide having a nucleotide sequence complementary to the nucleotide sequence of the recognition site of the methylation sensitive restriction enzyme, and is oligonucleotide that forms double strand by complementary base-pairing at least one site (even every site is possible) of several recognition sites of the methylation sensitive restriction enzyme contained in the objective DNA region in the single-stranded DNA (that is, the site is made into double-stranded state), thereby enabling the methylation sensitive restriction enzyme that uses only double-stranded DNA as a substrate to digest the site, and improving digestion efficiency at the site for the methylation sensitive restriction enzyme capable of digesting single-stranded DNA (methylation sensitive restriction enzyme capable of digesting single-stranded DNA also digests double-stranded DNA, and digestion efficiency thereof is higher with respect to double-stranded DNA than with respect to single-stranded DNA), and means oligonucleotide not inhibiting formation of double strand between single-stranded DNA containing the objective DNA region and single-stranded immobilized oligonucleotide. Further, when a sample is a single-stranded DNA, the masking oligonucleotide should be oligonucleotide that is unavailable in a reaction for extending an extension primer by using a later-described reverse primer (plus strand) as the extension primer and the masking oligonucleotide (minus strand) as a template. As a nucleotide length, 8 to 200 bases long is preferred.

The masking oligonucleotide to be mixed with a DNA sample derived from genomic DNA may be one kind or plural kinds. When plural kinds are used, many of recognition sites of the methylation sensitive restriction enzyme in the single-stranded DNA containing the objective DNA region become double-strand state, and "DNA remaining undigested" as will be described later by the methylation sensitive restriction enzyme can be minimized. For example, it is particularly useful to use the masking oligonucleotide designed in accordance with a site intended not to be digested when it is methylated and intended to be digested when it is not methylated among several recognition sequences of the methylation sensitive restriction enzyme contained in the objective DNA region (for example, the site that is methylated at 100% in a diseased patient specimen, but is not methylated at 100% in a healthy specimen).

As a concern in a digestion treatment in First step, a fear that a recognition sequence containing non-methylated cytosine cannot be completely digested (so called "DNA remaining undigested") can be recited. When such a fear is problematic, since the "DNA remaining undigested" can be minimized if recognition sites of the methylation sensitive restriction enzyme abundantly exist, it is considered that as the objective DNA region, the one having one or more recognition sites of the methylation sensitive restriction enzyme is preferred and the more the better.

One preferable embodiment is that "a DNA sample derived from a genomic DNA contained in a biological specimen" is a DNA sample digested in advance with a restriction enzyme recognition cleavage site for which in not present in the objective DNA region possessed by the genomic DNA. Here, when a digested substance of a genomic DNA contained in a biological specimen is selected with the use of present immobilized oligonucleotide, shorter template DNA is more likely to be selected, and when the objective region is amplified by PCR, shorter template DNA is more preferred. Therefore, a digestion treatment may be executed while using a restriction enzyme whose recognition cleavage site excludes the objective DNA region directly on the DNA sample derived from a genomic DNA contained in a biological specimen. As a method of digesting with a restriction enzyme recognition cleavage site for which is not present in the objective DNA region, a commonly used restriction enzyme treatment method may be used. These embodiments are preferred because the methylation amount can be determined accurately by digesting the biological specimen itself in advance with a restriction enzyme as described above. Such a method is useful for avoiding the "DNA remaining undigested" as described above.

As a method of digesting a sample derived from a genomic DNA contained in a biological specimen with the methylation sensitive restriction enzyme, when the biological specimen is a genomic DNA itself, the method similar to that described above is preferred, and when the biological specimen is a tissue lysate, a cell lysate or the like, a digestion treatment may be executed using a large excess of methylation sensitive restriction enzyme, for example, a methylation sensitive restriction enzyme in an amount of 500 times (10 U) or more with respect to 25 ng of the DNA amount, according to a similar method as described above.

Basically, genomic DNA exists as double-stranded DNA. Therefore, in the present operation, not only a methylation sensitive restriction enzyme (for example, HhaI) capable of digesting single-stranded DNA, but also a methylation sensitive restriction enzyme capable of digesting double-stranded DNA (for example, HpaII, BstUI, NarI, SacII, HhaI and the like) may be used.

In Second step of the present measuring method, methylated single-stranded DNA is obtained from the DNA sample that has been subjected to the digestion treatment and obtained in First step, and the methylated single-stranded DNA is bound to an immobilized methylated DNA antibody, thereby selecting the single-stranded DNA. Second step comprises a Second (A) step of separating into methylated single-stranded DNA the methylated double-stranded DNA contained in the DNA sample that has been subjected to the digestion treatment and obtained in First step; and Second (B) step of binding the methylated single-stranded DNA obtained in Second (A) step to an immobilized methylated DNA antibody.

In Second (A) step of the present measuring method, in "separating into methylated single-stranded DNA the methylated double-stranded DNA contained in the DNA sample that has been subjected to the digestion treatment and obtained in First step", a commonly used operation for making double-stranded DNA into single-stranded DNA may be conducted. Concretely, a DNA sample derived from genomic DNA contained in a biological specimen may be dissolved in an appropriate amount of ultrapure water, heated at 95°C for 10 minutes, and rapidly cooled on ice.

In Second (B) step, the methylated single-stranded DNA obtained in Second (A) step is bound to an immobilized methylated DNA antibody, thereby selecting the single-stranded DNA. The immobilized methylated DNA antibody is used for selecting methylated single-stranded DNA from a DNA sample derived from genomic DNA contained in a biological specimen. The immobilized methylated DNA antibody may be one immobilizable to a support, and the expression "one immobilizable to a support" means that a immobilized methylated DNA antibody can be immobilized to a support directly or indirectly. For achieving such immobilization, a immobilized methylated DNA antibody may be immobilized to a support according to a commonly used genetic engineering operation method or a commercially available kit, apparatus or the like (binding to a solid phase). Concretely, a method of immobilizing a biotinylated immobilized methylated DNA antibody obtained by biotinylating an immobilized methylated DNA antibody to a support coated with streptavidin (for example, a PCR tube coated with streptavidin, magnetic beads coated with streptavidin and so on) can be recited.

Also there is a method of letting a molecule having an active functional group such as an amino group, a thiol group, or an aldehyde group covalently bind to an immobilized methylated DNA antibody, and letting the resultant bound body covalently bind to a support made of glass, a polysaccharide derivative, silica gel, the synthetic resin or thermostable plastic whose surface is activated with a silane coupling agent or the like. Covalent bonding may be achieved, for example, using a spacer formed by serially connecting five triglycerides, a cross linker or the like.

An immobilized methylated DNA antibody may be directly immobilized to a support, or an antibody against an immobilized methylated DNA antibody (secondary antibody) may be immobilized to a support, and a methylated antibody may be bound to the secondary antibody to achieve immobilization to a support.

It suffices that the present immobilized immobilized methylated DNA antibody is immobilized to a support when single-stranded DNA (plus strand) containing the objective DNA region is selected, and (1) immobilization may be achieved by binding between the present immobilized immobilized methylated DNA antibody and a support before binding between the single-stranded DNA (plus strand) and the present immobilized immobilized methylated DNA antibody, or (2) immobilization may be achieved by binding between the present immobilized methylated DNA antibody and a support after binding between the single-stranded DNA (plus strand) and the present immobilized immobilized methylated DNA antibody.

In Second (B) step, when "the single-stranded DNA is selected by binding between methylated single-stranded DNA and an immobilized immobilized methylated DNA antibody," it may be concretely executed in the following manner, for example, using a "biotin-labeled biotinylated methylated cytosine antibody" as an immobilized immobilized methylated DNA antibody.
(a) An avidin-coated PCR tube is added with an appropriate amount (for example, 0.1 µg/50 µL) of a biotinylated, methylated cytosine antibody, left still at room temperature for about an hour, to promote immobilization between the biotinylated methylated cytosine antibody and streptavidin. Then the remaining solution is removed and washing is performed. A washing buffer [for example, a 0.05% Tween 20-containing phosphate buffer (1 mM KH₂PO₄, 3 mM Na₂HPO·7H₂O, 154 mM NaCl, pH 7.4)] is added in a proportion of 100 µL/tube, and the solution is removed. This washing operation is repeated several times, to leave the biotinylated methylated cytosine antibody immobilized to a support inside the PCR tube.
(b) Double-stranded DNA derived from genomic DNA contained in a biological specimen is mixed with a buffer (for example, 33 mM Tris-Acetate pH 7.9, 66 mM KOAc, 10 mM MgOAc₂, 0.5 mM Dithiothreitol) and heated at 95°C for several minutes. Then the reaction is rapidly cooled to about 0 to 4°C, and kept for several minutes at this temperature to cause formation of single-stranded DNA. Then the reaction is returned to room temperature.
(c) The formed single-stranded DNA is added to an avidin-coated PCR tube to which a biotinylated methylated cytosine antibody is immobilized, and then left still at room temperature for about an hour, to promote binding between the biotinylated methylated cytosine antibody and methylated single-stranded DNA among the single-stranded DNA (formation of a bound body)(in this stage, at least single-stranded DNA containing an unmethylated DNA region does not form a bound body). Thereafter, the remaining solution is removed and washing is performed. A washing buffer [for example, a 0.05% Tween 20-containing phosphate buffer (1 mM KH₂PO₄, 3 mM Na₂HPO·7H₂O, 154 mM NaCl, pH 7.4)] is added in a proportion of 100 µL/tube, and the solution is removed. This washing operation is repeated several times, to leave the bound body inside the PCR tube (selection of a bound body).

The buffer used in (b) is not limited to the above buffer and may be any buffer that is suited for separating double-stranded DNA derived from genomic DNA from a biological sample into single-stranded DNA.

The washing operation in (a) and (c) is important for removing an unimmobilized immobilized methylated DNA antibody suspended in the solution, unmethylated single-stranded DNA that does not bind with a immobilized methylated DNA antibody and hence is suspended in the solution, and DNA suspended in a solution digested by a restriction enzyme as will be described later, from the reaction solution. The washing buffer is not limited to the foregoing washing buffer, and any buffer suited for removing the free immobilized methylated DNA antibody, single-stranded DNA and so on suspended in the solution and the like is applicable, and a DELFIA buffer (available from Perkin Elmer, Tris-HCl pH 7.8 with Tween 80), a TE buffer and the like may be used.

In Second (A) step, as a preferred embodiment in separating methylated single-stranded DNA, addition of a counter oligonucleotide and the like can be recited. A counter oligonucleotide means a short oligonucleotide comprising a part of the same nucleotide sequence as that of the objective DNA region. It may be designed to have a length of usually 10 to 100 bases, and more preferably 20 to 50 bases. Here, a counter oligonucleotide is not designed on the nucleotide sequence where a forward primer or a reverse primer complementarily binds with the target DNA region. A counter oligonucleotide is added in excess relative to genomic DNA, and is added so as to prevent a complementary strand of a target DNA region (minus strand) and a single strand of a target DNA region (plus strand) from re-binding by complementation when binding with an immobilized methylated DNA antibody is caused after making a target DNA region into a single strand (plus strand). This is because in measuring a methylation frequency of DNA or an index value having correlation therewith while a methylated DNA antibody is bound to the target DNA region, the target region is more likely to bind with the methylated DNA antibody when it is a single strand. Preferably, a counter oligonucleotide is added in an amount of at least 10 times, usually 100 times or more relative to the target DNA region.

"Adding a counter oligonucleotide in separating methylated single-stranded DNA" may be concretely achieved by mixing a DNA sample derived from genomic DNA contained in a biological specimen with a counter oligonucleotide to form a double strand between the complementary strand of the target DNA region and the counter oligonucleotide so as to select methylated single-stranded DNA from a DNA sample derived from genomic DNA contained in a biological specimen. For example, the DNA sample and the counter oligonucleotide are added to 5 µL of a buffer (330 mM Tris-Acetate pH 7.9, 660 mM KOAc, 100 mM MgOAc₂, 5 mM Dithiothreitol), 5 µL of a 100 mM MgCl₂ solution, and 5 µL of a 1 mg/mL of BSA solution, and the resultant mixture is added with sterile ultrapure water to a liquid volume of 50 µL, and mixed, heated at 95°C for 10 minutes, rapidly cooled to 70°C, kept at this temperature for 10 minutes, then cooled to 50°C, kept at this temperature for 10 minutes, and then kept at 37°C for 10 minutes and returned to room temperature.

Disclosed herein is a third step comprising, as a pre step of each of the following regular steps:
a step (First pre step) of separating the single-stranded DNA selected in Second step from the immobilized immobilized methylated DNA antibody to provide DNA in a single-stranded state (plus strand);
a step (Second pre step) of extensionally-forming a double-stranded DNA from a single-stranded DNA (plus strand) containing the objective DNA region by a single extension of an extension primer, using the genomic DNA (plus strand) provided in a single-stranded state in First pre step and the extension primer, wherein the extension primer (forward primer) comprises the nucleotide sequence (minus strand) complementary to a partial nucleotide sequence (plus strand) of the nucleotide sequence of the DNA in a single-stranded state (plus strand), the partial nucleotide sequence (plus strand) being located on further 3'-end side than the 3'-end of the nucleotide sequence (plus strand) of the objective DNA region; and
a step (Third pre step) of temporarily separating the double-stranded DNA extensionally formed in Second pre step into a single-stranded DNA (plus strand) containing the objective DNA region and a single-stranded DNA (minus strand) containing the nucleotide sequence complementary to the objective DNA region;
and as regular steps:
(a) Step A (regular step) of extensionally forming double-stranded DNA from the single-stranded DNA containing the objective DNA region, by a single extension of the extension primer, using as a template the generated single-stranded DNA (plus strand) containing the objective DNA region, and the forward primer as the extension primer; and
(b) Step B (regular step) of extensionally forming double-stranded DNA from the single-stranded DNA containing the objective DNA region, by a single extension of an extension primer, using as a template the generated single-stranded DNA (minus strand) containing the nucleotide sequence complementary to the objective DNA region, and using as the extension primer an extension primer (reverse primer) comprising the nucleotide sequence (plus strand) complementary to a partial nucleotide sequence (minus strand) of the nucleotide sequence of the single-stranded DNA (minus strand) containing the nucleotide sequence complementary to the objective DNA region, the partial nucleotide sequence (minus strand) being located on further 3'-end side than the 3'-end of the nucleotide sequence (minus strand) complementary to the nucleotide sequence (plus strand)of the objective DNA region; and wherein Third step further comprises amplifying the methylated DNA in the objective DNA region to a detectable level by repeating each regular step of Third step after temporarily separating the extensionally formed double-stranded DNA obtained in each of the regular steps into a amplified DNA.

In Third step discussed above, first, as First pre step step among the respective pre steps of the following regular steps, single-stranded DNA selected in Second step is temporarily separated from the immobilized immobilized methylated DNA antibody into DNA in a single-stranded state. Concretely, for example, by adding an annealing buffer to single-stranded DNA selected in Second step, a mixture is obtained. Then the resultant mixture is heated at 95°C for several minutes, to obtain DNA in a single-stranded state (plus strand). Thereafter, in Second pre step, concretely, for example, DNA in a single-stranded state (plus strand) obtained in First pre step and a forward primer are mixed in a solution that is prepared by adding 17.85 µL of sterile ultrapure water, 3 µL of an optimum buffer (for example, 100 mM Tris-HCl pH 8.3, 500 mM KCl, 15 mM MgCl₂), 3 µL of 2 mM dNTP, and 6 µL of 5 N betaine, and adding the resultant mixture with 0.15 µL of AmpliTaq (a kind of DNA polymerase: 5 U/µL) to a liquid volume of 30 µL, and incubated at 37°C for about two hours, to extensionally form double-stranded DNA from the single-stranded DNA (plus strand) containing an objective DNA region. In Third pre C step, concretely, for example, the double-stranded DNA extensionally formed in Second pre step is added with an annealing buffer to obtain a mixture, and the DNA is temporarily separated into single-stranded DNA containing the target DNA region by heating the mixture at 95°C for several minutes.

Thereafter, the following regular steps are conducted.
(i) The reaction is rapidly cooled to a temperature lower than Tm of the forward primer by about 0 to 20°C, and kept at this temperature for several minutes for annealing the forward primer to the generated single-stranded DNA (plus strand) containing the target DNA region.
(ii) Thereafter, the reaction is returned to room temperature.
(iii) Double-stranded DNA is extensionally formed from single-stranded DNA comprising the nucleotide sequence complementary to the objective DNA region by one extension of an extension primer by using the DNA in a single-stranded state annealed in the above (i) as a template, and the forward primer as an extension primer (namely, Step A). Concretely, it may be executed, for example, according to the later-described explanation, or the operation method in an extension reaction in Second pre step of the present invention as described above.
(iv) Single-stranded DNA is made into extensionally formed double-stranded DNA by one extension of an extension primer by using the generated single-stranded DNA (minus strand) comprising the nucleotide sequence complementary to the target DNA region as a template, and an extension primer (reverse primer) comprising a nucleotide sequence (plus strand) which is complementary to a partial nucleotide sequence (minus strand) of the nucleotide sequence comprised by the single-stranded DNA (minus strand) containing the target DNA region, the partial nucleotide sequence (minus strand) located on further 3'-end side than 3'-end of the nucleotide sequence (minus strand) complementary to the nucleotide sequence (plus strand) of the target DNA region as the extension primer (namely, Step B). Concretely, it may be executed, for example, according to the operation method in an extension reaction in Second pre step similarly to Step A of the above (iii).
(v) By repeating the regular steps of Third step after temporarily separating the extensionally formed double-stranded DNA obtained in each of the regular steps into a single-stranded state (for example, Step A and Step B), the methylated DNA in the objective DNA region is amplified to a detectable level and a content of the amplified DNA is quantified.

In Third step, discussed above, concretely the reaction starting from First pre step and up to regular steps may be executed as a single PCR reaction. Also, from First pre step step to Third pre step, each reaction may be independently executed, and only regular steps may be executed as a PCR reaction.

As a method of amplifying an objective DNA region (namely, an objective region) contained in the selected single-stranded DNA, PCR is used. Using a primer preliminarily labeled with fluorescence or the like and utilizing the label as an index in amplifying the target region make it possible to evaluate presence or absence of an amplification product without executing a burdensome operation such as electrophoresis. As a PCR reaction solution, for example, a reaction solution obtained by mixing DNA obtained in Second step of the present measuring method with 0.15 µL of a 50 µM primer solution, 2.5 µL of 2 mM dNTP, 2.5 µL of a 10 × buffer (100 mM Tris-HCl pH 8.3, 500 mM KCl 20 mM MgCl₂, 0.01% Gelatin), and 0.2 µL of AmpliTaq Gold (one kind of thermostable DNA polymerase: 5 U/µL), and adding sterilized ultrapure water to make the liquid volume 25 µL can be recited.

Since an objective DNA region (namely, an objective region) often has a GC rich nucleotide sequence, the reaction may be occasionally executed with addition of an appropriate amount of betaine, DMSO or the like. In one exemplary reaction condition, the reaction solution as described above is kept at 95°C for 10 minutes, and then a cycle including incubation of 30 seconds at 95°C, 30 seconds at 55 to 65°C, and 30 seconds at 72°C is repeated 30 to 40 times. After conducting such PCR, the obtained amplification product is detected. For example, when a preliminarily labeled primer is used, an amplification amount by a PCR reaction can be evaluated by measuring an amount of a fluorescent label after executing washing and purification operations similar to those as described above. When PCR is conducted using a normal primer that is not labeled, a probe or the like that is labeled with a gold colloid particle, fluorescence or the like is caused to anneal, and detection may be achieved by measuring an amount of the probe bound to the target region. Also, for determining an amount of an amplification product more accurately, for example, a real time PCR method may be used. Real time PCR is a method in which PCR is monitored in real time, and the obtained monitor result is analyzed kinetically, and is known as a high-accuracy quantitative PCR method capable of detecting a very small difference of as small as twice in a gene amount. As such a real time PCR method, for example, a method using a probe such as a template-dependent nucleic acid polymerase probe, a method using an intercalator such as SYBR-Green and the like can be recited. As an apparatus and a kit for the real time PCR method, those commercially available may be used. As described above, detection may be executed by any method conventionally well-known without any particular limitation. These methods make it possible to conduct the operations up to detection without requiring change of the reaction container.

The present invention may be used in the following situations.

It is known that DNA methylation abnormality occurs in various diseases (for example, cancer), and it is believed that the degree of various diseases can be measured by detecting this DNA methylation abnormality.

For example, when there is a DNA region where methylation occurs at 100% in genomic DNA contained in a diseased biological specimen, and the present invention is executed for the DNA region, the amount of methylated DNA will increase. For example, when there is a DNA region where methylation does not occur at 100% in genomic DNA contained in a diseased biological specimen, and the present measuring method is executed for the DNA region, the amount of methylated DNA will be approximately 0. For example, when there is a DNA region which is in hypomethylation in genomic DNA contained in a specimen derived from a healthy subject, and in hypermethylation in genomic DNA contained in a specimen derived from a disease subject, and the present measuring method is executed for the DNA region, the amount of methylated DNA would be approximately 0 for the healthy subject, and a significantly higher value than that of the healthy subject will be exhibited by the disease patient, so that the "degree of disease" can be determined based on this difference in value. The "degree of disease" used herein has the same meaning commonly used in this field of art, and concretely means, for example, malignancy when the biological specimen is a cell, and means, for example, abundance of disease cells in the tissue when the biological specimen is a tissue. Further, when the biological specimen is plasma or serum, it means the probability that the individual has the disease. Therefore, the present measuring method makes it possible to diagnose various diseases by examining methylation abnormality.

Restriction enzymes, primers or probes that can be used in various methods for measuring a methylated DNA amount in a target region in the present measuring method are useful as reagents of a detection kit. The present invention also provides a detection kit containing these restriction enzymes, primers or probes as reagents, and a detection chip in which these primers, probes and so on are immobilized on a support, and a scope of the present measuring method or the present methylation rate measuring method of course embraces use in the form of a detection kit or a detection chip as described above utilizing the substantial principle of the method.

### Examples

In the following, the present invention will be explained in detail by way of examples, however, the present invention will not be limited to these examples.

### Example 1

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.1 µg/100 µL solution of an antibody in a 0.1% BSA-containing phosphate buffer (1 mM KH₂PO₄, 3 mM Na₂HPO·7H₂O, 154 mM NaCl, pH 7.4)].

To each PCR tube coated with streptavidin (a total of 9 tubes), 50 µL of the synthetically obtained biotin-labeled methylcytosine antibody solution was added and immobilized to the PCR tube by leaving it still for about an hour at room temperature. Then, after removing the solution by pipetting, 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to preparation of an immobilized methylated DNA antibody used in the present measuring method).

A partially methylated oligonucleotide GPR7-2079-2176/98 mer-M(7) in which a recognition site of HpaII comprising the nucleotide sequence of SEQ ID NO: 17 is methylated; a partially methylated oligonucleotide GPR7-2079-2176/98 mer-HM(5) in which part of a recognition site of HpaII having the nucleotide sequence of SEQ ID NO: 18 is not methylated; and an unmethylated oligonucleotide GPR7-2079-2176/98 mer-UM having the nucleotide sequence of SEQ ID NO: 19 were synthesized, and a 0.001 pmol/10 µL solution in a TE buffer was prepared for each oligonucleotide.

### <Partially methylated oligonucleotide in which recognition sequence of HpaII is methylated>

N denotes methylated cytosine.
GPR7-2079-2176/98mer-M(7):

### <Partially methylated oligonucleotide in which recognition sequence of HpaII is not methylated>

N denotes methylated cytosine.
GPR7-2079-2176/98mer-HM(5):

### <Unmethylated oligonucleotide>

GPR7-2079-2176/98mer-UM:

Each of the obtained solutions (each solution was prepared in triplicate) was subjected to the following A treatment, B treatment or C treatment (each prepared singly).

A treatment group (no treatment group): The sample prepared above was added with 5 µL of a buffer (330 mM Tris-Acetate pH 7.9, 660 mM KOAc, 100 mM MgOAc₂, 5 mM Dithiothreitol), and 5 µL of BSA (Bovine serum albumin 1 mg/mL), and the resultant mixture was added with sterile ultrapure water to a liquid volume of 50 µL.

B treatment group (HpaII treatment group): The sample prepared above was added with 5 µL of a buffer (330 mM Tris-Acetate pH 7.9, 660 mM KOAc, 100 mM MgOAc₂, 5 mM Dithiothreitol), 5 µL of BSA (Bovine serum albumin 1 mg/mL), and 10 U of HpaII, and the resultant mixture was added with sterile ultrapure water to a liquid volume of 50 µL.

C treatment group (addition of masking oligonucleotide + HpaII treatment group): The sample prepared above was added with 5 µL of a buffer (330 mM Tris-Acetate pH 7.9, 660 mM KOAc, 100 mM MgOAc₂, 5 mM Dithiothreitol), 5 µL of BSA (Bovine serum albumin 1 mg/mL), 10 U of HpaII, and 5 pmol of the oligonucleotide MA comprising the nucleotide sequence of SEQ ID NO: 20 as masking oligonucleotide, and the resultant mixture was added with sterile ultrapure water to a liquid volume of 50 µL.

### <Masking oligonucleotide>

MA: 5'-GCCACCCGGCGCGA-3' (SEQ ID NO: 20)

Each reaction mixture was incubated overnight at 37°C (these correspond to First step of the present invention).

The PCR tube coated with streptavidin to which a biotin-labeled methylcytosine antibody was immobilized was added with 50 µL of a reaction solution of an oligonucleotide prepared as described above, and left still for an hour at room temperature. Then the solution was removed by pipetting, and 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to Second step of the present invention).

Next, the above PCR tube was subjected to PCR using each solutions of a primer comprising the nucleotide sequences of SEQ ID NO: 21 and a primer comprising the nucleotide sequences of SEQ ID NO: 22 (PF1 and PR1), and the following reaction condition, to amplify methylated DNA in a target DNA region (GPR7-2079-2176, SEQ ID NO: 23, methylated cytosine is also denoted by C).

### <Primers>

PF1: 5'-GTTGGCCACTGCGGAGTCG-3' (SEQ ID NO: 21)
PR1: 5'-GCACGACGAGTGTGACGATC-3' (SEQ ID NO: 22)

### <Target DNA region>

A reaction solution of PCR was prepared by mixing each 5 µL of a solution of primer comprising the nucleotide sequence of SEQ ID NO: 21 and a solution of primer comprising the nucleotide sequence of SEQ ID NO: 22 prepared to 3 µM, each 5 µL of 2 mM dNTPs, 5 µL of a buffer (100 mM Tris-HCl pH 8.3, 500 mM KCl, 15 mM MgCl₂, 0.01% Gelatin), 0.25 µL of 5 U/µL thermostable DNA polymerase (AmpliTaq Gold), and 10 µL of a 5 N betaine aqueous solution to DNA which is a template, and adding sterile ultrapure water to a liquid volume of 50 µL. The reaction solution was kept at 95°C for 10 minutes, and then subjected to PCR conducting 25 cycles of incubation each including 30 seconds at 95°C-, 30 seconds at 59°C, and 45 seconds at 72°C.

After conducting PCR, the amplification of DNA was checked by 2% agarose gel electrophoresis (these correspond to Third step of the present invention). The result is shown in Fig. 1.

In the case of A treatment group (no treatment group), in the partially methylated oligonucleotide GPR7-2079-2176/98 mer-M(7) in which the recognition sequence of HpaII is methylated, and the partially methylated oligonucleotide GPR7-2079-2176/98 mer-HM(5) in which part of the recognition sequence of HpaII is not methylated, amplification of DNA was observed, and an amplification product thereof (target DNA region: GPR7-2079-2176) was obtained. In the unmethylated oligonucleotide GPR7-2079-2176/98 mer-UM, amplification of DNA was not observed, and an amplification product thereof was not obtained. Also in the case of B treatment group (HpaII treatment group), the result was similar to that in A treatment group. In the case of C treatment group (addition of masking oligonucleotide + HpaII treatment group), in the partially methylated oligonucleotide GPR7-2079-2176/98 mer-M(7) in which the recognition sequence of HpaII is methylated, amplification of DNA was observed, and an amplification product thereof (target DNA region: GPR7-2079-2176) was obtained. Contrarily, in the cases of the partially methylated oligonucleotide GPR7-2079-2176/98 mer-HM(5) in which part of the recognition sequence of HpaII is not methylated, and in the unmethylated oligonucleotide GPR7-2079-2176/98 mer-UM, amplification of DNA was not observed, and an amplification product thereof was not obtained.

From the above, it was demonstrated that single-stranded DNA containing a methylated target DNA region can be selected by an immobilized methylated cytosine antibody, and a target DNA region in which methylation sensitive restriction enzyme recognition site is unmethylated and protected by a masking oligonucleotide can be digested by a treatment with a methylation sensitive restriction enzyme after addition and mixing of a masking oligonucleotide, and only methylated DNA can be amplified to a detectable level and an amount of amplified DNA can be quantified while unmethylated DNA in the target DNA region is not amplified.

### Example 2

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.25 µg/µL solution of an antibody in a 0.1% BSA-containing phosphate buffer (1 mM KH₂PO₄, 3 mM Na₂HPO·7H₂O, 154 mM NaCl, pH 7.4)].

To each PCR tube coated with streptavidin (a total of 8 tubes), 50 µL of the synthetically obtained biotin-labeled methylcytosine antibody solution was added and immobilized to the PCR tube by leaving it still for about an hour at room temperature. Then, after removing the solution by pipetting, 100 pL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to preparation of an immobilized methylated DNA antibody used in the present measuring method).

For genomic DNA derived from human blood purchased from Clontech, a DNA fragment (X, SEQ ID NO: 26, a region corresponding to the base numbers 25687390 to 25687775 shown in Genbank Accession No. NT_029419 and so on) to be used as a test sample was amplified by conducting PCR using an oligonucleotide primer of SEQ ID NO: 24 and an oligonucleotide primer of SEQ ID NO: 25 (PF2 and PR2) and the following reaction condition.

### <Oligonucleotide primers designed for PCR>

PF2: 5'-CTCAGCACCCAGGCGGCC-3' (SEQ ID NO: 24)
PCR2: 5'-CTGGCCAAACTGGAGATCGC-3' (SEQ 1D NO: 25)

### <DNA fragment>

As a reaction solution of PCR, 5 ng of genomic DNA which is a template, mixed with each 3 µL of oligonucleotide primer solutions prepared to 5 µM, each 5 µL of 2 mM dNTPs, 5 µL of a 10 × buffer (100 mM Tris-HC1 pH 8.3, 500 mM KC1, 15 mM MgCl₂, 0.01% Gelatin), and 0.25 µL of 5 U/µL thermostable DNA polymerase (AmpliTaq Gold, available from ABI), and added with sterile ultrapure water to a liquid volume of 50 µL was used. The reaction solution was kept at 95°C for 10 minutes, and then subjected to PCR conducting 40 cycles of incubation each including 30 seconds at 95°C, 30 seconds at 61°C, and 45 seconds at 72°C.

After conducting PCR, amplification was checked by 1.5% agarose gel electrophoresis, and a DNA fragment X was purified with Wizard SV Gel/PCR Kit (PROMEGA).

For a part of the obtained DNA fragment solution, a reaction solution was prepared by mixing 1 µL of SssI methylase (available from NEB), 10 uL of a 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MX, SEQ ID NO: 27).

### <DNA fragment> (N denotes 5-methylcytosine.)

For each obtained DNA fragment X, the following solutions were prepared.
Solution A: 100 pg/5 µL solution in TE
Solution B: 10 pg/5 µL solution in TE
Solution C: 1 pg/5 µL solution in TE
Solution D: TE solution (negative control solution)

For each obtained DNA fragment MX, the following solutions were prepared.
Solution MA: 100 pg/5 µL solution in TE
Solution MB: 10 pg/5 µL solution in TE
Solution MC: 1 pg/5 µL solution in TE
Solution MD: TE solution (negative control solution)

For each of the solutions of the DNA fragment X and the solutions of the methylated DNA fragment MX, the following treatment was executed.

Five (5) µL of the DNA fragment solution prepared above was added with 2 µL of a buffer (330 mM Tris-Acetate pH 7.9, 660 mM KOAc, 100 mM MgOAc₂, 5 mM Dithiothreitol), 2 µL of BSA (Bovine serum albumin 1 mg/ml), 12 U of a methylation sensitive restriction enzyme-HpaII, and the resultant mixture was further added with sterile ultrapure water to a liquid volume of 20 µL. Each of the mixture was incubated 37°C for 3 hours (these correspond to First step of the present measureing method).

Counter oligonucleotides C1 to C12 comprising the nucleotide sequences of SEQ ID NO: 29 to SEQ ID NO: 40 capable of complementarily base-pairing with a minus strand of the target DNA region X' comprising the nucleotide sequence of SEQ ID NO: 28 were synthesized, and each 0.01 µM solutions in TE buffer were prepared.

### <Target DNA region>

### <Counter oligonucleotides>

C1:5' - GCCACCGCGAAAGTCACCGCCAGCACGGCG -3' (SEQ ID NO: 29)
C2:5' - GCCAGCAGTACGCTGCTGTTGCCCAGCACG -3' (SEQ ID NO: 30)
C3:5' - CGGGACGTCTTGCGCGGCGTCCGGTGCAGA -3' (SEQ ID NO: 31)
C4:5' - AGGCTGAGGTGTCGGATGAAGAGGTGCATG -3' (SEQ ID NO: 32)
C5:5' - ACCTGGAAGAATGCCACGGCCAGGTCGGCC -3' (SEQ ID NO: 33)
C6:5' - TAGGTGATGTCCCAGCACATTTGCGGCAGC -3' (SEQ ID NO: 34)
C7:5' - CGGCACAGCCAGTCGGGGCCGCGGAAGCGG -3' (SEQ ID NO: 35)
C8:5' - ATGCCGAACACCTGCAGGTGCTTCACCACG -3' (SEQ ID NO: 36)
C9:5' - ATGACTACCAGCATGTAGGCCGACGCAAAC -3' (SEQ ID NO: 37)
C10:5' - TGGCACACCGCGATGTAGCGGTCGGCTGTC -3' (SEQ ID NO: 38)
C11:5' - CGCGCGGGCTGTTGCAGAGTCTTGAGCGGG -3' (SEQ ID NO: 39)
C12:5' - CAGGCGGCCGCGATCATGAGGCGCGAGCGG -3' (SEQ ID NO: 40)

To the above reaction solution, 10 µL of a counter oligonucleotide solution prepared as described above, 5 µL of a buffer (330 mM Tris-Acetate pH 7.9, 660 mM KOAc, 100 mM MgOAc₂, 5 mM Dithiothreitol), 5 µL of a 100 mM MgCl₂ solution, and 5 µL of a 1 mg/mL BSA solution were added, and the resultant mixture was added with sterile ultrapure water to a liquid volume of 50 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to Second step of the present measuring method).

The PCR tube coated with streptavidin to which a biotin-labeled methylcytosine antibody was immobilized was added with 50 µL of a reaction solution of a DNA fragment prepared as described above, and left still for 30 minutes at room temperature. Then the solution was removed by pipetting, and 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to Third step of the present measuring method).

Then by subjecting the above PCR tube to PCR using respective solutions of oligonucleotide primers PF2 and PR2 comprising the nucleotide sequences of SEQ ID NO: 24 and SEQ ID NO: 25, and the following reaction condition, methylated DNA in a target DNA region X' comprising the nucleotide sequence of SEQ ID NO: 28 was amplified.

### <Oligonucleotide primers designed for PCR>

PF2: 5'-CTCAGCACCCAGGCGGCC-3' (SEQ ID NO: 24)
PR2: 5'-CTGGCCAAACTGGAGATCGC-3' (SEQ ID NO: 25)

### <Target DNA region>

As a reaction solution of PCR, DNA which is a template, mixed with each 3 µL of oligonucleotide primer solutions prepared to 5 µM, each 5 µL of 2 mM dNTPs, 5 µL of a buffer (100 mM Tris-HCl pH 8.3, 500 mM KCl 15 mM MgCl₂, 0.01% Gelatin), and 0.25 µL of 5 U/µL thermostable DNA polymerase (AmpliTaq Gold, available from ABI), and added with sterile ultrapure water to a liquid volume of 50 µL was used. The reaction solution was kept at 95°C for 10 minutes, and then subjected to PCR conducting 25 cycles of incubation each including 20 seconds at 95°C, 30 seconds at 61°C, and 30 seconds at 72°C.

After conducting PCR, amplification was checked by 1.5% agarose gel electrophoresis (these correspond to Fourth step of the present measuring method).

The result is shown in Fig. 2. In Solutions MA, MB and MC of the methylated DNA fragment MX, amplification was observed, and an amplification product thereof was obtained. In the negative control solution MD, amplification of DNA was not observed, and an amplification product was not obtained. In solutions A, B, C and D of the unmethylated DNA fragment X, amplification was not observed, and an amplification product thereof was not obtained.

From the above, it was demonstrated that DNA containing a methylated target DNA region can be selected by an immobilized methylcytosine antibody, and amplified DNA can be detected with higher sensitivity by amplifying methylated DNA to a detectable level.

### Example 3

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.25 µg/µL solution of an antibody in a 0.1% BSA-containing phosphate buffer (1 mM KH₂PO₄, 3 mM Na₂HPO·7H₂O, 154 mM NaCl, pH 7.4)].

To each PCR tube coated with streptavidin (a total of 8 tubes), 50 µL of 0.1 µg/50 µL solution of the synthetically obtained biotin-labeled methylcytosine antibody was added and immobilized to the PCR tube by leaving it still for about an hour at room temperature. Then, after removing the solution by pipetting, 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to preparation of an immobilized methylated DNA antibody used in the present measuring method).

For genomic DNA derived from human blood purchased from Clontech, a DNA fragment (Y, SEQ ID NO: 43, a region corresponding to the base numbers 76606 to 76726 shown in Genbank Accession No. ac009800 and so on) to be used as a test sample was amplified by conducting PCR using oligonucleotide primers (PF3 and PR3) of SEQ ID NO: 42 and SEQ ID NO: 43 and the following reaction condition.

### <Oligonucleotide primers designed for PCR>

PF3: 5'-TGAGCTCCGTAGGGCGTCC-3' (SEQ ID NO: 41)
PR3: 5'-GCGCCGGGTCCGGGCCC-3' (SEQ ID NO: 42)

### <DNA fragment>

As a reaction solution of PCR, 5 ng of genomic DNA which is a template, mixed with each 3 µL of oligonucleotide primer solutions prepared to 5 µM, each 5 µL of 2 mM dNTPs, 5 µL of a 10 × buffer (100 mM Tris-HCl pH 8.3, 500 mM KCl 15 mM MgCl₂, 0.01% Gelatin), and 0.25 µL of 5 U/µL thermostable DNA polymerase (AmpliTaq Gold, available from ABI), and added with sterile ultrapure water to a liquid volume of 50 µL was used. The reaction solution was kept at 95°C for 10 minutes, and then subjected to PCR conducting 50 cycles of incubation each including 30 seconds at 95°C, 30 seconds at 60°C, and 45 seconds at 72°C.

After conducting PCR, amplification of DNA was checked by 1.5% agarose gel electrophoresis, and a DNA fragment Y was purified with Wizard SV Gel/PCR Kit (PROMEGA).

For a part of the obtained DNA fragment solution, a reaction solution was prepared by mixing 1 µL of SssI methylase (available from NEB), 10 µL of a 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 5 times, to obtain a methylated DNA fragment (MY, SEQ ID NO: 44).

### <DNA fragment> (N denotes 5-methylcytosine.)

For each obtained DNA fragment Y, the following solutions were prepared.
Solution A: 100 pg/5 µL solution in TE
Solution B: 10 pg/5 µL solution in TE
Solution C: 1 pg/5 µL solution in TE
Solution D: TE solution (negative control solution)

For each obtained DNA fragment MY, the following solutions were prepared.
Solution MA: 100 pg/5 µL solution in TE
Solution MB: 10 pg/5 µL solution in TE
Solution MC: 1 pg/5 µL solution in TE
Solution MD: TE solution (negative control solution)

For each of the solutions of the DNA fragment Y and the solutions of the methylated DNA fragment MY, the following treatment was executed.

Five (5) µL of the DNA fragment solution prepared above was added with 2 µL of a buffer (330 mM Tris-Acetate pH 7.9, 660 mM KOAc, 100 mM MgOAc₂, 5 mM Dithiothreitol), 2 µL of BSA (Bovine serum albumin 1 mg/ml), 12 U of a methylation sensitive restriction enzyme HpaII, and the resultant mixture was further added with sterile ultrapure water to a liquid volume of 20 µL. Each of the mixture was incubated 37°C for 3 hours (these correspond to First step of the present measureing method).

Counter oligonucleotides C13, C14, and C15 comprising the each nucleotide sequences of SEQ ID NO: 46, SEQ ID NO: 47, and SEQ ID NO: 48 capable of complementarily base-pairing with a minus strand of the target DNA region Y' comprising the nucleotide sequence of SEQ ID NO: 45 were synthesized, and each 0.01 µM solutions in a TE buffer were prepared.

### <Target DNA region>

### <Counter oligonucleotides>

C13: 5'-GCGTCCCCCAAGGACGCCCTACGGAGCTCA-3' (SEQ ID NO: 46)
C14: 5'-CTCAACGAGGGCAGAGGAGCCGGCGACCTG-3' (SEQ ID NO: 47)
C15: 5'-CGCCGGGTCCGGGCCCGATGCGTTGGCGGG-3' (SEQ ID NO: 48)

To the above reaction solution, 10 µL of a counter oligonucleotide solution prepared as described above, 5 µL of a buffer (330 mM Tris-Acetate pH 7.9, 660 mM KOAc, 100 mM MgOAc₂, 5 mM Dithiothreitol), 5 µL of a 100 mM MgCl₂ solution, and 5 µL of a 1 mg/mL BSA solution were added, and the resultant mixture was added with sterile ultrapure water to a liquid volume of 50 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to Second step of the present measuring method).

The PCR tube coated with streptavidin to which a biotin-labeled methylcytosine antibody was immobilized was added with 50 µL of a reaction solution of a DNA fragment prepared as described above, and left still for 30 minutes at room temperature. Then the solution was removed by pipetting, and 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to Third step of the present measuring method).

Then by subjecting the above PCR tube to PCR using respective solutions of oligonucleotide primers PF3 and PR3 comprising the nucleotide sequences of SEQ ID NO: 41 and SEQ ID NO: 42, and the following reaction condition, methylated DNA in a target DNA region Y' comprising the nucleotide sequence of SEQ ID NO: 45 was amplified.

### <Oligonucleotide primers designed for PCR>

PF3: 5'-TGAGCTCCGTAGGGCGTCC-3' (SEQ ID NO: 41)
PR3: 5'-GCGCCGGGTCCGGGCCC-3' (SEQ ID NO: 42)

### <Target DNA region>

As a reaction solution of PCR, DNA which is a template, mixed with each 3 µL of oligonucleotide primer solutions prepared to 5 µM, each 5 µL of 2 mM dNTPs, 5 µL of a 10 × buffer (100 mM Tris-HCl pH 8.3, 500 mM KCl 15 mM MgCl₂, 0.01% Gelatin), and 0.25 µL of 5 U/µL thermostable DNA polymerase (AmpliTaq Gold, available from ABI), and added with sterile ultrapure water to a liquid volume of 50 µL was used. The reaction solution was kept at 95°C for 10 minutes, and then subjected to PCR conducting 25 cycles of incubation each including 20 seconds at 95°C, 30 seconds at 60°C, and 30 seconds at 72°C.

After conducting PCR, amplification of DNA was checked by 1.5% agarose gel electrophoresis (these correspond to Third step of the present measuring method).

The result is shown in Fig. 3. In Solutions MA, MB and MC of the methylated DNA fragment MY, amplification was observed. In the negative control solution MD, amplification of DNA was not observed. In solutions A, B, C and D of the unmethylated DNA fragment Y, amplification of DNA was not observed.

From the above, it was demonstrated that DNA containing a methylated target DNA region can be selected by an immobilized methylcytosine antibody, and amplified DNA can be detected with higher sensitivity by amplifying methylated DNA to a detectable level.

### Example 4

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.25 µg/µL solution of an antibody in a 0.1% BSA-containing phosphate buffer (1 mM KH₂PO₄, 3 mM Na₂HPO·7H₂O, 154 mM NaCl, pH 7.4)].

To each PCR tube coated with streptavidin (a total of 8 tubes), 50 µL of 0.1 µg/50 µL solution of the synthetically obtained biotin-labeled methylcytosine antibody was added and immobilized to the PCR tube by leaving it still for about an hour at room temperature. Then, after removing the solution by pipetting, 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two timers (these correspond to preparation of an immobilized methylated DNA antibody used in the present measuring method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 0.1% 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

From the obtained genomic DNA, a DNA fragment to be used as a test sample (T, SEQ ID NO: 51, a region corresponding to the base numbers 384569 to 384685 of yeast chromosome VII shown in Genbank Accession No. NC_001139 and so on) was amplified by conducting PCR using oligonucleotide primers (PF4 and PR4) designed for PCR of SEQ ID NO: 49 and SEQ ID NO: 50 and the following reaction condition.

### <Oligonucleotide primers designed for PCR>

PF4: 5'-GGACCTGTGTTTGACGGGTAT-3' (SEQ ID NO: 49)
PR4: 5'-AGTACAGATCTGGCGTTCTCG-3' (SEQ ID NO: 50)

### <DNA fragment>

As a reaction solution of PCR, 10 ng of genomic DNA which is a template, mixed with each 3 µL of oligonucleotide primer solutions prepared to 5 µM, each 5 µL of 2 mM dNTPs, 5 µL of a 10 × buffer (100 mM Tris-HCl pH 8.3, 500 mM KCl 15 mM MgCl₂, 0.01% Gelatin), and 0.25 µL of 5 U/µL thermostable DNA polymerase (AmpliTaq Gold, available from ABI), and added with sterile ultrapure water to a liquid volume of 50 µL was used. The reaction solution was kept at 95°C for 10 minutes, and then subjected to PCR conducting 40 cycles of incubation each including 20 seconds at 95°C, 30 seconds at 58°C, and 30 seconds at 72°C.

After conducting PCR, amplification was checked by 1.5% agarose gel electrophoresis, and a DNA fragment T was purified with Wizard SV Gel/PCR Kit (PROMEGA).

For a part of the obtained DNA fragment solution, a reaction solution was prepared by mixing 1 µL of SssI methylase (available from NEB), 10 µL of a 10 × NEBuffer 2 (available from NEB), and 1 µL of S-adenosyl methionine (3.2 mM, available from NEB), and adding sterile ultrapure water to a liquid volume of 100 µL. The reaction solution was incubated at 37°C for 15 to 30 minutes, and further added with 1 µL of S-adenosyl methionine (3.2 mM, available from NEB) and incubated at 37°C for 15 to 30 minutes. This was then purified with Wizard SV Gel/PCR Kit (PROMEGA). These operations were repeated another 3 times, to obtain a methylated DNA fragment (MT, SEQ ID NO: 52).

### <DNA fragment> (N denotes 5-methylcytosine.)

For each obtained DNA fragment T, the following solutions were prepared.
Solution A: 100 pg/5 µL solution in TE
Solution B: 10 pg/5 µL solution in TE
Solution C: 1 pg/5 µL solution in TE
Solution D: TE solution (negative control solution)

For each obtained DNA fragment MZ, the following solutions were prepared.
Solution MA: 100 pg/5 µL solution in TE
Solution MB: 10 pg/5 µL solution in TE
Solution MC: 1 pg/5 µL solution in TE
Solution MD: TE solution (negative control solution)

For each of the solutions of the DNA fragment T and the solutions of the methylated DNA fragment MT, the following treatment was executed.

Five (5) µL of the DNA fragment solution prepared above was added with 2 µL of a buffer (330 mM Tris-Acetate pH 7.9, 660 mM KOAc, 100 mM MgOAc₂, 5 mM Dithiothreitol), 2 µL of BSA (Bovine serum albumin 1 mg/ml), 12 U of a methylation sensitive restriction enzyme HpaII, and the resultant mixture was further added with sterile ultrapure water to a liquid volume of 20 µL. Each of the mixture was incubated 37°C for 3 hours (these correspond to First step of the present measureing method).

Counter oligonucleotides C16 to C19 each comprising the nucleotide sequences of SEQ ID NO: 54 to SEQ ID NO: 57 capable of complementarily base-pairing with a minus strand of the target DNA region T' comprising the nucleotide sequence of SEQ ID NO: 53 were synthesized, and each 0.01 µM solutions in a TE buffer were prepared.

### <Target DNA region>

### <Counter oligonucleotides>

C16: 5'-GGACCTGTGTTTGACGGGTAT-3' (SEQ ID NO: 54)
C17: 5'-AACACTAAGTTGCGCAATTTGCTGT-3' (SEQ ID NO: 55)
C18: 5'-ATTGCGAAATCCGCCCGGACGATAT-3' (SEQ ID NO: 56)
C19: 5'-CACTCTTGAGCGCATGTGCCGTTTC-3' (SEQ ID NO: 57)

To the above reaction solution, 10 µL of a counter oligonucleotide solution prepared as described above, 5 µL of a buffer (330 mM Tris-Acetate pH 7.9, 660 mM KOAc, 100 mM MgOAc₂, 5 mM Dithiothreitol), 5 µL of a 100 mM MgCl₂ solution, and 5 µL of a 1 mg/mL BSA solution were added, and the resultant mixture was added with sterile ultrapure water to a liquid volume of 50 µL, and mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to Second step of the present measuring method).

The PCR tube coated with streptavidin to which a biotin-labeled methylcytosine antibody was immobilized was added with 50 µL of a reaction solution of a DNA fragment prepared as described above, and left still for 30 minutes at room temperature. Then the solution was removed by pipetting, and 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to Third step of the present measuring method).

Then the above PCR tube was subjected to PCR using respective solutions of oligonucleotide primers PF4 and PR4 comprising the nucleotide sequences of SEQ ID NO: 49 and SEQ ID NO: 50, and the following reaction condition, methylated DNA in a target DNA region T' comprising the nucleotide sequence of SEQ ID NO: 53 was amplified.

### <Oligonucleotide primers designed for PCR>

PF4: 5'-GGACCTGTGTTTGACGGGTAT-3' (SEQ ID NO: 49)
PR4: 5'-AGTACAGATCTGGCGTTCTCG-3' (SEQ ID NO: 50)

### <Target DNA region> (Also 5-methylcytosine is denoted by C.)

As a reaction solution of PCR, DNA which is a template, mixed with each 3 µL of oligonucleotide primer solutions prepared to 5 µM, each 5 µL of 2 mM dNTPs, 5 µL of a buffer (100 mM Tris-HCl pH 8.3, 500 mM KCl, 15 mM MgCl₂, 0.01% Gelatin), and 0.25 µL of 5 U/µL thermostable DNA polymerase (AmpliTaq Gold, available from ABI), and added with sterile ultrapure water to a liquid volume of 50 µL was used. The reaction solution was kept at 95°C for 10 minutes, and then subjected to PCR conducting 28 cycles of incubation each including 20 seconds at 95°C, 30 seconds at 58°C, and 30 seconds at 72°C.

After conducting PCR, amplification of DNA was checked by 1.5% agarose gel electrophoresis (these correspond to Fourth step of the present measuring method).

The result is shown in Fig. 4. In Solutions MA, MB and MC of the methylated DNA fragment MT, amplification of DNA was observed, and an amplification product thereof was obtained. In the negative control solution MD, amplification of DNA was not observed, and an amplification product was not obtained. In solutions A, B, C and D of the unmethylated DNA fragment T, amplification of DNA was not observed, and an amplification product thereof was not obtained.

From the above, it was demonstrated that DNA containing a methylated target DNA region can be selected by an immobilized methylcytosine antibody, and amplified DNA can be detected with higher sensitivity by amplifying methylated DNA to a detectable level.

### Example 5

A commercially available methylated cytosine antibody (available from Aviva Systems Biology) was labeled with biotin using a commercially available biotinylating kit (Biotin Labeling Kit-NH₂, available from DOJINDO Laboratories) according to the method described in the catalogue. The obtained biotin-labeled methylated cytosine antibody was refrigerated as a solution [about 0.25 µg/µL solution of an antibody in a 0.1% BSA-containing phosphate buffer (1 mM KH₂PO₄, 3 mM Na₂HPO·7H₂O, 154 mM NaCl, pH 7.4)].

To each PCR tube coated with streptavidin (a total of 8 tubes), 50 µL of 0.1 µg/50 µL solution of the synthetically obtained biotin-labeled methylcytosine antibody was added and immobilized to the PCR tube by leaving it still for about an hour at room temperature. Then, after removing the solution by pipetting, 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to preparation of an immobilized methylated DNA antibody used in the present measuring method).

Yeast strain X2180-1A of baker's yeast was cultured in a YPD medium (1% Yeast extract, 2% Peptone, 2% Glucose, pH 5.6 to 6.0) to a turbidity of OD₆₀₀ 0.6 to 1.0, and centrifuged at 10,000 g for 10 minutes, to prepare 1 × 10⁷ of yeast cells. From the prepared yeast cells, a yeast genome was acquired using a generally used preparation method of a yeast genome as described in Methods in Yeast Genetics (Cold Spring Harbor Laboratory).

The prepared yeast cells were suspended in Buffer A (1 M sorbitol, 0.1 M EDTA, pH 7.4), added with 0.1% 2-mercaptoethanol (final concentration 14 mM) and 100 U zymolase (10 mg/ml), and incubated under stirring at 30°C for an hour until the solution became clear. After collecting a protoplast by centrifugation at 550 g for 10 minutes, it was suspended in Buffer B (50 mM Tris-HCl, pH 7.4, 20 mM EDTA), added with sodium dodecyl sulfate in 1% (w/v), and then incubated at 65°C for 30 minutes. Sequentially, 5 M CH₃COOK was added and mingled in a volume ratio of 2/5, and the mixture was cooled on ice for 30 minutes, and then centrifuged at 15,000 g for 30 minutes to collect the supernatant. The collected supernatant was added with 3 M CH₃COONa in a volume ratio of 1/10 and an equal amount of isopropanol and mingled well, and the precipitate obtained by centrifugation at 15,000 g at 4°C for 30 minutes was rinsed with 70% ethanol and collected. After drying, the precipitate was dissolved in 1 mL of TE buffer (10 mM Tris-HCl, pH 8.0, 1 mM EDTA), and added with RNase A (available from Sigma) in a concentration of 40 µg/ml, incubated at 37°C for an hour, and then the mixture was added with proteinase K (available from Sigma) and sodium dodecyl sulfate in a concentrations of 500 µg/mL and 1% (w/v), respectively, and shaken at 55°C for about 16 hours. After end of the shaking, the mixture was extracted with phenol [saturated with 1 M Tris-HCl (pH 8.0)]·chloroform. An aqueous layer was collected, added with NaCl in a concentration of 0.5 N, and allowed to precipitate from ethanol, and the generated precipitate was collected. The collected precipitate was rinsed with 70% ethanol, to obtain genomic DNA.

Part of the obtained genomic DNA was mixed with 1 µL of SssI methylase (available from NEB), 10 µL of a 10 × NEBuffer 2 (available from NEB), and 1 µL of S-adenosyl methionine (3.2 mM, available from NEB), and added with sterile ultrapure water to a liquid volume of 100 µL. The reaction solution was incubated at 37°C for 15 to 30 minutes, added with 1 µL of S-adenosyl methionine (3.2 mM, available NEB) and incubated at 37°C for 15 to 30 minutes. This was then purified by Wizard SV Gel/PCR Kit (PROMEGA). This operation was repeated three times, and methylated genomic DNA was obtained.

For the obtained yeast genomic DNA, the following solutions were prepared.
Solution A: 10 ng/5 µL solution in TE
Solution B: 1 ng/5 µL solution in TE
Solution C: 0.1 ng/5 µL solution in TE
Solution D: TE solution (negative control solution)

For the obtained methylated yeast genomic DNA, the following solutions were prepared.
Solution MA: 10 ng/5 µL solution in TE
Solution MB: 1 ng/5 µL solution in TE
Solution MC: 0.1 ng/5 µL solution in TE
Solution MD: TE solution (negative control solution)

For each of the above yeast genomic DNA solutions and methylated yeast genomic DNA solutions, the following treatment was executed.

A PCR tube was added with 5 µL of the genomic DNA solution prepared as described above, 5 U of a restriction enzyme XspI, and 1 µL of a 10 × buffer (200 mM Tris-HCl pH 8.5, 100 mM MgCl₂, 10 mM Dithiothreitol, 1000 mM KCl) suited for XspI, and added with sterile ultrapure water to a liquid volume of 10 µL. The reaction solution was incubated at 37°C for an hour.

The above solution was added with 2 µL of a buffer (330 mM Tris-Acetate pH 7.9, 660 mM KOAc, 100 mM MgOAc₂, 5 mM Dithiothreitol), 2 µL of BSA (Bovine serum albumin 1 mg/ml), 12 U of a methylation sensitive restriction enzyme HpaII, and the resultant mixture was further added with sterile ultrapure water to a liquid volume of 20 µL. Each of the mixture was incubated 37°C for 3 hours (these correspond to First step of the present measureing method).

Counter oligonucleotides C16 to C23 each comprising the nucleotide sequences of SEQ ID NO: 54 to SEQ ID NO: 57 and SEQ ID NO: 59 to SEQ ID NO: 62, capable of complementarily base-pairing with a minus strand of the target DNA region T'(a region corresponding to the base numbers 384523 to 384766 of yeast chromosome VII shown in Genbank Accession No. NC_001139 and so on) comprising the nucleotide sequence of SEQ ID NO: 58 were synthesized, and each 0.01 µM solutions in a TE buffer were prepared.

### <Target DNA region>

### <Counter oligonucleotides>

C16: 5'-GGACCTGTGTTTGACGGGTAT-3' (SEQ ID NO: 54)
C17: 5'-AACACTAAGTTGCGCAATTTGCTGT-3' (SEQ ID NO: 55)
C18: 5'-ATTGCGAAATCCGCCCGGACGATAT-3' (SEQ ID NO: 56)
C19: 5'-CACTCTTGAGCGCATGTGCCGTTTC-3' (SEQ ID NO: 57)
C20: 5'-AATACATTCCGAGGGCGCCCGCACAAGGCC-3' (SEQ ID O: 59)
C21: 5'-GCGATCGCACACGAGGAGACA-3' (SEQ ID NO: 60)
C22: 5'-AGCGTCACGTGTTTTGCCATTTTGTACGAC-3' (SEQ ID NO: 61)
C23: 5'-AAATGAACCGCCTGGCCACGCCTCTAATC-3' (SEQ ID NO: 62)

To the above reaction solution, 10 µL of a counter oligonucleotide solution prepared as described above, 5 µL of a buffer (330 mM Tris-Acetate pH 7.9, 660 mM KOAc, 100 mM MgOAc₂, 5 mM Dithiothreitol), 5 µL of a 100 mM MgCl₂ solution, and 5 µL of a 1 mg/mL BSA solution were added, and the resultant mixture was added with sterile ultrapure water to a liquid volume of 50 µL, and_mixed. Thereafter, this PCR tube was heated at 95°C for 10 minutes, rapidly cooled to 70°C, and kept at this temperature for 10 minutes. Then the tube was cooled to 50°C and kept at this temperature for 10 minutes, and further kept at 37°C for 10 minutes, and returned to room temperature (these correspond to Second step of the present measuring method).

The PCR tube coated with streptavidin to which a biotin-labeled methylcytosine antibody was immobilized was added with 50 µL of a reaction solution of a DNA fragment prepared as described above, and left still for 30 minutes at room temperature. Then the solution was removed by pipetting, and 100 µL of a washing buffer [0.05% Tween 20-containing phosphate buffer (1 mM KH₂PO₄, 3 mM Na₂HPO·7H₂O, 154 mM NaCl, pH 7.4)] was added, and then the buffer was removed by pipetting. This operation was repeated another two times (these correspond to Third step of the present measuring method).

Then by subjecting the above PCR tube to PCR using respective solutions of oligonucleotide primers PF4 and PR4 comprising the nucleotide sequences of SEQ ID NO: 49 and SEQ ID NO: 50, and the following reaction condition, methylated DNA in a target DNA region T' comprising the nucleotide sequence of SEQ ID NO: 53 was amplified.

### <Oligonucleotide primers designed for PCR>

PF3: 5'-GGACCTGTGTTTGACGGGTAT-3' (SEQ ID NO: 49)
PR3: 5'-AGTACAGATCTGGCGTTCTCG-3' (SEQ ID NO: 50)

### <Target DNA region>

As a reaction solution of PCR, DNA which is a template, mixed with each 3 µL of oligonucleotide primer solutions prepared to 5 µM, each 5 µL of 2 mM dNTPs, 5 µL of a buffer (100 mM Tris-HCl pH 8.3, 500 mM KCl, 15 mM MgCl₂, 0.01% Gelatin), and 0.25 µL of 5 U/µL thermostable DNA polymerase (AmpliTaq Gold, available from ABI), and added with sterile ultrapure water to a liquid volume of 50 µL was used. The reaction solution was kept at 95°C for 10 minutes, and then subjected to PCR conducting 31 cycles of incubation each including 20 seconds at 95°C, 30 seconds at 58°C, and 30 seconds at 72°C.

After conducting PCR, amplification was checked by 1.5% agarose gel electrophoresis (these correspond to Fourth step of the present measuring method).

The result is shown in Fig. 5. In Solutions MA, MB and MC of methylated yeast genomic DNA, amplification of DNA was observed. In the negative control solution MD, amplification of DNA was not observed. In solutions A, B, C and D of unmethylated yeast genomic DNA, amplification of DNA was not observed.

From the above, it was demonstrated that DNA containing a methylated target DNA region can be selected by an immobilized methylcytosine antibody, and methylated DNA is amplified to a detectable level and the amplified DNA can be detected with higher sensitivity.

### INDUSTRIAL APPLICABILITY

Based on the present invention, it becomes possible to provide a method of measuring the content of methylated DNA in an objective DNA region in a genomic DNA contained in a biological specimen in a simple and convenient manner, and so on.

### Free Text in Sequence Listing

SEQ ID NO:17
   Designed oligonucleotide consisting of objective DNA domain (GPR7-2079-2176)
SEQ ID NO:18
   Designed oligonucleotide consisting of objective DNA domain (GPR7-2079-2176)
SEQ ID NO:19
   Designed oligonucleotide consisting of objective DNA domain (GPR7-2079-2176)
SEQ ID NO:20
   Designed oligonucleotide for experiment
SEQ ID NO:21
   Designed oligonucleotide primer for PCR
SEQ ID NO:22
   Designed oligonucleotide primer for PCR
SEQ ID NO:23
   Designed oligonucleotide consisting of objective DNA domain (GPR7-2079-2176)
SEQ ID NO:24
   Designed oligonucleotide primer for PCR
SEQ ID NO:25
   Designed oligonucleotide primer for PCR
SEQ ID NO:26
   Amplified oligonucleotide consisting of objective DNA domain (Genbank Accession No.NT_029419 25687390-25687775 Homo sapiens)
SEQ ID NO:27
   Amplified oligonucleotide consisting of objective DNA domain (Genbank Accession No.NT_029419 25687390-25687775 Homo sapiens)
SEQ ID NO:28
   Amplified oligonucleotide consisting of objective DNA domain (Genbank Accession No.NT_029419 25687390-25687775 Homo sapiens)
SEQ ID NO:29
   Designed counter oligonucleotide for making an objective DNA domain a single strand DNA
SEQ ID NO:30
   Designed counter oligonucleotide for making an objective DNA domain a single strand DNA
SEQ ID NO:31
   Designed counter oligonucleotide for making an objective DNA domain a single strand DNA
SEQ ID NO:32
   Designed counter oligonucleotide for making an objective DNA domain a single strand DNA
SEQ ID NO:33
   Designed counter oligonucleotide for making an objective DNA domain a single strand DNA
SEQ ID NO:34
   Designed counter oligonucleotide for making an objective DNA domain a single strand DNA
SEQ ID NO:35
   Designed counter oligonucleotide for making an objective DNA domain a single strand DNA
SEQ ID NO:36
   Designed counter oligonucleotide for making an objective DNA domain a single strand DNA
SEQ ID NO:37
   Designed counter oligonucleotide for making an objective DNA domain a single strand DNA
SEQ ID NO:38
   Designed counter oligonucleotide for making an objective DNA domain a single strand DNA
SEQ ID NO:39
   Designed counter oligonucleotide for making an objective DNA domain a single strand DNA
SEQ ID NO:40
   Designed counter oligonucleotide for making an objective DNA domain a single strand DNA
SEQ ID NO:41
   Designed oligonucleotide primer for PCR
SEQ ID NO:42
   Designed oligonucleotide primer for PCR
SEQ ID NO:43
   Amplified oligonucleotide consisting of objective DNA domain (Genbank Accession No.AC009800 76606-76726 Homo sapiens)
SEQ ID NO:44
   Designed oligonucleotide consisting of objective DNA domain (Genbank Accession No.AC009800 76606-76726 Homo sapiens ) n=m5c
SEQ ID NO:45
   Designed oligonucleotide consisting of objective DNA domain (Genbank Accession No.AC009800 76606-76726 Homo sapiens)
SEQ ID NO:46
   Designed counter oligonucleotide for making an objective DNA domain a single strand DNA
SEQ ID NO:47
   Designed counter oligonucleotide for making an objective DNA domain a single strand DNA
SEQ ID NO:48
   Designed counter oligonucleotide for making an objective DNA domain a single strand DNA
SEQ ID NO:49
   Designed oligonucleotide primer for PCR
SEQ ID NO:50
   Designed oligonucleotide primer for PCR
SEQ ID NO:51
   Amplified oligonucleotide consisting of objective DNA domain (Genbank Accession No.NC001139 384569-384685 Saccharomyces cereviciae chromosome VII)
SEQ ID NO:52
   Designed oligonucleotide consisting of objective DNA domain (Genbank Accession No.NC001139 384569-384685 Saccharomyces cereviciae chromosome VII) n=m5c
SEQ ID NO:53
   Designed oligonucleotide consisting of objective DNA domain (Genbank Accession No.NC001139 384569-384685 Saccharomyces cereviciae chromosome VII)
SEQ ID NO:54
   Designed counter oligonucleotide for making an objective DNA domain a single strand DNA
SEQ ID NO:55
   Designed counter oligonucleotide for making an objective DNA domain a single strand DNA
SEQ ID NO:56
   Designed counter oligonucleotide for making an objective DNA domain a single strand DNA
SEQ ID NO:57
   Designed counter oligonucleotide for making an objective DNA domain a single strand DNA
SEQ ID NO:58
   Designed oligonucleotide primer for PCR
SEQ ID NO:59
   Designed counter oligonucleotide for making an objective DNA domain a single strand DNA
SEQ ID NO:60
   Designed counter oligonucleotide for making an objective DNA domain a single strand DNA
SEQ ID NO:61
   Designed counter oligonucleotide for making an objective DNA domain a single strand DNA
SEQ ID NO:62
   Designed counter oligonucleotide for making an objective DNA domain a single strand DNA

## Claims

1. A method of measuring the content of methylated DNA in an objective DNA region in a genomic DNA contained in a biological specimen, the objective DNA region having a recognition site of at least one kind of methylation sensitive restriction enzyme, comprising:
(1) First step of mixing a DNA sample comprising a single-stranded DNA (plus strand) containing the objective DNA region and a masking oligonucleotide comprising a nucleotide sequence complementary to a nucleotide sequence of a recognition site for the at least one kind of methylation-sensitive restriction enzyme, thereby obtaining a partially single-stranded DNA in which the recognition site for the methylation-sensitive restriction enzyme forms double strand by complementary base-pairing with the masking oligonucleotide, followed by digesting the DNA sample with the methylation-sensitive restriction enzyme wherein said enzyme digests a recognition site containing unmethylated cytosine and does not digest a recognition site containing methylated cytosine;
(2) Second step of obtaining methylated single-stranded DNA from the DNA sample that has been subjected to the digestion treatment in First step by separating methylated double-stranded DNA contained in the DNA sample into methylated single-stranded DNA, and binding the methylated single-stranded DNA to an immobilized methylated DNA antibody, thereby selecting the single-stranded DNA; and
(3) Third step of amplifying the methylated DNA in the objective DNA region from the selected single-stranded DNA to a detectable level by a polymerase chain reaction (PCR) and quantifying the amount of the amplified DNA.

2. The method of claim 1, wherein the immobilized methylated DNA antibody is a methylcytosine antibody.

3. The method of claim 1 or 2, wherein the biological specimen is blood, a bodily fluid, serum, plasma, a cell lysate, or a tissue lysate from a mammal.

4. The method of any one of claims 1 to 3, wherein the DNA sample derived from the genomic DNA contained in the biological specimen is a DNA sample digested in advance with a restriction enzyme for which a recognition cleavage site is not present in the objective DNA region of the genomic DNA, or a DNA sample purified in advance.

5. The method of any one of claims 1 to 4, wherein the methylation-sensitive restriction enzyme is HhaI.

6. The method of any one of claims 1 to 5, wherein a counter oligonucleotide is added when separating the methylated double-stranded DNA into the methylated single-stranded DNA in Second step.

## Patentansprüche

1. Verfahren zum Messen des Gehalts an methylierter DNA in einer Ziel-DNA-Region in einer genomischen DNA, die in einer biologischen Probe enthalten ist, wobei die Ziel-DNA-Region eine Erkennungsstelle für mindestens eine Art eines methylierungssensitiven Restriktionsenzyms hat, das umfasst:
(1) einen ersten Schritt des Mischens einer DNA-Probe, die eine einzelsträngige DNA (Plus-Strang) umfasst, die die Ziel-DNA-Region enthält und eines maskierenden Oligonucleotids, das eine Nucleotidsequenz umfasst, die komplementär ist zu einer Nucleotidsequenz einer Erkennungsstelle für die mindestens eine Art eines methylierungssensitiven Restriktionsenzyms, wodurch eine teilweise einzelsträngige DNA erhalten wird, in der die Erkennungsstelle für das methylierungssensitive Restriktionsenzym einen Doppelstrang durch komplementäre Basenpaarung mit dem maskierenden Oligonucleotid bildet, gefolgt vom Verdauen der DNA-Probe mit dem methylierungssensitiven Restriktionsenzym, wobei das Enzym eine Erkennungsstelle verdaut, die unmethyliertes Cytosin enthält und keine Erkennungsstelle verdaut, die methyliertes Cytosin enthält;
(2) einen zweiten Schritt des Erhaltens von methylierter einzelsträngiger DNA von der DNA-Probe, die der Verdau-Behandlung im ersten Schritt ausgesetzt war, durch das Auftrennen methylierter doppelsträngiger DNA, die in der DNA-Probe enthalten ist, in methylierte einzelsträngige DNA und das Binden der methylierten einzelsträngigen DNA an einen immobilisierten Antikörper gegen methylierte DNA, wodurch die einzelsträngige DNA selektiert wird; und
(3) einen dritten Schritt des Amplifizierens der methylierten DNA in der Ziel-DNA-Region aus der selektierten einzelsträngigen DNA auf einen nachweisbaren Wert durch eine Polymerasekettenreaktion (PCR) und das Quantifizieren der Menge an amplifizierter DNA.

2. Verfahren nach Anspruch 1, wobei der immobilisierte Antikörper gegen methylierte DNA ein Methylcytosin-Antikörper ist.

3. Verfahren nach Anspruch 1 oder 2, wobei die biologische Probe Blut, eine Körperflüssigkeit, Serum, Plasma, ein Zelllysat oder ein Gewebelysat von einem Säuger ist.

4. Verfahren nach einem der Ansprüche 1 bis 3, wobei die DNA-Probe, die von der genomischen DNA stammt, die in der biologischen Probe enthalten ist, eine DNA-Probe ist, die vorab mit einem Restriktionsenzym verdaut wurde, dessen Erkennungsschnittstelle in der Ziel-DNA-Region der genomischen DNA nicht vorhanden ist, oder eine DNA-Probe ist, die vorab aufgereinigt wurde.

5. Verfahren nach einem der Ansprüche 1 bis 4, wobei das methylierungssensitive Restriktionsenzym Hhal ist.

6. Verfahren nach einem der Ansprüche 1 bis 5, wobei ein Gegen-Oligonucleotid im zweiten Schritt zugegeben wird, wenn die methylierte doppelsträngige DNA in die methylierte einzelsträngige DNA aufgetrennt wird.

## Revendications

1. Procédé pour mesurer la teneur en ADN méthylé dans une région d'ADN cible dans un ADN génomique contenu dans un échantillon biologique, la région d'ADN cible comportant un site de reconnaissance d'au moins un type d'enzyme de restriction sensible à la méthylation, comprenant :
(1) une première étape de mélange d'un échantillon d'ADN comprenant un ADN simple brin (brin positif) contenant la région d'ADN cible et d'un oligonucléotide de masquage comprenant une séquence de nucléotides complémentaire à une séquence de nucléotides d'un site de reconnaissance pour le au moins un type d'enzyme de restriction sensible à la méthylation, en obtenant ainsi un ADN partiellement simple brin dans lequel le site de reconnaissance pour l'enzyme de restriction sensible à la méthylation forme un double brin par un appariement de bases complémentaires avec l'oligonucléotide de masquage, suivi d'une digestion de l'échantillon d'ADN avec l'enzyme de restriction sensible à la méthylation, où ladite enzyme digère un site de reconnaissance contenant une cytosine non méthylée et ne digère pas un site de reconnaissance contenant une cytosine méthylée ;
(2) une deuxième étape d'obtention d'un ADN simple brin méthylé à partir de l'échantillon d'ADN qui a été soumis au traitement de digestion au cours de la première étape en séparant l'ADN double brin méthylé contenu dans l'échantillon d'ADN en ADN simple brin méthylé, et de liaison de l'ADN simple brin méthylé à un anticorps anti-ADN méthylé immobilisé, en sélectionnant ainsi l'ADN simple brin ; et
(3) une troisième étape d'amplification de l'ADN méthylé dans la région d'ADN cible à partir de l'ADN simple brin sélectionné à un niveau détectable par une réaction en chaîne par polymérase (PCR), et de quantification de la quantité de l'ADN amplifié.

2. Procédé selon la revendication 1, dans lequel l'anticorps anti-ADN méthylé immobilisé est un anticorps anti-méthylcytosine.

3. Procédé selon la revendication 1 ou 2, dans lequel l'échantillon biologique est du sang, un fluide corporel, du sérum, du plasma, un lysat cellulaire, ou un lysat tissulaire d'un mammifère.

4. Procédé selon l'une quelconque des revendications 1 à 3, dans lequel l'échantillon d'ADN dérivé de l'ADN génomique contenu dans l'échantillon biologique est un échantillon d'ADN digéré au préalable par une enzyme de restriction pour laquelle un site de clivage de reconnaissance n'est pas présent dans la région d'ADN cible de l'ADN génomique, ou un échantillon d'ADN purifié au préalable.

5. Procédé selon l'une quelconque des revendications 1 à 4, dans lequel l'enzyme de restriction sensible à la méthylation est HhaI.

6. Procédé selon l'une quelconque des revendications 1 à 5, dans lequel un contre-oligonucléotide est ajouté lors de la séparation de l'ADN double brin méthylé en l'ADN simple brin méthylé au cours de la deuxième étape.
